# EUROPEAN PATENT APPLICATION

(11) **EP 2 811 298 A1**
(43) Date of publication of application: **10.12.2014**
(21) Application number: 13002949.9
(22) Date of filing: 07.06.2013
(51) Int. Cl.: G01N 33/53, G01N 33/542

(54) **FRET-Method for identifying a biomolecule-modulating compound**

(71) Applicant: ETH Zurich, 8092 Zurich (CH)
(72) Inventor: Hall, Jonathan, 4143 Dornach (CH); Pradere, Ugo, 8046 Zürich (CH); Roos, Martina, 8050 Zürich (CH)

(57) **Abstract**

The present invention relates to a method for identifying a compound modulating an interaction between two biomolecules or two domains of one biomolecule, the first biomolecule or first domain comprising at least one fluorophore donor and the second biomolecule or second domain comprising at least one fluorophore acceptor or a dark quencher, wherein the fluorophore donor and fluorophore acceptor or the fluorophore donor and dark quencher are spectrally paired such that the energy spectrum emitted by.the fluorophore donor and the excitation energy spectrum of the fluorophore acceptor or the energy spectrum absorbed by the dark quencher overlap at least partially. Preferably, the first and second biomolecules are selected from the group consisting of polypeptides, sugars, polynucleotides, polyamines and lipids, and more preferably the first biomolecule is selected from the group consisting of polypeptides interacting with polynucleotides and the second biomolecule is selected from the group consisting of polynucleotides, preferably microRNAs (miRNA).

## Description

The present invention relates to a method for identifying a compound modulating an interaction between two biomolecules or two domains of one biomolecule, the first biomolecule or first domain comprising at least one fluorophore donor and the second biomolecule or second domain comprising at least one fluorophore acceptor or a dark quencher, wherein the fluorophore donor and fluorophore acceptor or the fluorophore donor and dark quencher are spectrally paired such that the energy spectrum emitted by the fluorophore donor and the excitation energy spectrum of the fluorophore acceptor or the energy spectrum absorbed by the dark quencher overlap at least partially. Preferably, the first and second biomolecules are selected from the group consisting of polypeptides, sugars, polynucleotides, polyamines and lipids, and more preferably the first biomolecule is selected from the group consisting of polypeptides interacting with polynucleotides and the second biomolecule is selected from the group consisting of polynucleotides, preferably4 microRNAs (miRNA).

### Background of the invention

The field of the invention relates to the use of Förster resonance energy transfer (FRET), which is a mechanism describing energy transfer between two chromophores. When both chromophores are fluorescent, i.e. so-called fluorophores, the term "fluorescence resonance energy transfer" is often used instead. In order to avoid an erroneous interpretation of the phenomenon that is always a non-radiative transfer of energy (even when occurring between two fluorophores), the name "Förster resonance energy transfer" is preferred. A fluorophore donor, initially in its electronically excited state, may transfer energy to a fluorophore acceptor through non-radiative dipole-dipole coupling. The efficiency of this energy transfer is inversely proportional to the sixth power of the distance between donor and acceptor making FRET extremely sensitive to small distances.

Measurements of FRET efficiency can be used to determine if two fluorophores are within a certain distance of each other, typically in the proximity of 1 to 10 nm. Such measurements are used as research tools in biology and chemistry. FRET is typically determined by measuring the variation in acceptor emission intensity. When the donor and acceptor are in proximity the acceptor emission will increase because of the FRET from the donor to the acceptor (sensitized emission). FRET efficiencies can also be inferred from the photobleaching rates of the donor in the-presence and absence of an acceptor.

Alternatively, FRET can be measured between a fluorophore donor and a dark quencher. A dark quencher is a family of substances that absorbs emission energy from a fluorophore donor and dissipates the energy as non-UV-visible light or heat, whereas a typical "fluorescent quencher" i.e. fluorophore acceptor re-emits much of the "donated" energy as light. Black hole quencher (BHQ™) dyes from Biosearch Technologies, Inc., Novato, California. USA) are examples of members of the dark quencher family. Dark quenchers such as BHQ dyes are used in molecular biology in conjunction with fluorophores. When the two are close together, e.g. 10-100 A, such as in a molecule, e.g. a protein, the donor's emission is at least partially suppressed by the quencher. This effect can be used to study molecular geometry and motion.

MicroRNAs (miRNA) are a large class of small non-coding RNAs which modulate protein translation as negative gene regulators by post-transcriptionally repressing gene expression. They are involved in cell differentiation, development and metabolism. MiRNAs can function as tumor suppressors and oncogenes. The dysregulation of miRNA expression has been linked to various human malignancies, in particular human cancers, and therefore miRNAs represent a new class of potential drug targets. Mature miRNAs are produced from long primary miRNA transcripts (pri-miRNAs) through sequential cleavages by the Micro-processor and Dicer complexes to release pre-miRNA and mature miRNA species, respectively. MiRNAs regulate the expression of a large part of the human genome by binding to partially complementary sites in the 3' UTRs of mRNAs and inhibiting protein translation or inducing deadenylation and degradation.

Lin28, also called Lin28a and its homologue Lin28b are one of many thousands of RNA binding proteins (RBPs, see list in appended Table 1) which bind to a specific motif (GGAG, or GNNG where N is any ribonucleotide) in pri- and pre-let-7 miRNAs inhibiting their processing and depleting cells of mature let-7. This mechanism appears to be quite general and occurs in about 15% of tumors of different histology and Lin28 and Lin28b activation is associated with advanced disease and poor clinical prognosis (see e.g. Viswanathan, et al., Nat Gen, 2009, 41, 843).

Roos et al. (Poster by ETH Zürich, Institute of Pharmaceutical Sciences, Department of Applied Sciences: "Antisense oligonucleotides inhibit LIN28 binding to pre-let-7" Keystone Conference, "Noncoding RNAs in Development and Cancer", January 20-25, 2013, Vancouver, Canada) designed and tested methoxy antisense oligonucleotides (ASO) to specifically antagonize Lin28 from binding to the terminal loop region of pre-let-7 in order to elevate processing by Dicer and Drosha in order to prevent the cell from mature let-7 loss. These ASOs were tested by an RNA-based competition ELISA. Kd-values of a selection of tested ASO were determined by Surface Plasmon Resonance (SPR) measurements. The two best ASOs were tested in a biochemical Dicer assay using HPCL-MS for analysis to ensure a proper pre-let-7a-2 processing by Dicer in presence of ASO.

Yeom et al. (EMBO REPORTS, 12:7, 690-696, 2011) teaches a method for integrating single-molecule fluorescence microscopy and immunopurification.to investigate Lin28-mediated microRNA uridylation by TUT4 (terminal uridylyl transferase 4, polyU polymerase), which also regulates let-7 microRNA biogenesis. TUT4 immunoprecipitates together with fluorescent Cy5-labelled miRNA pre-let-7. The real-time analysis of the uridylation by the TUT4 immunoprecipitates suggests that Lin28 functions as a processivity factor of TUT4 in the Lin28/TUT4/let-7 complex.

WO 2009/048935 A2 discloses a method for promoting miRNA processing of pri-let-7 miRNA to mature miRNA in a human cancer cell, the method comprising contacting a cell with an agent inhibiting the activity or expression of Lin-28.

It is the objective of the present invention to provide an improved and high throughput screening (HTS)-suitabie-assay for identifying a compound modulating an interaction between two biomolecules such as e.g. polypeptides, sugars, polynucleotides, polyamines and lipids or between two domains of the same biomolecule. In particular it is an objective of the present invention to provide an improved HTS-suitable assay for identifying a compound modulating an interaction between polypeptides and polynucleotides, preferably microRNAs(miRNA).

This objective is solved according to the present invention by a method for identifying a compound modulating an interaction between two biomolecules or two domains of one biomolecule, the first biomolecule or first domain comprising at least one fluorophore donor and the second biomolecule or second domain comprising at least one fluorophore acceptor or a dark quencher, wherein the fluorophore donor and fluorophore acceptor or the fluorophore donor and dark quencher are spectrally paired such that the energy spectrum emitted by the fluorophore donor and the excitation energy spectrum of the fluorophore acceptor or the energy spectrum absorbed by the dark quencher overlap at least partially, comprising the steps of
(i) providing the first and second biomolecules or a biomolecule comprising said first and second domains,
(ii) providing a compound of interest,
(iii) contacting the first and second biomolecules or the biomolecule comprising the first and second domains with the compound of interest under conditions, that allow for FRET (Förster Resonance Energy Transfer) between the fluorophore donor and fluorophore acceptor or the fluorophore donor and the dark quencher,
(iv) identifying the compound of interest as a compound modulating, preferably inhibiting or enhancing an interaction between the two biomolecules or the two domains of the biomolecule if the FRET (Förster Resonance Energy Transfer) between the first and second biomolecules or the first and second domains differs in the presence of the compound of interest compared to the FRET between the first and second biomolecules or the first and second domains in the absence of the compound of interest.

The term "compound modulating an Interaction between two molecules or two domains of one biomolecule" as used herein is meant to encompass any type of compound, e.g. small or large molecular weight inorganic or organic compound, amino acid, peptide, polypeptide, lipid, (poly)nucleotide, etc., that is capable of at least partially (decreasing) or fully interrupting (inhibiting) the interaction between the biomolecules or domains, or that is capable of initiating or increasing the interaction between the biomolecules or domains. In other words, the compound will decrease, interrupt, initiate or increase binding between the biomolecules or domains. An interaction between two molecules is regularly understood to encompass covalent, ionic and "weak" bonds such as dipole-dipole interactions, ion-dipole interactions, London dispersion force, van der Waals interactions, charge transfer interactions and hydrogen bonding.

The term "biomolecule" as used herein indicates any molecule existing in a living cell and having a biological function in said cell as well as functional derivatives and fragments thereof. Functional derivatives and fragments of a biomolecule typically share at least 10, 20, 30 or 40 %, preferably at least 50, 60, 70 or 80 %, more preferably at least 90%, most preferably at least 95 % (percent) identity with the structure of the biomolecule in nature and feature the same or a substantially similar biological function as the biomolecule in nature. For example, structural identity for polynucleotides and polypeptides can be determined by standard algorithms that calculate the percentage identity of the sequences and for polynucleotides structural identity can also be determined by hybridization assays.

For polynucleotides the term "% (percent) identity" as known to the skilled artisan and used herein indicates the degree of relatedness among two or more nucleic acid molecules that is determined by agreement among the sequences. The percentage of "identity" is the result of the percentage of identical regions in.two or more sequences while taking into consideration the gaps and other sequence peculiarities. The identity of related nucleic acid molecules can be determined with the assistance of known methods. In general; special computer programs are employed that use algorithms adapted to accommodate the specific needs of this task. Preferred methods for determining identity begin with the generation of the largest degree of identity among the sequences to be compared. Preferred computer programs for determining the identity among two nucleic acid sequences comprise, but are not limited to, BLASTN (Altschul etal., J. Mol. Biol., 215, 403-410,1990) and LALIGN (Huang and Miller, Adv. Appl. Math., 12, 337-357, 1991). The BLAST programs can be obtained from the National Center for Biotechnology Information (NCBI) and from other sources (BLAST handbook, Altschul et al., NCB NLM NIH Bethesda, MD 20894).

The structural identity of related nucleic acids can also be characterized in that they have the ability to hybridize to a specifically referenced nucleic acid sequence, preferably under stringent conditions. Next to common and/or standard protocols in the prior art for determining the ability to hybridize to a specifically referenced nucleic acid sequence under stringent conditions (e.g. Sambrook and Russell, Molecular cloning: A laboratory manual (3 volumes), 2001), it is preferred to analyze and determine the ability to hybridize to a specifically referenced nucleic acid sequence under stringent conditions by comparing the nucleotide sequences, which may be found in gene databases (e.g. http://www.ncbi.nlm.-nih.gov/entrez/query.fcgi?db=nucleotide) with alignment tools, such as e.g. the above-mentioned BLASTN (Altschul et al., J. Mol. Biol., 215, 403-410,1990) and LALIGN alignment tools.

Preferably the ability of a nucleic acid to hybridize to a related nucleic acid derivative or fragment is confirmed in a Southern blot assay under the following conditions: 6x sodium chloride/sodium citrate (SSC) at 45°C followed by a wash in 0.2x SSC, 0.1 % SDS at 65°C.

The percentage identity of amino acid molecules in nature to functional fragments or derivatives thereof can be determined with the assistance of known methods. In general, special computer programs are employed that use algorithms adapted to accommodate the specific needs of this task. Preferred methods for determining identity begin with the generation of the largest degree of identity among the sequences to be compared. Preferred computer programs for determining the identity among two amino acid sequences comprise, but are not limited to, TBLASTN, BLASTP, BLASTX or TBLASTX (Altschul et al., J. Mol. Biol., 215, 403-410, 1990). The BLAST programs can be obtained from the National Center for Biotechnology Information (NCBI) and from other sources (BLAST handbook, Altschul et al., NCB NLM NIH Bethesda, MD 20894).

The term "functional derivative" of a compound, in particular a polypeptide or polynucleotide mentioned herein is meant to include any polypeptide, polynucleotide or fragment thereof that has been chemically or-genetically modified in its amino acid or nucleotide sequence, e.g. by addition, substitution and/or deletion of amino acid or nucleotide residue(s) and/or has been chemically modified in at least one of its atoms and/or functional chemical groups, e.g. by additions, deletions, rearrangement, oxidation, reduction, etc. as long as the derivative still has at least one of the biological activities of the original polypeptide or polynucleotide in nature to a measurable extent, e.g. at least about 1 to 10 % of the biological activity of the original unmodified polypeptide or polynucleotide.

In a preferred embodiment each of the first and second biomolecules or the biomolecule with the first and second domains is selected from the group consisting of polypeptides, sugars, polynucleotides, polyamines and lipids.

Even though the invention is applicable to any type of biomolecule capable of interacting, i.e. binding to some extent to any other type of biomolecule or capable of interacting with at least two domains on the same biomolecule, the present invention is preferably directed to a method, wherein the first biomolecule is selected from the group consisting of polypeptides interacting with polynucleotides and the second biomolecule is selected from the group consisting of polynucleotides, preferably microRNA (miRNA).

In a more preferred embodiment the polypeptide interacting with polynucleotides is selected from any one of the polypeptides listed in Table 1, preferably a polypeptide interacting with miRNA, preferably selected from
(i) the group consisting of FOX protein family members (Fox1: NM_001142333.1 → NP_001135805.1; Fox2: NM_001031695.2 → NP_001026865.1; Fox3: NM_001082575.2 → NP_001076044.1), DAZ protein family members (BOLL: NM_033030.5 → NP_149019.1; DAZL: NM_001277863.1 → NP_001264792.1; DAZ1: N_004081.5 → NP_004072.3), DND1 (NM_194249.2 → NP_919225.1), ELAV1 and ELAV-like protein family members (preferably ELAV1: NM_001419.2 → NP_001410.2), GW182 protein family members (GW182: NM_001011116.1 → NP_001011116.1; TNRC6B: NM_001024843.1 → NP_001020014.1; TNRC6C: NM_001142640.1 → NP_001136112.1), hnRNP protein family members, preferably hnRNPA1 (NM_002136.2 → NP_002127.1), IGF2BP1 (NM_001160423.1 → NP_001153895.1), PABP protein family members, preferably PABPC1 (NM_002568.3 → NP_002559.2), SFRS1 (NM_001078166.1 → NP_001071634.1), DGCR8 (NM_001190326.1 → NP_001177255.1), Dicer1 (NM_001195573.1 → NP_001182502.1), Drosha (NM_001100412.1 → NP_001093882.1), TARBP2 (NM_004178.4 → NP_004169.3), FMR1 (NM_001185075.1 → NP_001172004.1), FXR1 (NM_001013438.2 → NP_001013456.1), FXR2 (NM_004860.3 → NP_004851.2), KHSRP (NM_003685.2 → NP_003676.2), STAR (NM_000349.2 → NP_000340.2), AGO protein family members, preferably AGO1 (NM_012199.2 → NP_036331.1), TRIM proteins with NHL domain, preferably TRIM1 (NM_012216.3 → NP_036348.2), ZFP36 (NM_003407.3 → NP_003398.2), PUM1 (NM_001020658.1 → NP_001018494.1), PUM2 (NM_015317.1 → NP_056132.1), DDX5 (NM_004396.3 → NP_004387.1), DDX6 (NM_001257191.1 → NP_001244120.1), DDX17 (NM_001098504.1 → NP_001091974.1), DDX20 (NM_007204.4 → NP_009135.4), DDX42 (NM_007372.2 → NP_031398.2), MOV10 (NM_001130079.1 (mRNA)→ NP_001123551.1), Lin28a (NM_024674.4→ NP_078950.1) and Lin28b (NM_001004317.3 → NP_001004317.1),
(ii) preferably the group consisting of hnRNPA1, KHSRP, Fox1 and Fox2, Lin28a and Lin28b,
(iii) more preferably the group consisting of Lin28b or Lin28a.

The above and below mentioned reference numbers refer to the NCBI (National Center for Biotechnology Information) reference number code (www.ncbi.nlm.nih.gov).

The Lin28, also referred to as Lin-28 homologue a (Lin 28a) and its homologue Lin28b are proteins encoded by the *LIN28* genes [NM_024674.4 → NP_078950.1 and NM_001004317.3 → NP_001004317.1; for a review see: Viswanathan & Daley; Lin28: A MicroRNA Regulator with a Macro Role, Cell, 2010/ Lehrbach & Miska, Regulation of pre-miRNA Processing, Chapter 7, Springer 2010/ Thornton et al. How does Lin28 let-7 control development and disease, Trends in Cell Biology, 2012)].

As defined herein, the term Lin28 is meant to encompass any Lin28 homologue such as homologues Lin28a and Lin28b as well as functional derivatives and fragments thereof as defined above.

Human Lin28a sequence information is available under ref. nos. 79729, UniProt: Q9H9Z2, NM_024674 (mRNA), NP_078950 and mouse Lin28a sequence information is available under ref. nos. 83557, UniProt: Q8K3Y3, NM_145833 (mRNA), NP_665832. The cDNA-Sequence of human Lin28b from NM_001004317.3 is shown below as SEQ ID NO: 1. LIN28 encodes a protein that binds to and enhances the translation of the IGF-2 (insulin-like growth factor 2) mRNA. Lin28 has also been shown to bind to the let-7 pre-miRNA and block production of the mature let-7 microRNA in embryonic stem cells. In pluripotent embryonic carcinoma cells LIN28 is localized in the ribosomes, P-bodies and stress granules. LIN28 regulates the self-renewal of stem cells. In nematodes, the LIN28 homolog lin-28 is a heterochronic gene that determines the onset of early larval stages of developmental events in *Caenorhabditis elegans* by regulating the self-renewal of nematode stem cells in the skin (called seam cells) and vulva (called VPCs) during development. In mice, LIN28 is highly expressed in mouse embryonic stem cells and during early embryogenesis: LIN28 is also highly expressed in human embryonic stem cells and has been used to enhance the efficiency of the formation of induced pluripotent stem (ips) cells from human fibroblasts. LIN28 overexpression in mice has been shown to cause gigantism and a delay in puberty onset, consistent with human genome-wide association studies suggesting that polymorphisms in the human LIN28B gene are associated with human height and puberty timing. Mutations in LIN28B have been found to be associated with precocious puberty. LlN28 can also regulate glucose homeostasis in mammals by increasing insulin-PI3K-mTOR-signaling and insulin sensitivity, thereby promoting resistance to high fat diet-induced obesity and type 2 diabetes. In summary, Lin28 homologues are highly relevant targets for designing and screening medically useful compounds, i.e. compounds modulating the interaction of Lin28 homologues with miRNAs and other regulators of cell growth, cell differentiation and cell homeostasis.

In a most preferred embodiment the polypeptide interacting with miRNA for practicing the method of the present invention is Lin28, preferably selected from the group consisting of mammalian, preferably human, non-human primate, rodent, monkey, mouse, rat, chicken, pig, guinea pig Lin28a and Lin28b, most preferably human Lin28a or Lin28b or a functional fragment or functional derivative thereof.

In a further preferred embodiment the polynucleotide for practicing the method of the invention is a pri- or pre-miRNA or mature miRNA, preferably selected from the group consisting of hsa-let-7a-1, hsa-let-7a-2, hsa-let-7a-3 hsa-let-7b, hsa-let-7c, hsa-let-7d, hsa-let-7e, hsa-let-7f-1, hsa-let-7f-2, hsa-let-7g, hsa-let-7i and functional derivatives or fragments thereof.

Sequence information for the above and further preferred miRNA embodiments can be found on the miRBase site (http://www.mirbase.org/) and is well-known to the skilled person:
hsa-let-7a-1 MI0000060, hsa-let-7a-2 MI0000061; hsa-let-7a-3 MI0000062; hsa-let-7b MI0000063; hsa-let-7c MI0000064; hsa-let-7d MI0000065; hsa-let-7e MI0000066; hsa-let-7f-1 MI0000067; hsa-let-7f-2 MI0000068; hsa-let-7g MI0000433; hsa-let-7i MI0000434; hsa-mir-106a MI0000113; hsa-mir-106b MI0000734; hsa-mir-107 MI0000114; hsa-mir-10a MI0000266; hsa-mir-155 MI0000681; hsa-mir-181b-1 MI0000270; hsa-mir-181b-2 MI0000683; hsa-mir-181c MI0000271; hsa-mir-181d MI0003139; hsa-mir-10b MI0000267; hsa-mir-18a MI0000072; hsa-mir-18b MI0001518; hsa-mir-19a MI0000073; hsa-mir-19b-1 MI0000074; hsa-mir-19b-2 MI0000075; hsa-mir-19a MI0000073; hsa-mir-19b-1 MI0000074; hsa-mir-19b-2 MI0000075; hsa-mir-32 MI0000090; hsa-mir-20a MI0000076; hsa-mir-20b MI0001519; hsa-mir-21 MI0000077; hsa-mir-34a MI0000268; hsa-mir-34b MI0000742; hsa-mir-34c MI0000743; hsa-mir-9-1 MI0000466; hsa-mir-9-2 MI0000467; hsa-mir-9-3 MI0000468; hsa-mir-98 MI0000100.

As used herein the term miRNA is generally meant to include any pri- and pre-precursor molecules of the mature miRNAs, the mature miRNA as well as any functional fragments and derivatives thereof as defined above.

A fluorophore is a fluorescent chemical compound that can re-emit light upon light excitation. Fluorophores typically contain several combined aromatic groups, or planar or cyclic sub-structures with several π bonds. A fluorophore donor according to the present invention absorbs light energy of a specific wavelength (donor excitation spectrum) and emits light at a longer wavelength (donor emission spectrum). The flurophore donor "donates" the excitation energy for the fluorophore acceptor (acceptor excitation spectrum), a fluorescent chemical that re-emits the accepted excitation energy at a longer wavelength (acceptor emission spectrum).

A dark quencher for practicing the method of the present invention is a substance that at least partially absorbs excitation energy from the fluorophore donor and dissipates the energy as heat. Black hole quencher (BHQ™) dyes are preferred dark quenchers for practicing the method of the present invention.

For practicing the method of the invention the fluorophore donor and fluorophore acceptor or the fluorophore donor and dark quencher are preferably spectrally paired, meaning that they are selected such that the energy spectrum emitted by the fluorophore donor (fluorophore donor emission spectrum) and the energy spectrum absorbed by the fluorophore acceptor (fluorophore acceptor excitation spectrum) or the energy absorbed by the dark quencher (quencher absorption spectrum) overlap at least partially.

Preferably, the fluorophore donor is capable of absorbing radiation having a wavelength between about 300 nm to 900 nm, more preferably between 350 nm and 800 nm and is capable of transferring energy to the fluorophore acceptor or dark quencher acceptor.

Preferably the acceptor fluorophore or dark quencher is capable of absorbing radiation having a wavelength between about 300 nm to 900 nm, more preferably between 350 nm and 800 nm, and has an excitation spectra overlapping with the emission of the fluorophore donor, such that the energy emitted by the donor can excite the acceptor.

Preferably, the acceptor fluorophore absorbs light at a wavelength which is at least 10 nm higher and more preferably at least 20 nm higher, most preferably at least 30 nm higher than the maximum absorbance wavelength of the donor fluorophore. Preferably, the dark quencher acceptor absorbs at least 30% of the emitted wavelength by the fluorophore donor, more preferably at least 50%, most preferably all of the emitted wavelength.

In a further preferred embodiment the spectral overlap for the fluorophore donor emission spectrum and the dark quencher absorption spectrum, preferably black hole quencher (BHQ™) absorption spectrum is at least 30, preferably at least 50, 60 or 70, more preferably at least 80, most preferably at least 95 or 100 %.

In a preferred embodiment the at least one fluorophore donor for practicing the method of the invention is selected from fluorescent proteins and small fluorescent dye molecule,
(i) preferably fluorescent proteins selected from the group consisting of
   (i.1) blue fluorescent proteins, preferably selected from the group consisting of EBFP, EBFP2, Azurite and mTagBFP,
   (i.2) cyan fluorescent proteins, preferably selected from the group consisting of ECFP, mECFP, Cerulean, mTurquoise, CyPet, AmCyan1, Midori-Ishi Cyan, TagCFP and mTFP1 (Teal),
   (i.3) yellow fluorescent proteins, preferably selected from the group consisting of EYFP, Topaz, Venus, mCitrine, YPet, TagYFP, PhiYFP, ZsYellow1 and mBanana,
   (i.4) orange fluorescent proteins, preferably selected from the group consisting of Kusabira Orange, Kusabira Orange2, mOrange, mOrange2, dTomato, dTomato-Tandem, TagRFP, TagRFP-T, DsRed, DsRed2, DsRed-Express (T1), DsRed-Monomer and mTangerine,
   (i.5) red fluorescent proteins, preferably selected from the group consisting of mRuby, mApple, mStrawberry, AsRed2, mRFP1, JRed, mCherry, HcRed1, mRaspberry, dKeima-Tandem, HcRed-Tandem, mPlum and AQ143, and
   (i.6) green fluorescent proteins (GFP), preferably selected from the group consisting of EGFP, Emerald, Superfolder GFP, Azami Green, mWasabi, TagGFP, TurboGFP, AcGFP, ZsGreen and T-Sapphire,
      more preferably green fluorescent proteins (i.6) and yellow fluorescent proteins (i.3), most preferably EGFP;
(ii) preferably small fluorescent dye molecules selected from the group consisting of
   ii.1) acridines, preferably acridine orange or acridine yellow,
   (ii.2) cyanines, preferably Cy2, Cy3, Cy3B, Cy3.5, Cy5, Cy5.5, Cy7,
   (ii.3) fluorones, preferably Fluorescein, Carboxyfluorescein, Dichlorofluorescein, Eosin, Eosin B, Eosin Y or Erythrosine,
   (ii.4) oxazines, preferably Cresyl violet, Nile blue or Nile red,
   (ii.5) phenanthridines, preferably Ethidium bromide, Gelred or Propidium iodide, and
   (ii.6) rhodamines, preferably Rhodamine, Rhodamine 123, Rhodamine 6G, Rhodamine B, Auramine, Sulforhodamine 101, Sulforhodamine B or Texas red,
(iii) more preferably cyanine and rhodamine dyes.

The above fluorophore donors are common knowledge in the art. For example, they are cited under e.g. fluirophores.org.

The most preferred fluorophore donor is Green fluorescent protein (GFP), which features 238 amino acids and exhibits bright green fluorescence (eGFP, Exₘₐₓ=488nm, Emₘₐₓ=509 nm) upon excitation. The term Green fluorescent protein(s) as used herein is generally meant to include its derivatives, preferably those selected from the group consisting of EGFP, Emerald, Superfofder GFP, Azami Green, mWasabi, TagGFP, TurboGFP, AcGFP, ZsGreen and T-Sapphire,

In a preferred embodiment the at least one fluorophore acceptor is selected from the group consisting of
(i) acridines, preferably acridine orange or acridine yellow,
(ii) cyanines, preferably Cy2, Cy3, Cy3B, Cy3.5, Cy5, Cy5.5 or Cy7,
(iii) fluorones, preferably Fluorescein, Carboxyfuorescein, Dichlorofluorescein, Eosin, Eosin B, Eosin Y or Erythrosine,
(iv) oxazines, preferably Cresyl violet, Nile blue or Nile red,
(v) phenanthridines, preferably ethidium bromide, Gelred or propidium iodide, and
(vi) rhodamines, preferably Rhodamine, Rhodamine 123, Rhodamine 6G, Rhodamine B, Auramine, Sulforhodamine 101, Sulforhodamine B or Texas red,
preferably cyanines (ii), more preferably Cy3.

Cyanine 3 (Cy3) is a small molecule fluorophore featuring a bright pink fluorescence (Exₘₐₓ=550nm, Emₘₐₓ=570nm) upon excitation.

The preferred Cy3 compound for demonstrating the method of the invention as illustrated in the examples is attached to a ribonucleotide in the miRNA by reaction with the azide (1), which has the following formula (I):

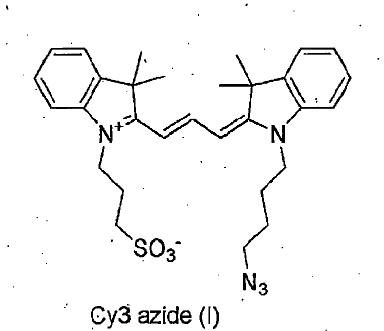

In another preferred embodiment the at least one dark quencher is selected from the group consisting of Dabcyl, Dabsyl, Black Hole Quenchers (BHQ™) dyes, preferably BHQ-0, BHQ-1, BHQ-2 or BHQ-3, QXL quenchers, preferably QXL 490, QXL 570, QXL 610, QXL 670, or QXL 680 Iowa Black quenchers, preferably Iowa black FQ or Iowa Black RQ, and IRDyes, preferably IRDye 8b0, IRDye 800CW, IRDye 800RS, IRDye 680, IRDye 680LT, IRDye 700, or IRDye 700DX, more preferably Black Hole Quenchers (BHQ™) dyes, most preferably BHQ-1.

The term Black Hole Quenchers (BHQ™) dyes as used herein is meant to include any functional derivatives. Black hole quencher 1 (BHQ1) is a small molecule that absorbs fluorescence when excited (broad range, Ex=480 to 580nm). The preferred BHQ1 functional derivative for demonstrating the method of the invention as illustrated in the examples is attached to a ribonucleotide in the miRNA by reaction with the azide (II), which has the following formula (II):

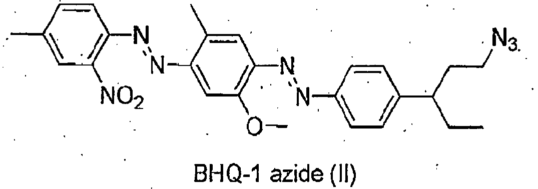

The skilled person can readily select spectrally paired fluorophore donors and fluorophore acceptors or spectrally paired fluorophore donors and dark quenchers based on the known or easily measurable emission spectra for the donors, the known or easily measurable excitation spectra of the acceptors and the known or easily measurable absorption spectra of the dark quenchers.

In a preferred embodiments the spectrally paired fluorophore donor and fluorophore acceptor or the spectrally paired fluorophore donor and dark quencher for practicing the method of the invention are selected from the group consisting of
(i) protein-protein pairs, preferably selected from the group consisting of ECFP-Citrine, ECFP-Venus, Cerulean-Citrine, Cerulean-Venus, Cerulean-Ypet, Cerulean-YFP, CyPet-EYFP, CyPet-Venus, CyPet-YPet, CyPet-Citrine, mTurquoise-Venus, mTurquoise-Ypet, mTurquoise-Citrine, ECFP-EYFP, TagGFP-TagRFP, mTFP1-Citrine, Citrine-mKate2, mTurquoise1-SEYFP, mTurquoise2-SEYFP and clover-mRuby2,
(ii) protein-organic dye pairs, preferably selected from the group consisting of EGFP-mCherry, SYFP2-mStrawberry, mTFP1-mOrange, Clover-mCherry, GFP-Cy3, YFP-Cy3, ECFP-BHQ-0, EYFP-BHQ-2, EGFP-Cy3 and EGFP-BHQ-1,
(iii) organic dye-organic-dye pairs, preferably selected from the group consisting of mOrange-mCherry, Alexa4B8-Alexa555, Alexa488-Cy3, Alexa 568-Alexa633, Cy3-Cy5, Alexa 488-Alexa514, Alexa488-Alexa532, Alexa488-546, Alexa488-610, Alexa647-Alexa 680, Alexa647-Alexa680, Alexa647-Aelxa700, Alexa647-Alexa750, BHQ-1-FAM, BHQ-1-TET, BHQ-1-JOE, BHQ-1-HEX, BHQ-1-Oregon green, BHQ-2-TAMRA, BHQ-2-ROX, BHQ-2-Cy3, BHQ-2-Cy3.5, BHQ-2-CAL Red, BHQ-2-Red 640, BHQ-3-Cy5, or BHQ-3-Cy5.5, Dabcyl-Edans and Dabsyl-Edans, fluorescine.

In a further preferred embodiment the first biomolecule for practicing the invention is Lin28(a or b) and the second biomolecule is (pri or pre-)let-7a-2 or functional derivatives or fragments of these biomolecules. A preferred embodiment of a functional derivative of prelet-7a-2 is shown in the examples below and designated truncated pre-let-7a-2.

Pre-let-7a-2 is a member of the let-7 miRNA family. It is an approx. 67 nucleotides long non-coding miRNA precursor which is produced by processing pri-let-7a-2, a long transcript of several hundred nucleotides in length, so-called pri-miRNA. The pre-let-7 family is presently composed of 12 members (12 pre-cursors, but only 9 mature miRNAs).

As already generally indicated above for miRNAs in general, the terms let-7, let-7a, let-7a-2, etc. include the corresponding pri- and pre-precursors, mature miRNAs as well as any functional derivatives or fragments of these biomolecules, meaning pre- and pri-precursors, mature miRNA as well as any derivatives or fragments of these biomolecules having the same or a substantially similar biological activity in a living cell as the corresponding native miRNA in said cell. Preferably, functional derivatives of let-7, let-7a, let-7a-2, etc. comprise at least the tetranucleotide hairpin binding region for Lin28, more preferably the tetrapeptide hairpin binding region GGAG.

It is preferred that the fluorophore donor of the first biomolecule or first domain of the biomolecule, preferably Lin28, is at least one green or yellow fluorescent protein and/or that the fluorophore acceptor of the second biomolecule or of the second domain of the biomolecule, preferably an miRNA, more preferably pri- or pre-let-7a-2, is at least one Cy3.

It is also preferred that the fluorophore donor of the first biomolecule or first domain of the biomolecule, preferably Lin28, is at least one green or yellow fluorescent protein and/or that the dark quencher of the second biomolecule or of the second domain of the biomolecule, preferably an miRNA, more preferably pri- or pre-let-7a-2, is at least one dark quencher, preferably at least one black hole quencher (BHQ™), most preferably at least one BHQ-1 or at least one BHQ-2 or a functional derivative tehreof.

In a most preferred embodiment for practicing the method of the present invention the at least one fluorophore donor, preferably at least one GFP, more preferably at least one EGFP, is bound to the *N*- and/or C-terminal position of Lin28a or Lin28b or a functional derivative or fragment thereof, and the at least one fluorophore acceptor, preferably Cy3 and/or at least one dark quencher, preferably black hole quencher (BHQ™), more preferably BHQ1 is bound to at least one of positions 1 to 67, preferably 5 to 60, more preferably 10 to 57, most preferably one of positions 10, 19, 34 and 57 of pri- or pre-let-7a-2 or a functional fragment or derivative thereof.

Preferred examples of pri- or pre-let-7a-2 mono- and bis Cy3- or BHQ1-labeled embodiments are: Position 19 mono-Cy3-labeled (pre)-let-7a-2, Position 19 & 34-bis-Cy3-labeled (pre)-let-7a-2, Position 19 mono-BHQ1-labeled (pre)-let-7a-2, Position 10 & 34-bis-BHQ1-labeled (pre)-let-7a-2 and Position 19 & 34-bis-BHQ1-labeled (pre)-let-7a-2 as well as truncated fragments thereof. In a more preferred embodiment the miRNA is let-7a-2 full sequence (nucleotides 1 to 67) or the truncated sequence thereof (nucleotides 10 to 57).

In another most preferred embodiment for practicing the method of the present invention the at least one fluorophore donor, preferably GFP, more preferably EGFP is bound to the *N-* and/or *C-*terminal position of Lin28a or Lin28b and the at least one fluorophore acceptor, preferably Cy3 and/or at least one dark quencher, preferably a black hole quencher (BHQ™), more preferably BHQ-1 is bound to at least one, preferably at least two of positions 10, 19, 34 and 57 of pre-let-7a-2, preferably to position 10 and 19 or positions 10 and 34.

For practicing the method of the invention the contacting of the first and second biomolecules or the biomolecule comprising the first and second domains with the compound of interest must be under conditions that allow for FRET (Förster Resonance Energy Transfer) between the fluorophore donor and fluorophore acceptor or the fluorophore donor and the dark quencher. Conditions for molecular interactions between biomolecules such as e.g. polypeptides and nucleotides are common knowledge in the art. The conditions should be chosen to facilitate covalent and/or preferably non-covalent binding of the biomolecules of interest so that the fluorophore donor(s) and the fluorophore acceptor(s) or dark quencher(s) come into a close distance of about 10 or less nanometers. And the conditions should preferably not denature the biomolecules or otherwise affect their natural structure and function. Also, the conditions should not reduce the FRET signal.

When practicing the method of the invention the compound of interest is identified as a compound modulating, preferably inhibiting or enhancing an interaction between the two biomolecules or the two domains of the biomolecule, if the FRET (Forster Resonance Energy Transfer) between the first and second biomolecules or the first and second domains differs in the presence of the compound of interest compared to the FRET between the first and second biomolecules or the first and second domains in the absence of the compound of interest.

If the compound inhibits the biomolecule interaction, the distance of the fluorophore donor and the fluorophore acceptor or the fluorophore donor and the dark quencher will, increase, thus decreasing or abolishing the FRET. If the compound enhances the biomolecule interaction the FRET will increase. For positively identifying the compound of interest as a modulator of the first and second molecule or first and second domain the FRET in the presence and absence of the compound of interest should be compared. Preferably, compounds known to modulate the interaction of the first and second biomolecules or the first and second domains are used as further reference and compared to the FRET results with and without the compound of interest.

The method of the invention and its preferred embodiments have a number of advantages over the screening methods commonly used to identify compounds of interest that modulate, in particular reduce, inhibit, initiate or increase biomolecule interaction. The inventive method is simple, it only requires contacting the compound of interest with spectrally paired biomolecules or domains under conditions suitable for biomolecule interaction and FRET, and the readout is spectral and therefore easily automated. The method is particularly suitable for screening polypeptide-miRNA interactions, which represent important targets for medical treatment.

The method is particularly effective for Lin28 polypeptid - pre-let-7 miRNA family member interactions. In particular the combination of GFPs or YFPs with Cy3 or GFPs or YFPs with BHQ1 or BHQ-2 or functional derivatives thereof is very FRET effective in Lin28-miRNA systems. It was surprisingly found that double-labeling miRNA with two or even more fluorophores, preferably Cy3 greatly enhances FRET (around two times). Using dark quenchers instead of fluorophore acceptors such as Cy3 suppressed spectral bleed through significantly. And also surprisingly, the use of a black hole quencher, preferably BHQ1, abolished bleed through and maximized the FRET efficiency up to 2.5 times.

A further independent aspect of the present invention, that is generally applicable to any type of molecule and which is not limited to biomolecules relates to a method for identifying a compound modulating an interaction between two molecules or two domains of one molecule, the first molecule or first domain comprising at least one fluorophore donor and the second molecule or second domain comprising at least one fluorophore acceptor or a dark quencher, wherein the fluorophore donor and fluorophore acceptor or the fluorophore donor and dark quencher are spectrally paired such that the energy spectrum emitted by the fluorophore donor and the excitation energy spectrum of the fluorophore acceptor or the energy spectrum absorbed by the dark quencher overlap at least partially, comprising the steps of
(i) providing the first and second molecules or a molecule comprising said first and second domains,
(ii) providing a compound of interest,
(iii) contacting the first and second molecules or the molecule comprising the first and second domains with the compound of interest under conditions that allow for FRET (Förster Resonance Energy Transfer) between the fluorophore donor and fluorophore acceptor,
(iv) identifying the compound of interest as a compound modulating, preferably inhibiting or enhancing an interaction between the two molecules or the two domains of the molecule if the FRET (Förster Resonance Energy Transfer) between the first and second molecules or the first and second domains differs in the presence of the compound of interest compared to the FRET between the first and second molecules or the first and second domains in the absence of the compound of interest,
wherein the fluorophore donor is a green fluorescent protein, preferably EGFP or a yellow fluorescent protein, the fluorophore acceptor is cy3 and the dark quencher is a black hole quencher (BHQ™), preferably BHQ-1 or BHQ-2 or a functional derivative thereof.

Another independent aspect of the present invention that is not limited to biomolecules in particular rotates to the use of a fluorophore donor / acceptor or a fluorophore donor / dark quencher pair for FRET measurement in general, wherein the fluorophore donor is a green fluorescent protein, preferably EGFP or a yellow fluorescent protein, the fluorophore acceptor is cy3 and the dark quencher is a black hole quencher (BHQ™), preferably BHQ-1 or BHQ-2 or a functional derivative thereof

### Figures

**Fig. 1** is a graph showing the percentage of the GFP signal quenched at 507nm depending on the concentration of position 10-cy3-labeled truncated pre-let-7a-2 (nucleotides 10 to 57 of SEQ ID NO: 2 above). At a 80nM concentration of truncated pre-let-7a-2 there is a FRET of 12.3%.
**Fig. 2** is a graph showing the percentage of the GFP signal quenched at 507nm depending on the concentration of position 19-cy3-labeled truncated pre-let-7a-2 (nucleotides 10 to 57 of SEQ ID NO: 2 above). At a 80nM concentration of truncated pre-let-7a-2 there is a FRET of 20.9%.
**Fig. 3** is a graph showing the percentage of the GFP signal quenched at 507nm depending on the concentration of position 34-cy3-labeled truncated pre-let-7a-2 (nucleotides 10 to 57 of SEQ ID NO: 2 above). At a 80nM concentration of truncated pre-let-7a-2 there is a FRET of 21.5%.
**Fig. 4** is a graph showing the percentage of the GFP signal quenched at 507nm depending on the concentration of position 57-cy3-labeled truncated pre-let-7a-2 (nucleotides 10 to 57 of SEQ ID NO: 2 above). At a 80nM concentration of truncated pre-let-7a-2 there is a FRET of 16%.
**Fig. 5** is a graph showing the percentage of the GFP signal quenched at 507nm depending on the concentration of positions 10 and 19 bis-cy3-labeled truncated pre-let-7a-2 (nucleotides 10 to 57 of SEQ ID NO: 2 above). At a 80nM concentration of truncated pre-let-7a-2 there is a FRET of 32.9%.
**Fig. 6** is a graph showing the percentage of the GFP signal quenched at 507nm depending on the concentration of positions 19 and 34 bis-cy3-labeled truncated pre-let-7a-2 (nucleotides 10 to 57 of SEQ ID NO: 2 above). At a 80nM concentration of truncated prelet-7a-2 there is a FRET of 37.2%.
**Fig. 7** is a graph comparing FRET based on the quenched GFP signal with truncated pre-let-7a-2 mono- and bis-labeled with cy3 in different positions.
**Fig. 8** is a graph showing the percentage of the GFP signal quenched at 507nm depending on the concentration of position 10-BHQ-labeled truncated pre-let-7a-2 (nucleotides 10 to 57 of SEQ ID NO: 2 above). At a 80nM concentration of truncated pre-let-7a-2 there is a FRET of 27.0%.
**Fig. 9** is a graph showing the percentage of the GFP signal quenched at 507nm depending on the concentration of position 19-BHQ-labeled truncate pre-let-7a-2 (nucleotides 10 to 57 of SEQ ID NO: 2 above). At a 80nM concentration of truncated pre-let-7a-2 there is a FRET of 45.2%.
**Fig. 10** is a graph showing the percentage of the GFP signal-quenched at 507nm depending on the concentration of position 34-BHQ-tabeled truncated pre-let-7a-2 (nucleotides 10 to 57 of SEQ ID NO: 2 above). At a 80nM concentration of truncated pre-let-7a-2 there is a FRET of 26.4%.
**Fig. 11** is a graph showing the percentage of the GFP signal quenched at 507nm depending on the concentration of positions 10 and 19 bis-BHQ-labeled truncated pre-let-7a-2 (nucleotides 10 to 57 of SEQ ID NO: 2 above). At a 80nM concentration of truncated pre-let-7a-2 there is a FRET of 30.3%.
**Fig. 12** is a graph showing the percentage of the GFP signal quenched at 507nm depending on the concentration of positions 19 and 34 bis-BHQ-labefed truncated pre-let-7a-2 (nucleotides 10 to 57 of SEQ ID NO: 2 above). At a 80nM concentration of truncated pre-let-7a-2 there is a FRET of 42.4%.
**Fig. 13** is a graph comparing FRET based on the quenched GFP signal with truncated pre-let-7a-2 mono- and bis-labeled with BHQ-1 in different positions.

In the following the embodiments of the present invention will be illustrated by examples, none of which are intended to be interpreted as limiting to the scope of the claims as appended.

### Examples

For sequence information regarding Lin28 and let-7a-2 reference is made to the following sequence listings:
SEQ ID NO: 1: cDNA-Sequence Lin28b from NM_001004317.3: 
ATGGCCGAAGGCGGGGCTAGCAAAGGTGGTGGAGAAGAGCCCGGGAAGCTGCCGGA
GCCGGCAGAGGAGGAATCCCAGGTTTTGCGCGGAACTGGCCACTGTAAGTGGTTCAA
TGTGCGCATGGGATTTGGATTCATCTCCATGATAAACCGAGAGGGAAGCCCCTTGGAT
ATTCCAGTCGATGTATTTGTACACCAAAGCAAACTATTCATGGAAGGATTTAGAAGCCT
AAAAGAAGGAGAACCAGTGGAATTCACATTTAAAAAATCTTCCAAAGGCCTTGAGTCAA
TACGGGTAACAGGACCTGGTGGGAGCCCCTGTTTAGGAAGTGAAAGAAGACCCAAAG
GGAAGACACTACAGAAAAGAAAACCAAAGGGAGATAGATGCTACAACTGTGGTGGCCT
TGATCATCATGCTAAGGAATGTAGTCTACCTCCTCAGCCAAAGAAGTGCCATTACTGTC
AGAGCATCATGCACATGGTGGCAAACTGCCCACATAAAAATGTTGCACAGCCACCCGC
GAGTTCTCAGGGAAGACAGGAAGCAGAATCCCAGCCATGCACTTCAACTCTCCCTCGA
GAAGTGGGAGGCGGGCATGGCTGTACATCACCACCGTTTCCTCAGGAGGCTAGGGCA
GAGATCTCAGAACGGTCAGGCAGGTCACCTCAAGAAGCTTCCTCCACGAAGTCATCTA
TAGCACCAGAAGAGCAAAGCAAAAAGGGGCCTTCAGTTCAAAAAAGGAAAAAGACATA
A
SEQ ID NO: 2: sequence of pre-let-7a-2:

U1GAGGUAGUA10GGUUGUAUAG20UUUAGAAUUA30CAUCAAGGGA40GAUAACUGU
A50CAGCCUCCUA60GCUUUCC

The subscripts in SEQ ID NO: 2 indicate the positions referenced above, in the examples below and the claims as appended. The tetranucleotide hairpin binding region GGAG of pre-let-7a-2 for Lin 28 is underlined.

### Example 1: GFP-tagged lin28

### Cloning pEGFP-C2 Lin28b

Lin28b cDNA was amplified from Lin28B Human cDNA ORF Clone (Origene) and cloned into the SacI and Sacll sites of pEGFP-C2 Vector (BD Biosciences Clontech). Primers used for cloning are: forward primer FW: TCGAGCTCAATGGCCGAAGGCGGGGCTA reverse primer RV: ATCCGCGGGTTATGTCTTTTTCCTTTTTTG AACTGAAGGCCCC.

### Recombinant Protein overexpression

For recombinant protein expression 160'000 HEK 293T cells/well (ATCC ATCC^{®} CRL-11268™) were seeded in a 6well plate and 320 ng pEGFP-C2-Lin28b fusion plasmid was complexed with jetPEI according to manufacturer's procedure and transfected. 72h after transfection cells were checked for fluorescent protein overexpression and lysed in 500ul buffer containing 50mM Tris, 0.1 mM ZnCl2, 200mM MgCl2, 0,5 % Triton X-100 and 0.5mM TCEP. Lysis buffer was spun for 15min at 13'000 rpm and supernatant was used for further FRET assay procedures.

### Generating stable cell lines

For stable overexpression of EGFP-C2-Lin28b fusion plasmid HEK 293T cells were transfected as described above. 16h post transfection medium was exchanged to medium supplemented with 1 g/l neomycin (G418, Invitrogen). Medium was exchanged every second day for 14 days. Subsequently, stable cells were trypsinized and diluted to 150 cells/ml. Monoclonal cultures were grown for two weeks in a 96 well plate and checked for proper EGFP and Lin28b overexpression.

### Example 2: Synthesis of Cy3 and BHQ-1 labeled truncated pre-let-7a-2

Cy3 azide was prepared as follows:

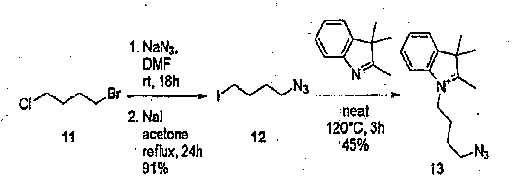

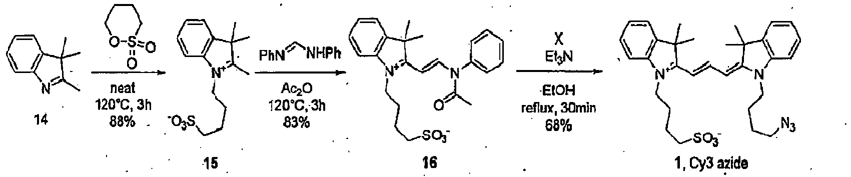

1-Azido-4-iodobutane 12 (148759-55-1) was prepared following the procedure of Yao et al., J. Org. Chem. 2004, 69, 1720-1722.To a solution of 1-bromo-4-chlorobutane 11 (25g, 146 mmol, 1 equiv.) in 250 mL of DMF was added sodium azide (9.5g, 146 mmol, 1 equiv.). After stirring (20 h) at room temperature, the reaction mixture was poured into 1000 mL of water and extracted three times with Et₂O (200 mL). The combined organic layers were washed twice with water (100 mL), once with brine (100 mL), dried over Na₂SO₄, filtered and evaporated to dryness. The residue was dissolved in acetone (300 mL) in a 500 mL round bottom flask and sodium iodide (43.8 g, 292 mmol, 2 equiv.) was added. The reaction mixture was stirred for 48 h at reflux, cooled to room temperature, poured into 1000 mL of water and extracted three times with Et₂O (200 mL). The combined organic layers were dried over Na₂SO₄, filtered and evaporated to dryness to give 1-Azido-4-iodobutane 12 as a slightly yellow oil (27.7g, 123 mmol, 84% yield). ¹H-NMR (400 MHz, CDCl₃) δ=3.33 (t, *J*=6.7 Hz, 2H), 3.21 (t, *J*=6.8 Hz, 2H), 1.95-1.88 (m, 2H), 1.75-1.68 (m, 2H). ¹³C-NMR (100 MHz, DMSO-d₆) δ=50.30, 30.42, 29.69, 5.61.

2,3,3-Trimethyl-1-(4-azidobutyl)-3H-indolium **13** To a 5 mL round bottom flask was added 2,3,3-trimethylindolenine **14** (1g, 6.3 mmol, 1 equiv.) and 1-azido-4-iodobutane 12 (1.55g, 6.9 mmol, 1.1 equiv.). The reaction mixture was stirred at 120 °C for 4 h, cooled to room temperature and was directly purified on silica gel flash chromatography with a gradient 1% to 5% MeOH in dichloromethane to afford 2,3,3-Trimethyl-1-(4-azidobutyl)-3*H*-indolium 13 as a dark oil (720 mg, 2.8 mmol, 45%). ¹H-NMR (400 MHz, COCl₃) δ=7.70-7.66 (m, 1 H), 7.46-7.38 (m, 3H), 4.52 (t, *J*=7.5 Hz, 2H), 3.27 (t, *J*=6.5 Hz, 2H), 2.95 (s, 3H), 1.91 (quin, *J*=7.9 Hz, 2H), 1.65 (quin, *J*=7.5 Hz, 2H), 1.45 (s, 6H). ¹³C-NMR (100 MHz, CDCl₃) δ=195.35, 140.85, 140.09, 129.54, 128.92, 122.83, 115.00, 54.06, 49.91, 48.49, 25.38, 24.74, 22.51, 16.45. ESI-MS: positive mode 257.00 ([M+H]⁺). Calc.: 256.17.

2,3,3-Trimethyl-1-(4-sulfonatobutyl)-3*H*-indolium **15** (54136-26-4) was prepared following the procedure of Kvach et al., Russ. Chem. Bull. 2006, 55, 159-163. To a 25 mL round bottom flash was added successively 2,3,3-trimethylindolenine **14** (3.97g, 25 mmol, 1 equiv.) and 1,4-butanesulfone (3.47 g, 25.5 mmol, 1.02 equiv.). The reaction mixture was heated to 120 °C for 4 h until the reaction solidified. The solid was triturated with ether (20 mL), filtered and washed three times with acetone (10 mL) to give 2,3,3-Trimethyl-1-(4-sulfonatobutyl)-3*H*-indolium **15** as a reddish solid (6.5 g, 22 mmol, 88%). ¹H-NMR (400 MHz, CDCl₃) δ=7.83-7.81 (m, 1H), 7.51-7.48 (m, 3H), 4.70 (t, *J*=7.9 Hz, 2H), 2.97 (s, 3H), 2.82 (t, *J*=7.9 Hz, 2H), 2.11 (quin, *J*=7.5 Hz, 2H), 1.95 (quin, *J*=7.0 Hz, 2H), 1.54 (s, 6H). ¹³C-NMR (100 MHz, CDCl₃) δ=196.20, 141.48, 141.11, 129.66, 129.46, 122.8.7, 115.74, 54.34, 49.85, 48.32, 26.35, 22.87, 22.70, 22.34, 14.63. ESI-MS: positive mode 295.98 ([M+H]⁺). Calc.: 295.12.

2-[2-(Acetylphenylamino)ethenyl]-3,3-dimethyl-1-(4-sulfobutyl)-3*H*-indolium **16** (876746-16-6) To a stirred solution of compound **15** (1g, 3.39 mmol, 1 equiv.) in acetic anhydride (15 mL) was added *N,N'*-diphenylformamidine (797 mg, 4.1 mmol, 1.2 equiv.). After 30 min stirring at 120 °C, the reaction mixture was evaporated to dryness and dissolved in dichloromethane (5 mL). Ether (100 mL) was added and the resulting gum was filtered. The gum was dissolved in dichloromethane (5 mL) and precipitated by addition of ether (100 mL) affording hemicyanine **16** (1.24g, 2.8 mmol, 83% yield) as a brown solid, which was directly used in the following reaction without further purification.

2-[3-[1,3-Dihydro-3,3-dimethyl-1-(4-sulfobutyl)-2*H*-indol-2-ylidene]-1-propen-1-yl]-3,3-dimethyl-1-(4-azidobutyl)-3*H*-Indolium **1** (Cy3 azide) To solution of hemicyanine **16** (171.8 mg, 0.39 mmol, 1 equiv.) and compound X (100mg, 0.39 mmol, 1 equiv.) in EtOH (10 mL) was added triethylamine (0.15 mL, 1.16 mmol, 3.equiv.). The reaction mixture was refluxed for 30 min, cooled to room temperature and evaporated to dryness. The residue was purified by flash chromatography with a gradient 5% to 8% MeOH in dichloromethane to afford Cy3 azide 1 (148 mg, 0.26 mmol, 68% yield) as a purple solid. ¹H-NMR (400 MHz, CDCl₃) δ=8.38 (t, *J*=13.4 Hz, 2H), 7.40-7.32 (m, 5H), 7.24-7.19 (m, 2H), 7.15-7.07 (m, 3H), 4.32-4.23 (m, 4H), 3.46 (t, *J*=5.5 Hz, 2H), 3.05 (t, *J*=7.4 Hz, 2H), 2.21 (quin, J=6.8 Hz, 2H), 2.00 (quin, J=7.6 Hz, 2H), 1.95-1.91 (m, 4H), 1.69 (d, *J*=3.2Hz, 12H). ¹³C-NMR (100 MHz, CDCl₃) δ=173.80, 173.26, 151.10, 142.15, 142.03, 140.71, 140.60, 128.85, 128.75, 125.10, 124.84, 121.97, 121.89, 111.04, 110.59, 105.62, 104.73, 51.28, 51.00, 48.78, 48.67, 44.50, 43.85, 28.15, 28.04, 26.38, 25.92, 24.79, 22.82. ESI-HRMS calculated for C31 H40 N5 03 S positive mode ([M+H]⁺) 562.2849. Calc.: 562:2846.

### Preparation of BHQ-1 azide

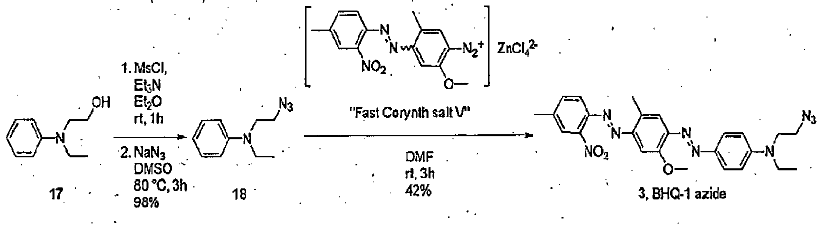

N-(2-azidoethyl)-N-ethylaniline **18** To a solution of 2-(N-ethylanilino)ethanol **17** (4.9 mL, 30.2 mmol) in Et₂O (250 mL) at 0 °C was added triethylamine (4.6 mL, 33.3 mmol, 1.1 equiv.) followed by a slow addition of methane sulfonyl chloride (2.6 mL, 33.3 mmol, 1.1 equiv.). After 1 h stirring at room temperature, the white precipitate was filtered and washed with Et₂O. The filtrate was concentrated to an oil and dissolved in 100 mL of DMSO. Sodium azide (3.9 g, 60.5 mmol, 2 equiv.) was added and the mixture was stirred at 80 °C for 3h. The reaction mixture was cooled to room temperature, diluted with water (400 mL) and extracted three times with 100 mL of Et₂O. The combined organic layer was washed with brine (100 mL), dried over Na₂SO₄, filtered and evaporated to dryness to afford N-(2-azidoethyl)-N-ethylaniline 18 as a colorless oil (5.6 g, 29.5 mmol, 98% yield). ¹H-NMR (400 MHz, CDCl₃) δ=7.34 (t, *J*=7.8 Hz, 2H), 6.84-6.81 (m, 3H), 3.59-3.49 (m, 6H), 1.27 (t, *J*=7.1 Hz, 3H). ¹³C-NMR(100 MHz, CDCl₃) δ=147.05, 129.25, 116.46, 112.14, 49.43, 48.81, 45.33, 12.01. ESI-MS: positive mode 190.72 ([M+H]⁺). Calc.: 190.12.

BHQ-1 azide **2** To a solution of Fast Corinth salt zinc chloride double salt (500 mg, 1 mmol, 1 equiv.) in DMF (100 mL) was added dropwise a solution of N-(2-azidoethyl)-N-ethylaniline 18 (209 mg, 1.1 mmol, 1,1 equiv.) in DMF (100 mL). The reaction mixture was stirred for 3h at room temperature and evaporated to dryness. The residue was dissolved in CH₂Cl₂ (200 mL) and washed once with 0.5 M HCl (80 mL) and once with brine (80 mL). The organic layer was dried over Na₂SO₄, filtered and evaporated to dryness. The residue was purified by flash column chromatography with a gradient 5 to 25% of EtOAc in hexanes to give BHQ-1 azide **3** as a dark solid (205 mg, 0.41 mmol, 42% yield). ¹H-NMR (400 MHz, CDCl₃) δ=7.92 (d, *J*=9.1 Hz, 2H), 7.69 (s, 1H), 7.66 (d, *J*=7.7 Hz, 1 H), 7.57 (s, 1H), 7.45 (dd, *J*=8.2, 1.0 Hz, 1H), 7.39 (s, 1H), 6.76 (d, *J*=9.2 Hz, 2H), 4.02 (s, 3H), 3.61-3.58 (m, 2H), 3.56-3.51 (m, 4H), 2.70 (s, 3H), 2.49 (m, 3H), 1.24 (t, *J*=7.1 Hz, 3H). ¹³C-NMR (100 MHz, CDCl₃) δ=154.80, 151.05, 149.98, 147.41, 145.18, 144.80, 143.35, 141.55, 133.43, 132.90, 125.89, 124.24, 118.99, 118.92, 111.42, 99.36, 56.26, 49.51, 48.93, 45.80, 21.22, 16.67, 12.26. ESI-HRMS calculated for C25 H28 N9 03 positive mode ([M+H]⁺) 502.2311. Calc.: 502.2310.

Oligoribonucleotide synthesis: Oligoribonucleotides were synthesized with regular 2'-O-TBDMS-phosphoramidites on a 50 nmol scale using.5 mg of CPG (1000A). For 2'-O-propargyl cytidine phosphoramidite, the coupling time was prolonged to 3 x 4 min. After synthesis, the CPG with the alkynyl-modified RNA was suspended in 300 µL of H₂O/MeOH (1:1) in an Eppendorf tube. Subsequently, DMF (40 µL) and a freshly-prepared solution of the azide (20 equiv., 1 µmol in 20 µL of DMF), TBTA (10 equiv., 500 nmol, 0.27 mg in 20 µL of DMF); Na-ascorbate (10 equivalents, 500 nmol, 10 µL of a solution containing 10 mg in 1mL of H₂O) and CuSO₄.5H₂O (1 equiv., 50 nmol, 10 µL of a solution containing 12.5 mg in 10 mL of H₂O) were added to the suspension, in this order. All solutions were prepared as stock solutions directly prior use. The reaction mixture was shaken for 16h at 45°C in an Eppendorf shaker. CPG was filtered, washed three times with 0.5mL of DMF, 0.1 N aqueous EDTA, DMF, MeCN and CHCl₃. CPG was transferred into an Eppendorf tube and treated with 200 µL of ammonia (25% in H₂O) and 200 µL of methylamine (40 % in H₂O) solutions for 5 h at room temperature. After filtration, the remaining RNA was washed from the solid support with 3 x 100 µL H₂O/EtOH (1:1). To the solution was added 20 µL of 1N Tris-base and it was evaporated to dryness in a SpeedVac. Desilylation was carried out by treatment with 130 µL of a mixture of NMP (60 µL), TEA (30 µL) and TEA.3HF (40 µL) at 70 °C for 90 min. The reaction was quenched with trimethylethoxysilane (160 µL) for 20 min at room temperature on an Eppendorf shaker. Diethylether (1 mL) was added, the mixture was vortexed and centrifuged at 4 °C for 2 min. The supernatant was taken off and the precipitate was washed twice with 1 mL diethylether, vortexed and centrifuged. The oligonucleotide was dissolved in 200 µL of water and purified DMT-on by RP-HPLC. The isolated product was dried in a SpeedVac, treated for 1h with 40% aq. acetic acid at room temperature, dried in a SpeedVac, dissolved in 200 µL of water and purified DMT-off by RP-HPLC. (Double amount of azides is used for the homo bis-labelling of RNA hairpin; for

poorly water soluble azides such as BHQ-1, the volume of DMF was increased to 100 µL and was mixed with 240 µL of a 1:1 mixture of H₂O/MeOH before addition of the reagents

### Example 3: FRET

### Materials

FRET measurements were acquired on a QuantaMaster 50 (PMI Photon Technology International) in a 500*µ*L Precision cell made of Quartz SUPRASIL (115F-QS, 10x2 mm, Hellma) cuvette.

### Solutions

EGFP-C2-Lin28b containing lysate was diluted with working buffer (25mM Hepes, 300mM NaCl, 0.01mM ZnCl2, 1% Topblock, 0.05% Tween and 0.5 mM TCEP) to a concentration of approximately 2 nM.

Dry cy3 or BHQ-1 labeled truncated pre-let-7a-2 (SEQ ID: NO 2: nucleotides 10 to 57) was diluted with Millipore water to a concentration of 20*µ*M.

### Protocol

Measurements were acquired without and with various concentrations of labeled pre-let-7a-2 of 0.313nM, 1.25nM, 5nM, 20nM and 80nM. Solutions were incubated for 30 min and their fluorescence spectra were acquired between 475-600 nm after excitation of the sample at 465 nm. The Förster Resonance Energy Transfer was calculated based on the difference of the EGFP emission signal intensity (at 507nm) between the solution containing only the GFP-tagged lin28 and the solutions containing GFP-tagged lin28 and various concentra-tions of labeled pre-let-7a-2. For Cy3-based FRET measurements a, background signal of Cy3 was subtracted to get rid of the bleach through. Measurements shown represent at least biological duplicates.

### 3.1 Fret measurements

### 3.1.1 Measurements with cy3-labeled truncated pre-let-7a-2

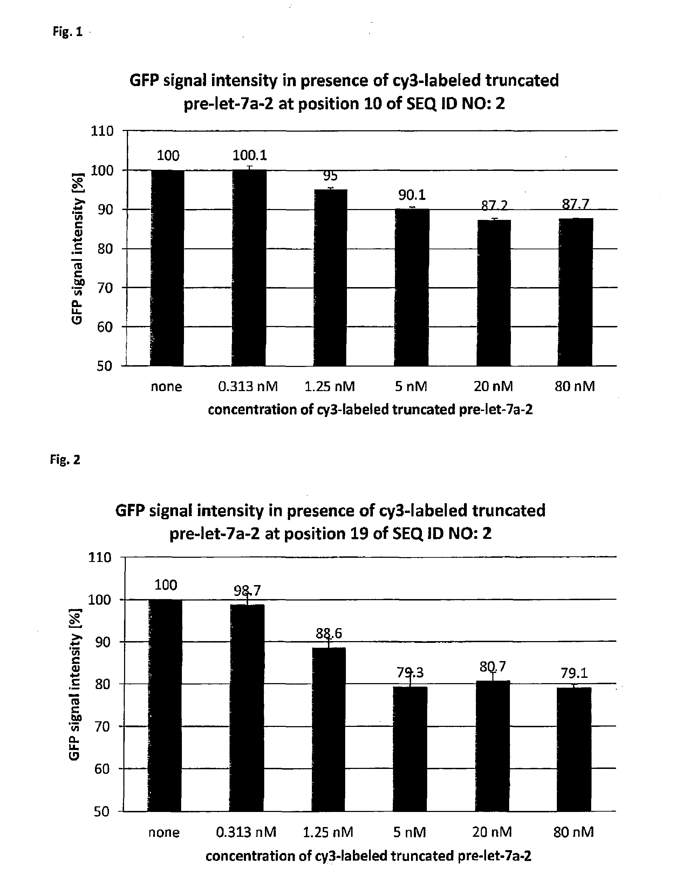

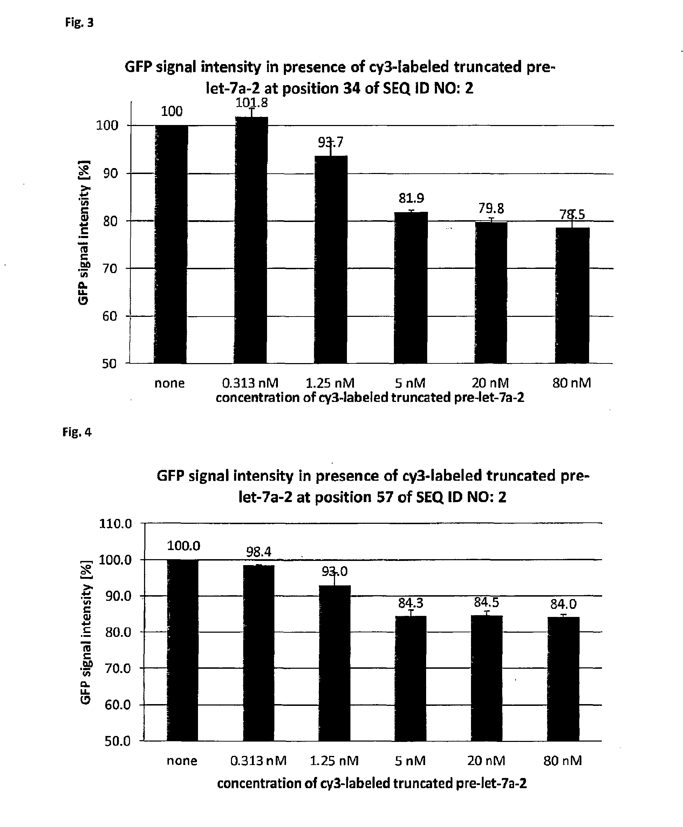

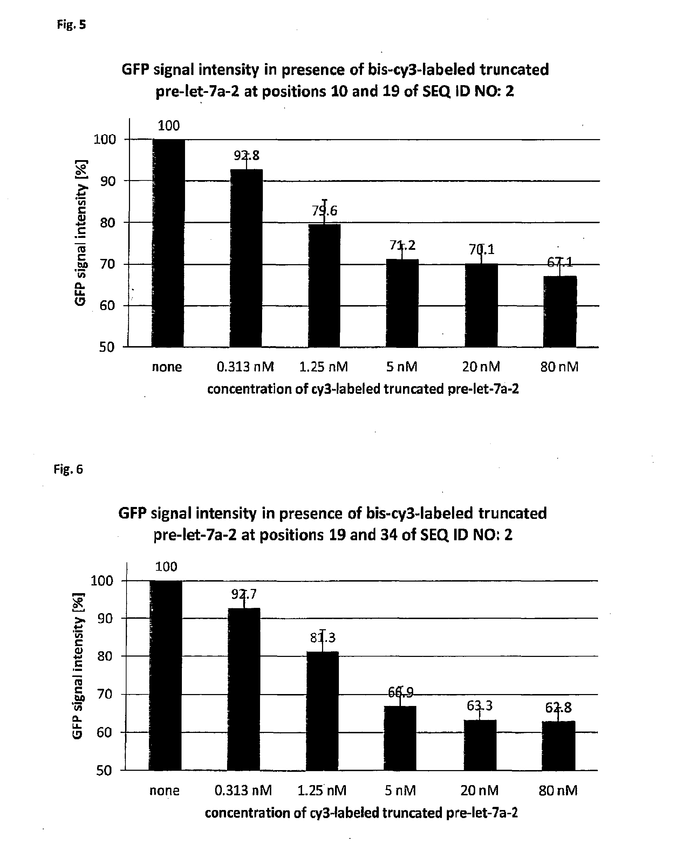

The results of the FRET measurements with mono- and bis-cy3-labeled truncated pre-let-7a-2 are shown in Figs. 1 to 7

### 3.1.2 Measurements with BHQ-1-labeled truncated pre-let-7a-2

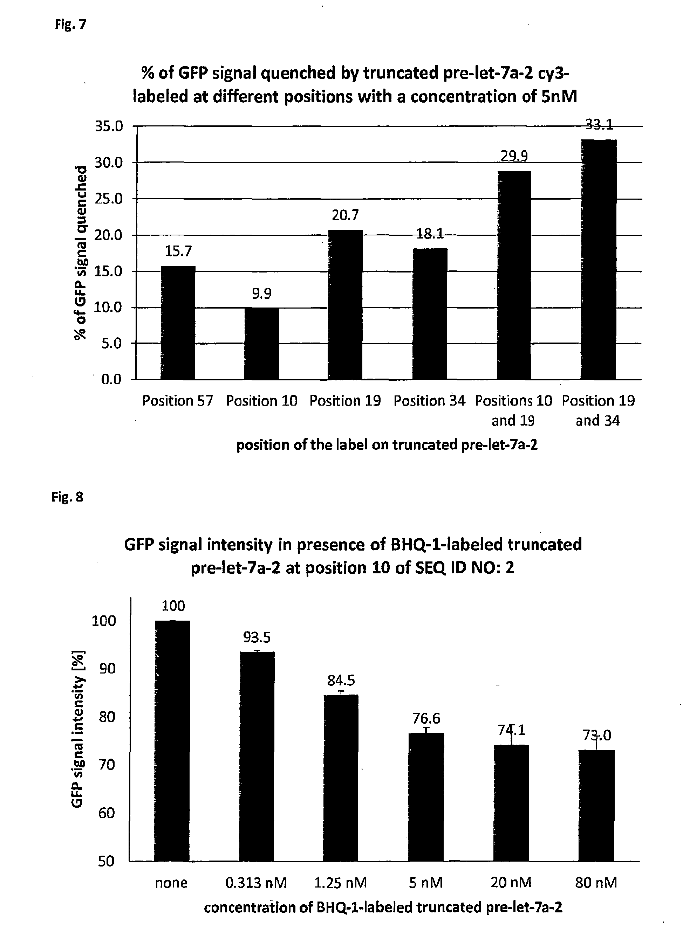

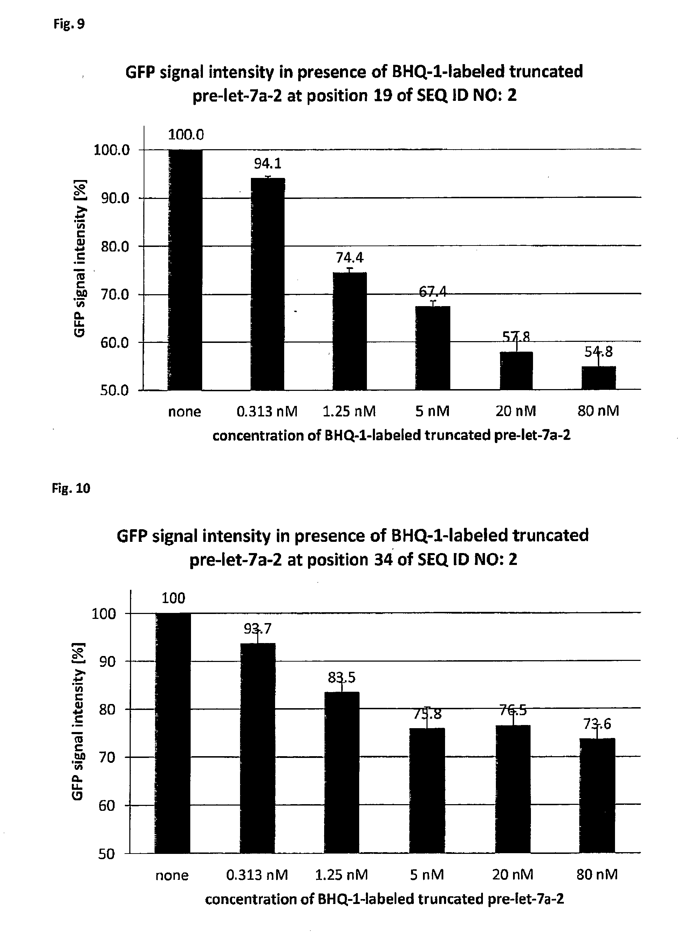

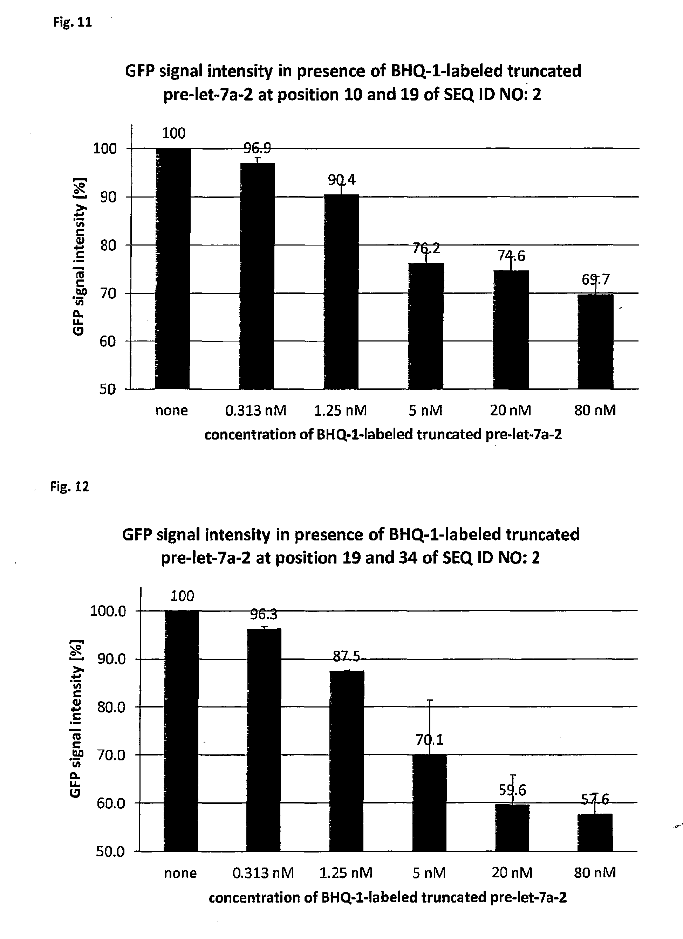

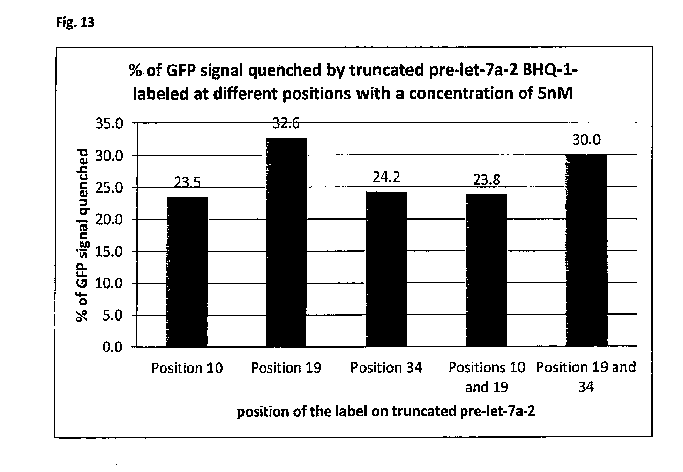

The results of the FRET measurements with mono- and bis-BHQ-1-labeled truncated pre-let-7a-2 are shown in Figs. 8 to 13.

**Table 1 Preferred polypeptides interacting with nucleotides (RBPs, ribonucleotide-binding polypeptides)**

| Abbrev. | Full name [source, accession number] |
|---|---|
| RBM5 | RNA binding motif protein 5 [Source:HGNC Symbol;Acc:9902] |
| SARM1 | sterile alpha and TIR motif containing 1 [Source:HGNC Symbol;Acc:17074] |
| RBM6 | RNA binding motif protein 6 [Source:HGNC Symbol;Acc:9903] |
| | Putative RNA exonuclease NEF-sp (EC3.1.-.-) [Source:UniProtKB/Swiss-Prot;Acc:Q96IC2] |
| YBX2 | Y box binding protein 2 [Source:HGNC Symbol;Acc:17948] |
| CSDE1 | cold shock domain containing E1, RNA-binding [Source:HGNC Symbol;Acc:29905] |
| SCMH1 | sex comb on midleg homolog 1 (Drosophila) [Source:HGNC Symbol;Acc:19003] |
| PTBP1 | polypyrimidine tract binding protein 1 [Source:HGNC Symbol;Acc:9583] |
| ZC3H3 | zinc finger CCCH-type containing 3 [Source:HGNC Symbol;Acc:28972] |
| MATR3 | matrin 3 [Source:HGNC Symbol;Acc:6912] |
| SAMD4A | sterile alpha motif domain containing 4A [Source:HGNC Symbol;Acc:23023] |
| LCP2 | lymphocyte cytosolic protein 2 (SH2 domain containing leukocyte protein of 76kDa) [Source:HGNC;Acc:6529] |
| EPHA3 | EPH receptor A3 [Source:HGNC Symbol;Acc:3387] |
| YTHDC2 | YTH domain containing 2 [Source:HGNC Symbol;Acc:24721] |
| ARAP2 | ArfGAP with RhoGAP domain, ankyrin repeat and PH domain 2 [Source:HGNC Symbol;Acc:16924] |
| SCML1 | sex comb on midleg-like 1 (Drosophila) [Source:HGNC Symbol;Acc:10580] |
| CUGBP2 | CUG triplet repeat, RNA binding protein 2 [Source:HGNC Symbol;Acc:2550] |
| PUM2 | pumilio homolog 2 (Drosophila) [Source:HGNC Symbol;Acc:14958] |
| RC3H2 | ring finger and CCCH-type zinc finger domains 2 [Source:HGNC Symbol;Acc:21461] |
| ZC3H11A | zinc finger CCCH-type containing 11A [Source:HGNC Symbol;Acc:29093] |
| | RING finger protein unkempt-like [Source:UniProtKB/Swiss-Prot;Acc:Q9H9P5] |
| PARP12 | poly (ADP-ribose) polymerase family, member 12 [Source:HGNC Symbol;Acc:21919] |
| CSDAP1 | cold shock domain protein A pseudogene 1 [Source:HGNC Symbol;Acc:2429] |
| CSDA | cold shock domain protein A pseudogene 1 [Source:HGNC Symbol;Acc:2429] |
| SFRS8 | splicing factor, arginine/serine-rich 8 (suppressor-of-white-apricot homolog, Drosophila) [Source:HGNC;Acc:10790] |
| EIF4B | eukaryotic translation initiation factor 4B [Source:HGNC Symbol;Acc:3285] |
| U2AF2 | U2 small nuclear RNA auxiliary factor 2 [Source:HGNC Symbol;Acc:23156] |
| SFRS14 | splicing factor, arginine/serine-rich 14 [Source:HGNC Symbol;Acc:18641] |
| ANKS1A | ankyrin repeat and sterile alpha motif domain containing 1A [Source:HGNC Symbol;Acc:20961] |
| SPEN | spen homolog, transcriptional regulator (Drosophila) [Source:HGNC Symbol;Acc:17575] |
| ZC3H15 | zinc finger CCCH-type containing 15 [Source:HGNC Symbol;Acc:29528] |
| YBX1 | Y box binding protein 1 [Source:HGNC Symbol;Acc:8014] |
| ELAV1 | ELAV (embryonic lethal, abnormal vision, Drosophila)-like 1 (Hu antigen R) [Source:HGNC Symbol;Acc:3312] |
| THUMPD1 | THUMP domain containing 1 [Source:HGNC Symbol;Acc:23807] |
| DHX8 | DEAH (Asp-Glu-Ala-His) box polypeptide 8 [Source:HGNC Symbol;Acc:2749] |
| SRBD1 | S1 RNA binding domain 1 [Source:HGNC Symbol;Acc:25521] |
| PABPC1 | poly(A) binding protein, cytoplasmic 1 [Source:HGNC Symbol;Acc:8554] |
| EPHA8 | EPH receptor A8 [Source:HGNC Symbol;Acc:3391] |
| DAZAP1 | DAZ associated protein 1 [Source:HGNC Symbol;Acc:2683] |
| TP63 | tumor protein p63 [Source:HGNC Symbol;Acc:15979] |
| IGF2BP2 | insulin-like growth factor 2 mRNA binding protein 2 [Source:HGNC Symbol;Acc:28867] |
| ZNF638 | zinc finger protein 638 [Source:HGNC Symbol;Acc:17894] |
| SART3 | squamous cell carcinoma antigen recognized by T cells 3 [Source:HGNC Symbol;Acc:16860] |
| MKRN2 | makorin ring finger protein 2 [Source:HGNC Symbol;Acc:7113] |
| RBM7 | RNA binding motif protein 7 [Source:HGNC Symbol;Acc:9904] |
| RBMS2 | RNA binding motif, single stranded interacting protein 2 [Source:HGNC Symbol;Acc:9909] |
| MBNL3 | muscleblind-like 3 (Drosophila) [Source:HGNC Symbol;Acc:20564] |
| DGKD | diacylglycerol kinase, delta 130kDa [Source:HGNC Symbol;Acc:2851] |
| SNRPA | small nuclear ribonucleoprotein polypeptide A [Source:HGNC Symbol;Acc:11151] |
| SYNJ2 | synaptojanin 2 [Source:HGNC Symbol;Acc:11504] |
| A2BP1 | Fox-1 homolog A (Ataxin-2-binding protein 1)(Hexaribonucleotide-binding protein 1) [Swiss-Prot;Acc:Q9NWB1] |
| | Trinucleotide repeat-containing gene 6C protein [Source:UniProtKB/Swiss-Prot;Acc:Q9HCI0] |
| TP73 | tumor protein p73 [Source:HGNC Symbol;Acc:12003] |
| DDX43 | DEAD (Asp-Glu-Ala-Asp) box polypeptide 43 [Source:HGNC Symbol;Acc:18677] |
| EPHA6 | EPH receptor A6 [Source:HGNC Symbol;Acc:19296] |
| KIAA0020 | KIAA0020 [Source:HGNC Symbol;Acc:29676] |
| CNOT4 | CCR4-NOT transcription complex, subunit 4 [Source:HGNC Symbol;Acc:7880] |
| YTHDC1 | YTH domain containing 1 [Source:HGNC Symbol;Acc:30626] |
| PPIE | peptidylprolyl isomerase E (cyclophilin E) [Source:HGNC Symbol;Acc:9258] |
| DDHD2 | DDHD domain containing 2 [Source:HGNC Symbol;Acc:29106] |
| CHERP | calcium homeostasis endoplasmic reticulum protein [Source:HGNC Symbol;Acc:16930] |
| RBM22 | RNA binding motif protein 22 [Source:HGNC Symbol;Acc:25503] |
| KHSRP | KH-type splicing regulatory protein [Source:HGNC Symbol;Acc:6316] |
| FUS | fused in sarcoma [Source:HGNC Symbol;Acc:4010] |
| RBM41 | RNA binding motif protein 41 [Source:HGNC Symbol;Acc:25617] |
| PCBP4 | poly(rC) bindingprotein 4 [Source:HGNC Symbol;Acc:8652] |
| PABPC4 | poly(A) binding protein, cytoplasmic 4 (inducible form) [Source:HGNC Symbol;Acc:8557] |
| TNRC6A | trinucleotide repeat containing 6A [Source:HGNC Symbol;Acc:11969] |
| RBM27 | RNA binding motif protein 27 [Source:HGNC Symbol;Acc:29243] |
| | Mitogen-activated protein kinase kinase kinase MLT (EC 2.7.11.25)(MLK-like mitogen-activated protein triple kinase)(Sterile alpha motif- and leucine zipper-containing kinase AZK)(Leucine zipper- and sterile alpha motifcontaining kinase)(Mixed linea /.../ase-related kinase)(MLK-related kinase)(MRK)(Cervical cancer suppressor gene 4 protein)(HCCS-4) [Source:UniProtKB/Swiss-Prot;Acc:Q9NYL2] |
| HNRNPC | heterogeneous nuclear ribonucleoprotein C(C1/C2) [Source:HGNC Symbol;Acc:5035] |
| DAZL | deleted in azoospermia-like [Source:HGNC Symbol;Acc:2685] |
| HNRNPH3 | heterogeneous nuclear ribonucleoprotein H3 (2H9) [Source:HGNC Symbol;Acc:5043] |
| SETD1A | SET domain containing 1A [Source:HGNC Symbol;Acc:29010] |
| CIRBP | cold inducible RNA binding protein [Source:HGNC Symbol;Acc:1982] |
| HNRNPM | heterogeneous nuclear ribonucleoprotein M [Source;HGNC Symbol;Acc:5046] |
| SHANK3 | SH3 and multiple ankyrin repeat domains 3 [Source:HGNC Symbol;Acc:14294] |
| TRMT2A | TRM2 tRNA methyltransferase 2 homolog A (S. cerevisiae) [Source:HGNC Symbol;Acc:24974] |
| SF3A1 | splicingfactor 3a, subunit 1, 120kDa [Source:HGNC Symbol;Acc:10765] |
| SNRPD3 | small nuclear ribonucleoprotein D3 polypeptide 18kDa [Source:HGNC Symbol;Acc:11160] |
| POLDIP3 | polymerase (DNA-directed), delta interacting protein 3 [Source:HGNC Symbol;Acc:23782] |
| ZMAT5 | zinc finger, matrin type 5 [Source:HGNC Symbol;Acc:28046] |
| FOX1 | RNA binding motif protein 9 [Source:HGNC Symbol;Acc:9906] |
| ZC3H7B | zinc finger CCCH-type containing 7B [Source:HGNC Symbol;Acc:30869] |
| RBM23 | RNA binding motif protein 23 [Source:HGNC Symbol;Acc:20155] |
| C14orf174 | SAM domain-containing protein C14orf174 [Source:UniProtKB/Swiss-Prot;Acc:Q9P1V8] |
| SFRS5 | splicing factor, arginine/serine-rich 5 [Source:HGNC Symbol;Acc:10787] |
| ZC3H14 | zinc finger CCCH-type containing 14 [Source:HGNC Symbol;Acc:20509] |
| ACIN1 | apoptotic chromatin condensation inducer 1 [Source:HGNC Symbol;Acc:17066] |
| PABPN1 | poly(A) binding protein, nuclear 1 [Source:HGNC Symbol;Acc:8565] |
| PABPC1L | poly{A} binding protein, cytoplasmic 1-like [Source:HGNC Symbol;Acc:15797] |
| SAMHD1 | SAM domain and HD domain 1 [Source:HGNC Symbol;Acc:15925] |
| BRUNOL4 | bruno-like 4, RNA binding protein (Drosophila) [Source:HGNC Symbol;Acc:14015] |
| CSTF2 | cleavage stimulation factor, 3' pre-RNA, subunit 2, 64kDa [Source:HGNC Symbol;Acc:2484] |
| ZC3H12B | zinc finger CCCH-type containing 12B [Source:HGNC Symbol;Acc:17407] |
| FMR1 | fragile X mental retardation 1 [Source:HGNC Symbol;Acc:3775] |
| SCML2 | sex comb on midleg-like 2 (Drosophila) [Source:HGNC Symbol;Acc:10581] |
| HTATSF1 | HIV-1 Tat specific factor 1 [Source:HGNC Symbol;Acc:5276] |
| RBM3 | RNA binding motif (RNP1, RRM) protein 3 [Source:HGNC Symbol;Acc:9900] |
| DGKH | diacylglycerol kinase, eta [Source:HGNC Symbol;Acc:2854] |
| ESRP2 | epithelial splicing regulatory protein 2 [Source:HGNC Symbol;Acc:26152] |
| MTHFSD | methenyltetrahydrofolate synthetase domain containing [Source:HGNC Symbol;Acc:25778] |
| BFAR | bifunctional apoptosis regulator [Source:HGNC Symbol;Acc:17613] |
| DNAJC17 | DnaJ (Hsp40) homolog, subfamily C, member 17 [Source:HGNC Symbol;Acc:25556] |
| MYEF2 | myelin expression factor 2 [Source:HGNC Symbol;Acc:17940] |
| ESRP1 | epithelial splicing regulatory protein 1 [Source:HGNC Symbol;Acc:25966] |
| HNRNPL | heterogeneous nuclear ribonucleoprotein L [Source:HGNC Symbol;Acc:5045] |
| SNRNP70 | small nuclear ribonucleoprotein 70kDa (U1) [Source:HGNC Symbol;Acc:11150] |
| SFRS16 | splicing factor, arginine/serine-rich 16 [Source:HGNC Symbol;Acc:17731] |
| TRMT1 | TRM1 tRNA methyltransferase 1 homolog (S. cerevisiae) [Source:HGNC Symbol;Acc:25980] |
| NOVA2 | neuro-oncological ventral antigen 2 [Source:HGNC Symbol;Acc:7887] |
| SF4 | splicing factor 4 [Source:HGNC Symbol;Acc:18643] |
| ZC3HAV1 | zinc finger CCCH-type, antiviral 1 [Source:HGNC Symbol;Acc:23721] |
| EPHB6 | EPH receptor B6 [Source:HGNC Symbol;Acc:3396] |
| EIF3B | eukaryotic translation initiation factor 3, subunit B [Source:HGNC Symbol;Acc:3280] |
| RBM28 | RNA binding motif protein 28 [Source:HGNC Symbol;Acc:21863] |
| LSM5 | LSM5 homolog, U6 small nuclear RNA associated (S. cerevisiae) [Source:HGNC Symbol;Acc:17162] |
| EIF4H | eukaryotic translation initiation factor 4H [Source:HGNC Symbol;Acc:12741] |
| ELAVL2 | ELAV (embryonic lethal, abnormal vision, Drosophila)-like 2 (Hu antigen B) [Source:HGNC Symbol;Acc:3313] |
| FUBP3 | far upstream element (FUSE) binding protein 3 [Source:HGNC Symbol;Acc:4005] |
| SEC23IP | SEC23 interacting protein [Source:HGNC Symbol;Acc:17018] |
| TNKS2 | tankyrase, TRF1-interacting ankyrin-related ADP-ribose polymerase 2 [Source:HGNC Symbol;Acc:15677] |
| CPEB3 | cytoplasmic polyadenylation element binding protein 3 [Source:HGNC Symbol;Acc:21746] |
| LARP4B | La ribonucleoprotein domain family, member 48 [Source:HGNC Symbol;Acc:28987] |
| SUPT6H | suppressor of Ty 6 homolog (S. cerevisiae) [Source:HGNC Symbol;Acc:11470] |
| STIM2 | stromal interaction molecule 2 [Source:HGNC Symbol;Acc:19205] |
| PPARGC1A | peroxisome proliferator-activated receptor gamma, coactivator 1 alpha [Source:HGNC Symbol;Acc:9237] |
| PPFIBP1 | PTPRF interacting protein, binding protein 1 (liprin beta 1) [Source:HGNC Symbol;Acc:9249] |
| MIR1279 | microRNA 1279 [Source:HGNC Symbol;Acc:35357] |
| CPSF6 | microRNA 1279 [Source:HGNC Symbol;Acc:35357] |
| KRR1 | KRR1, small subunit (SSU) processome component, homolog (yeast) [Source:HGNC Symbol;Acc:5176] |
| PHC1 | polyhomeotic homolog 1 (Drosophila) [Source:HGNC Symbol;Acc:3182] |
| SFRS9 | splicing factor, arginine/serine-rich 9 [Source:HGNC Symbol;Acc:10791] |
| SASH1 | SAM and SH3 domain containing 1 [Source;HGNC Symbol;Acc:19182] |
| SFRS3 | splicing factor, arginine/serine-rich 3 [Source:HGNC Symbol;Acc:10785] |
| RBM24 | RNA binding motif protein 24 [Source:HGNC Symbol;Acc:21539] |
| KHDRBS2 | KH domain containing, RNA binding, signal transduction associated 2 [Source:HGNC Symbol;Acc:18114] |
| QKI | quaking homolog, KH domain RNA binding (mouse) [Source:HGNC Symbol;Acc:21100] |
| CPEB4 | cytoplasmic polyadenylation element binding protein 4 [Source:HGNC Symbol;Acc:21747] |
| FXR1 | fragile X mental retardation, autosomal homolog 1 [Source:HGNC Symbol;Acc:4023] |
| NCBP2 | nuclear cap binding protein subunit 2, 20kDa [Source:HGNC Symbol;Acc:7659] |
| NCL | nucleolin [Source:HGNC Symbol;Acc:7667] |
| | Pre-mRNA branch site protein p14 (SF3B 14 kDa subunit) [Source:UniProtKB/Swiss-Prot;Acc:Q9Y3B4] |
| HDLBP | high density lipoprotein binding protein [Source:HGNC Symbol;Acc:4857] |
| SFRS7 | splicing factor, arginine/serine-rich 7, 35kDa [Source:HGNC Symbol;Acc:10789] |
| PNO1 | partner of NOB1 homolog (S. cerevisiae) [Source:HGNC Symbol;Acc:32790] |
| TIA1 | TIA1 cytotoxic granule-associated RNA binding protein [Source:HGNC Symbol;Acc:11802] |
| EPHA4 | EPH receptor A4 [Source:HGNC Symbol;Acc:3388] |
| SFRS4 | splicing factor, arginine/serine-rich 4 [Source:HGNC Symbol;Acc:10786] |
| SFPQ | splicing factor proline/glutamine-rich (polypyrimidine tract binding protein associated) [Source:HGNC;Acc:10774] |
| SFRS11 | splicing factor, arginine/serine-rich 11 [Source:HGNC Symbol;Acc:10782] |
| PRPF3 | PRP3 pre-mRNA processing factor 3 homolog (S. cerevisiae) [Source:HGNC Symbol;Acc:17348] |
| PTBP2 | polypyrimidine tract binding protein 2 [Source:HGNC Symbol;Acc:17662] |
| ROD1 | ROD1 regulator of differentiation 1 (S. pombe) [Source:HGNC Symbol;Acc:10253] |
| RBM18 | RNA binding motif protein 18 [Source:HGNC Symbol;Acc:28413] |
| C14orf156 | SRA stem-loop-interacting RNA-binding protein, mitochondrial Precursor [Source:UniProtKB/Swiss-Prot; Acc:Q9GZT3] |
| RBM2S | RNA binding motif protein 25 [Source:HGNC Symbol;Acc:23244] |
| ARAP3 | ArfGAP with RhoGAP domain, ankyrin repeat and PH domain 3 [Source:HGNC Symbol;Acc:24097] |
| ENOX1 | ecto-NOX disulfide-thiol exchanger 1 [Source:HGNC Symbol;Acc:25474] |
| TARDBP | TAR DNA binding protein [Source:HGNC Symbol;Acc:11571] |
| AKAP1 | A kinase (PRKA) anchor protein 1 [Source:HGNC Symbol;Acc:367] |
| PSPC1 | paraspeckle component 1 [Source:HGNC Symbol;Acc:20320] |
| ZCCHC17 | zinc finger, CCHC domain containing 17 [Source:HGNC Symbol;Acc:30246] |
| KHDRBS1 | KH domain containing, RNA binding, signal transduction associated 1 [Source:HGNC Symbol;Acc:18116] |
| SASH3 | SAM and SH3 domain containing 3 [Source:HGNC Symbol;Acc:15975] |
| ZC3H7A | zinc finger CCCH-type containing 7A [Source:HGNC Symbol;Acc:30959] |
| HNRNPA2B1 | heterogeneous nuclear ribonucleoprotein A2/B1 [Source:HGNC Symbol;Acc:5033] |
| BICC1 | bicaudal C homolog 1 (Drosophila) [Source:HGNC Symbol;Acc:19351] |
| RBM19 | RNA binding motif protein 19 [Source:HGNC Symbol;Acc:29098] |
| ZC3H13 | zinc finger CCCH-type containing 13 [Source:HGNC Symbol;Acc:20368] |
| SFRS6 | splicing factor, arginine/serine-rich 6 [Source:HGNC Symbol;Acc:10788] |
| RNF113A | ring finger protein 113A [Source:HGNC Symbol;Acc:12974] |
| SNRPD2 | small nuclear ribonucleoprotein D2 polypeptide 16.5kDa [Source:HGNC Symbol;Acc:11159] |
| SNRPB | small nuclear ribonucleoprotein polypeptides B and B1 [Source:HGNC Symbol;Acc:11153] |
| SNRPB2 | small nuclear ribonucleoprotein polypeptide B" [Source:HGNC Symbol;Acc:11155] |
| HNRNPR | heterogeneous nuclear ribonucleoprotein R [Source:HGNC Symbol;Acc:5047] with lethal |
| RALY | RNA binding protein, autoantigenic (hnRNP-associated yellow homolog (mouse)) [HGNC; Acc:15921] |
| RBM42 | RNA binding motif protein 42 [Source:HGNC Symbol;Acc:28117] |
| HNRNPH2 | heterogeneous nuclear ribonucleoprotein H2 (H') [Source:HGNC Symbol;Acc:5042] |
| ZFP36 | zinc finger protein 36, C3H type, homolog (mouse) [Source:HGNC Symbol;Acc:12862] |
| NAA38 | N(alpha)-acetyltransferase 38, NatC auxiliary subunit [Source:HGNC Symbol;Acc:20471] |
| SNRPN | small nuclear ribonucleoprotein polypeptide N [Source:HGNC Symbol;Acc:11164] |
| FXR2 | fragile X mental retardation, autosomal homolog 2 [Source:HGNC Symbol;Acc:4024] |
| SAFB2 | scaffold attachment factor B2 [Source:HGNC Symbol;Acc:21605] |
| LSM7 | LSM7 homolog, U6 small nuclear RNA associated (S. cerevisiae) [Source:HGNC Symbol;Acc:20470] |
| LSM4 | LSM4 homolog, U6 small nuclear RNA associated (S. cerevisiae) [Source:HGNC Symbol;Acc:17259] |
| SAMD10 | sterile alpha motif domain containing 10 [Source:HGNC Symbol;Acc:16129] |
| ZC3H4 | zinc finger CCCH-type containing 4 [Source:HGNC Symbol;Acc:17808] |
| EIF3G | eukaryotic translation initiation factor 3, subunit G [Source:HGNC Symbol;Acc:3274] |
| SNORA56 | small nucleolar RNA, H/ACA box 56 [Source:HGNC Symbol;Acc:32650] |
| DKC1 | small nucleolar RNA, H/ACA box 56 [Source:HGNC Symbol;Acc:32650] |
| PPIL4 | peptidylprolyl isomerase (cyclophilin)-like 4 [Source:HGNC Symbol;Acc:15702] |
| RBM39 | RNA binding motif protein 39 [Source:HGNC Symbol;Acc:15923] |
| RFTN1 | raftlin, lipid raft linker 1 [Source:HGNC Symbol;Acc:30278] |
| ANKHD1-EIF4BBI | ankyrin BBI ankyrin repeat and KH domain containing 1 [Source:HGNC Symbol;Acc:24714] |
| ANKHD1 | ankyrin repeat and KH domain containing 1 [Source:HGNC Symbol;Acc:24714] |
| PPFIA1 | protein tyrosine phosphatase, receptor type, f polypeptide (PTPRF), interacting protein (liprin), alpha 1 [HGNC Symbol;Acc:9245] |
| KHDRBS3 | KH domain containing, RNA binding, signal transduction associated 3 [Source:HGNC Symbol;Acc:18117] |
| RBM8A | RNA binding motif protein 8A [Source:HGNC Symbol;Acc:9905] |
| LIN28 | lin-28 homolog (C. elegans) [Source:HGNC Symbol;Acc:15986] |
| GRSF1 | G-rich RNA sequence binding factor 1 [Source:HGNC Symbol;Acc:4610] |
| ANKRD17 | ankyrin repeat domain 17 [Source:HGNC Symbol;Acc:23575] |
| UNK | unkempt homolog (Drosophila) [Source:HGNC Symbol;Acc:29369] |
| NIP7 | nuclear import 7 homolog (S. cerevisiae) [Source:HGNC Symbol;Acc:24328] |
| TOE1 | target of EGR1, member 1 (nuclear) [Source:HGNC Symbol;Acc:15954] |
| RBM38 | RNA binding motif protein 38 [Source:HGNC Symbol;Acc:15818] |
| EPHB2 | EPH receptor B2 [Source:HGNC Symbol;Acc:3393] |
| SRRM1 | serine/arginine repetitive matrix 1 [Source:HGNC Symbol;Acc:16638] |
| MKRN1 | makorin ring finger protein 1 [Source:HGNC Symbol;Acc:7112] |
| EIF2S1 | eukaryotic translation Initiation factor 2, subunit 1 alpha, 35kDa [Source:HGNC Symbol;Acc:3265] |
| THUMPD3 | THUMP domain containing 3 [Source:HGNC Symbol;Acc:24493] |
| RBMX2 | RNA binding motif protein, X-linked 2 [Source:HGNC Symbol;Acc:24282] |
| PUM1 | pumilio homolog 1 (Drosophila) [Source:HGNC Symbol;Acc:14957] |
| PHC2 | polyhomeotic homolog 2 (Drosophila) [Source:HGNC Symbol;Acc:3183] |
| MSI1 | musashi homolog 1 (Drosophila) [Source:HGNC Symbol;Acc:7330] |
| SYNCRIP | synaptotagmin binding, cytoplasmic RNA interacting protein [Source:HGNC Symbol;Acc:16918] |
| EPHA7 | EPH receptor A7 [Source:HGNC Symbol;Acc:3390] |
| ZC3H10 | zinc finger CCCH-type containing 10 [Source:HGNC Symbol;Acc:25893] |
| HNRNPA1 | heterogeneous nuclear ribonucleoprotein A1 [Source:HGNC Symbol;Acc:5031] |
| RC3H1 | ring finger and CCCH-type zinc finger domains 1 [Source:HGNC Symbol;Acc:29434] |
| IGF2BP3 | insulin-like growth factor 2 mRNA binding protein 3 [Source:HGNC Symbol;Acc:28868] |
| NUPL2 | nucleoporin like 2 [Source:HGNC Symbol;Acc:17010] |
| SFRS1 | splicing factor, arginine/serine-rich 1 [Source:HGNC Symbol;Acc:10780] |
| TRA2B | transformer 2 beta homolog (Drosophila) [Source:HGNC Symbol;Acc:10781] |
| CPEB2 | cytoplasmic polyadenylation element binding protein 2 [Source:HGNC Symbol;Acc:21745] |
| ALKBH8 | alkB, alkylation repair homolog 8 (E. coli) [Source:HGNC Symbol;Acc:25189] |
| SLTM | SAFB-like, transcription modulator [Source:HGNC Symbol;Acc:20709] |
| PNPT1 | polyribonucleotide nucleotidyltransferase 1 [Source:HGNC Symbol;Acc:23166] |
| THUMPD2 | THUMP domain containing 2 [Source:HGNC Symbol;Acc:14890] |
| ASCC1 | activating signal cointegrator 1 complex subunit 1 [Source:HGNC Symbol;Acc:24268] |
| SSB | Sjogren syndrome antigen B (autoantigen La) [Source:HGNC Symbol;Acc:11316] |
| HNRNPD | heterogeneous nuclear ribonucleoprotein D (AU-rich element RNA binding protein 1, 37kDa) [Source:HGNC Symbol;Acc:5036] |
| LARP1B | La ribonucleoprotein domain family, member 1B [Source:HGNC Symbol;Acc:24704] |
| G3BP2 | GTPase activating protein (SH3 domain) binding protein 2 [Source:HGNC Symbol;Acc:30291] |
| PAPSS1 | 3'-phosphoadenosine 5'-phosphosulfate synthase 1 [Source:HGNC Symbol;Acc:8603] |
| ZCRB1 | zinc finger CCHC-type and RNA binding motif 1 [Source:HGNC Symbol;Acc:29620] |
| PPFIA2 | protein tyrosine phosphatase, receptor type, f polypeptide (PTPRF), interacting protein (liprin), alpha 2 [Source:HGNC Symbol;Acc:9246] |
| SNRPF | small nuclear ribonucleoprotein polypeptide F [Source:HGNC Symbol;Acc:11162] |
| | Heterogeneous nuclear ribonucleoprotein A1-like protein 2 (hnRNP core protein A1-like protein 2) [Source:UniProtKB/Swiss-Prot;Acc:Q32P51] |
| SETD1B | SET domain containing 1B [Source:HGNC Symbol;Acc:29187] |
| RBM26 | RNA binding motif protein 26 [Source:HGNC Symbol;Acc:20327] |
| MBNL2 | muscle blind-like 2 (Drosophila) [Source:HGNC Symbol;Acc:16746] |
| RNF113B | ring finger protein 113B [Source:HGNC Symbol;Acc:17267] |
| NOVA1 | neuro-oncological ventral antigen 1 [Source:HGNC Symbol;Acc:7886] |
| BRUNOL6 | bruno-like 6, RNA binding protein (Drosophila) [Source:HGNC Symbol;Acc:14059] |
| | Putative uncharacterized protein ENSP00000269724 Fragment [Source:UniProtKB/TrEMBL;Acc:C9JNJ0] |
| DUS3L | dihydrouridine synthase 3-like (S. cerevisiae) [Source:HGNC Symbol;Acc:26920] |
| EPHA2 | EPH receptor A2 [Source:HGNC Symbol;Acc:3386] |
| CNKSR1 | connector enhancer of kinase suppressor of Ras 1 [Source:HGNC Symbol;Acc:19700] |
| SF3B4 | splicing factor 3b, subunit 4,49kDa [Source:HGNC Symbol;Acc:10771] |
| LGTN | ligatin [Source:HGNC Symbol;Acc:6583] |
| PPFIA4 | protein tyrosine phosphatase, receptor type, f polypeptide (PTPRF), interacting protein (liprin), alpha 4 [Source:HGNC Symbol;Acc:9248] |
| HNRPLL | heterogeneous nuclear ribonucleoprotein L-like [Source:HGNC Symbol;Acc:25127] |
| SNRPG | small nuclear ribonucleoprotein polypeptide G [Source:HGNC Symbol;Acc:11163] |
| ZC3H8 | zinc finger CCCH-type containing 8 [Source:HGNC Symbol;Acc:30941] |
| RBMS3 | RNA binding motif, single stranded interacting protein [Source:HGNC Symbol;Acc:13427] |
| EPHA5 | EPH receptor A5 [Source:HGNC Symbol;Acc:3389] |
| G3BP1 | GTPase activating protein (SH3 domain) binding protein 1 [Source:HGNC Symbol;Acc:30292] |
| SCML4 | sex comb on midleg-like 4 (Drosophila) [Source:HGNC Symbol;Acc:21397] |
| EPHA1 | EPH receptor A1 [Source:HGNC Symbol;Acc:3385] |
| NONO | non-POU domain containing, octamer-binding [Source:HGNC Symbol;Acc:7871] |
| RBMX | RNA binding motif protein, X-linked [Source:HGNC Symbol;Acc:9910] |
| LRSAM1 | leucine rich repeat and sterile alpha motif containing 1 [Source:HGNC Symbol;Acc:25135] |
| A1CF | APOBEC1 complementation factor [Source:HGNC symbol;Acc:24086] |
| PPRC1 | peroxisome proliferator-activated receptor gamma, coactivator-related 1 [Source:HGNC Symbol;Acc:30025] |
| PDCD11 | programmed cell death 11 [Source:HGNC Symbol;Acc:13408] |
| EEF1G | terminal uridylyl transferase 1, U6 snRNA-specific [Source:HGNC Symbol;Acc:26184] |
| TUT1 | terminal uridylyl transferase 1, U6 snRNA-specific [Source:HGNC Symbol;Acc:26184] |
| CUGBP1 | CUG triplet repeat, RNA binding protein 1 [Source:HGNC Symbol;Acc:2549] |
| RPS3 | ribosomal protein S3 [Source:HGNC Symbol;Acc:10420] |
| ZC3H12C | zinc finger CCCH-type containing 12C [Source:HGNC Symbol;Acc:29362] |
| CPSF7 | cleavage and polyadenylation specific factor 7, 59kDa [Source:HGNC Symbol;Acc:30098] |
| YTHDF1 | YTH domain family, member 1 [Source:HGNC Symbol;Acc:15867] |
| CNKSR2 | connector enhancer of kinase suppressor of Ras 2 [Source:HGNC Symbol;Acc:19701] |
| PABPC3 | poly(A) binding protein, cytoplasmic 3 [Source:HGNC Symbol;Acc:8556] |
| TIAL1 | TIA1 cytotoxic granule-associated RNA binding protein-like [Source:HGNC Symbol;Acc:11804] |
| RBM46 | RNA binding motif protein 46 [Source:HGNC Symbol;Acc:28401] |
| UHMK1 | U2AF homology motif (UHM) kinase 1 [Source:HGNC Symbol;Acc:19683] |
| BOLL | bol, boule-like (Drosophila) [Source:HGNC Symbol;Acc:14273] |
| RFP36L2 | zinc finger protein 36, C3H type-like 2 [Source:HGNC Symbol;Acc:1108] |
| PAN3 | PAN3 poly(A) specific ribonuclease subunit homolog (S. cerevisiae) [Source:HGNC Symbol;Acc:29991] |
| MBNL1 | muscle blind-like (Drosophila) [Source:HGNC Symbol;Acc:6923] |
| HNRPDL | heterogeneous nuclear ribonucleoprotein D-like [Source:HGNC Symbol;Acc:5037] |
| CARHSP1 | calcium regulated heat stable protein 1, 24kDa [Source:HGNC Symbol;Acc:17150] |
| RBMS1 | RNA binding motif, single stranded interacting protein 1 [Source:HGNC Symbol;Acc:9907] |
| CNKSR3 | CNKSR family member 3 [source: HGNC Symbol;Acc:23034] |
| SFRS12 | splicing factor, arginine/serine-rich 12 [Source:HGNC Symbol;Acc:17882] |
| MSI2 | musashi homolog 2 (Drosophila) [Source:HGNC Symbol;Acc:18585] |
| SFRS13B | splicing factor, arginine/serine-rich 13B [Source:HGNC Symbol;Acc:21220] |
| L3MBTL4 | I(3)mbt-like 4 (Drosophila) [Source:HGNC Symbol;Acc:26677] |
| EPHB1 | EPH receptor B1[Source:HGNC Symbol;Acc:3392] |
| C4orf23 | Probable tRNA (uracil-0(2)-)-methyltransferase (EC 2.1.1.n2) [Source:UniProtKB/Swiss-Prot;Acc:Q8IYL2] |
| SAMSN1 | SAM domain, SH3 domain and nuclear localization signals 1 [Source:HGMC Symbol;Acc:10528] |
| MKI67IP | MKI67 (FHA domain) interacting nucleolar phosphoprotein [Source:HGNC Symbol;Acc:17838] |
| LARP1 | La ribonucleoprotein domain family, member 1 [Source:HGNC Symbol;Acc:29531] |
| RBM45 | RNA binding motif protein 45 [Source:HGNC Symbol;Acc:24468] |
| PPARGC1B | peroxisome proliferator-activated receptor gamma, coactivator 1 beta [Source:HGNC Symbol;Acc:30022] |
| LSM11 | LSM11, U7 small nuclear RNA associated [Source:HGNC Symbol;Acc:30860] |
| SFRS15 | splicing factor, arginine/serine-rich 15 [Source:HGNC Symbol;Acc:19304] |
| SAMD8 | sterile alpha motif domain containing 8 [Source:HGNC Symbol;Acc;26320] |
| RBPMS | RNA binding protein with multiple splicing [Source:HGNC Symbol;Acc:19097] |
| PPP1R9A | protein phosphatase 1, regulatory (inhibitor) subunit9A [Source:HGNC Symbol;Acc:14946] |
| ZC3H18 | zinc finger CCCH-type containing 18 [Source:HGNC Symbol;Acc:25091] |
| SYNJ1 | synaptojanin 1 [Source:HGMC Symbol;Acc:11503] |
| IGF2BP1 | insulin-like growth factor 2 mRNA binding protein 1 [Source:HGNC Symbol;Acc:28866] |
| TNRC4 | trinucleotide repeat containing 4 [Source:HGNC Symbol;Acc:11967] |
| U2AF1 | U2 small nuclear RNA auxiliary factor 1 [Source:HGNC Symbol;Acc:12453] |
| SAFB | scaffold attachment factor B [Source:HGNC Symbol;Acc:10520] |
| CPSF4 | cleavage and polyadenylation specific factor 4, 30kDa [Source:HGNC Symbol;Acc:2327] |
| BRUNOL5 | bruno-like 5, RNA binding protein (Drosophila) [Source:HGNC Symbol;Acc:14058] |
| U2AF1L4 | U2 small nuclear RNA auxiliary factor 1-like 4 [Source:HGNC Symbol;Acc:23020] |
| 5FRS2 | splicing factor, arginine/serine-rich 2 [Source:HGNC Symbol;Acc:10783] |
| SHANK1 | SH3 and multiple ankyrin repeat domains 1 [Source:HGNC Symbol;Acc:15474] |
| LARP4 | La ribonucleoprotein domain family, member 4 [Source:HGNC Symbol;Acc:24320] |
| RAVER1 | ribonucleoprotein, PTB-binding 1 [Source:HGNC Symbol;Acc:30296] |
| SHANK2 | SH3 and multiple ankyrin repeat domains 2 [Source:HGNC Symbol;Acc:14295] |
| ELAVL4 | ELAV (embryonic lethal, abnormal vision, Drosophila)-like 4 (Hu antigen D) [Source:HGNC Symbol;Acc:3315] |
| RAVER2 | ribonucleoprotein, PTB-binding 2 [Source:HGNC Symbol;Acc:25577] |
| FUBP1 | far upstream element (FUSE) binding protein 1 [Source:HGNC Symbol;Acc:4004] |
| RBM15 | RNA binding motif protein 15 [Source:HGNC Symbol;Acc:14959] |
| DHX57 | DEAH (Asp-Glu-Ala-Asp/His) box polypeptide 57 [Source:HGNC Symbol;Acc:20086] |
| TDRD10 | tudor domain containing 10 [Source:HGNC Symbol;Acc:25316] |
| TIPARP | TCDD-inducible poly(ADP-ribose) polymerase [Source:HGNC Symbol;Acc:23696] |
| RBM47 | RNA binding motif protein 47 [Source:HGNC Symbol;Acc:30358] |
| SR140 | U2-associated protein SR140 (140 kDa Ser/Arg-rich domain protein) [Source:UniProtKB/Swiss-Prot; Acc:015042] |
| TTC14 | tetratricopeptide repeat domain 14 [Source:HGNC Symbol;Acc:24697] |
| SFMBT1 | Scm-like with four mbt domains 1 [Source:HGNC Symbol;Acc:20255] |
| LSM6 | LSM6 homolog, U6 small nuclear RNA associated (S. cerevisiae) [Source:HGNC Symbol;Acc:17017] |
| SAMD3 | sterile alpha motif domain containing 3 [Source:HGNC Symbol;Acc:21574] |
| TRA2A | transformer 2 alpha homolog (Drosophila) [Source:HGNC Symbol;Acc:16645] |
| DLC1 | deleted in livercancer 1 [Source:HGNC Symbol;Acc:2897] |
| MIR7-1 | microRNA 7-1 [Source:HGNC Symbol;Acc:31638] |
| HNRNPK | microRNA 7-1 [Source:HGNC Symbol;Acc:31638] |
| ANKS6 | ankyrin repeat and sterile alpha motif domain containing 6 [Source:HGNC Symbol];Acc:26724] |
| INPPL1 | inositol polyphosphate phosphatase-like 1 [Source:HGNC Symbol;Acc:6080] |
| ENOX2 | ecto-NOX disulfide-thiol exchanger 2 [Source:HGNC Symbol;Acc:2259] |
| LARP6 | La ribonucleoprotein domain family, member 6 [Source:HGNC Symbol;Acc:24012] |
| PPFIBP2 | PTPRF interacting protein, binding protein 2 (liprin beta 2) [Source:HGNC Symbol;Acc:9250] |
| KIAA0430 | KIAA0430 [Source:HGNC Symbol;Acc:29562] |
| RBPMS2 | RNA binding protein with multiple splicing 2 [Source:HGNC Symbol;Acc:19098] |
| SNRPD1 | small nuclear ribonucleoprotein D1 polypeptide 16kDa [Source:HGNC Symbol;Acc:11158] |
| SAMD14 | sterile alpha motif domain containing 14 [Source:HGNC Symbol;Acc:27312] |
| | Fox-1 homolog C [Source:UniProtKB/Swiss-Prot;Acc:A6NFN3] |
| STIM1 | stromal interaction molecule 1 [Source:HGNC Symbol;Acc:11386] |
| CASKIN1 | CASK interacting protein 1 [Source:HGNC Symbol;Acc:20879] |
| POLR2G | polymerase (RNA) II (DNA directed) polypeptide G [Source:HGNC Symbol;Acc:9194] |
| SF1 | splicing factor 1 [Source:HGNC Symbol;Acc:12950] |
| ANKS3 | ankyrin repeat and sterile alpha motif domain containing 3 [Source:HGNC Symbol;Acc:29422] |
| HNRNPH1 | heterogeneous nuclear ribonucleoprotein H1 (H) [Source:HGNC Symbol;Acc:5041] |
| ZRSR2 | zinc finger (CCCH type), RNA-binding motif and serina/arginine rich 2 [Source:HGNC Symbol;Acc:23019] |
| PCBP1 | poly(rC) binding protein 1 [Source:HGNC Symbol;Acc:8647] |
| RBMY1F | RNA binding motif protein, Y-linked, family 1, member F [Source:HGNC Symbol;Acc:23974] |
| HNRNPF | heterogeneous nuclear ribonucleoprotein F [Source:HGNC Symbol;Acc:5039] |
| HNRNPA3 | heterogeneous nuclear ribonucleoprotein A3 [Source:HGNC Symbol;Acc24941] |
| RBMXL2 | RNA binding motif protein, X-linked-like 2 [Source:HGNC Symbol;Acc:17886] |
| LSM3 | LSM3 homolog, U6 small nuclear RNA associated (S. cerevisiae) [Source:HGNC Symbol;Acc:17874] |
| NCBP2L | nuclear cap binding protein subunit 2-like [Source:HGNC Symbol;Acc:31795] |
| CSDC2 | cold shock domain containing C2, RNA binding [Source:HGNC Symbol;Acc:30359] |
| TAF15 | TAF15 RNA polymerase II, TATA box binding protein (TBP)-associated factor, 68kDa [Source:HGNC;Acc:11547] |
| | U6 snRNA-associated Sm-like protein LSm2 (snRNP core Sm-like protein Sm-x5) |
| | (Small nuclear ribonuclear protein D homolog)(Protein G7b) [Source;UniProtKB/Swiss-Prot;Acc:Q9Y333] |
| TNKS | tankyrase, TRF1-interacting ankyrin-related ADP-ribose polymerase [Source:HGNC Symbol;Acc:11941] |
| PHC3 | polyhomeotic homolog 3 (Drosophila) [Source:HGNC Symbol;Acc:15682] |
| RBM4B | RNA binding motif protein 4B [Source:HGNC Symbol;Acc:28842] |
| RBM4 | RNA binding motif protein 4 [Source:HGNC Symbol;Acc:9901] |
| LARP7 | La ribonucleoprotein domain family, member 7 [Source:HGNC Symbol;Acc:24912] |
| PABPC5 | poly(A) binding protein, cytoplasmic 5 [Source:HGNC Symbol;Acc:13629] |
| ANKS4B | ankyrin repeat and sterile alpha motif domain containing 4B [Source:HGNC Symbol;Acc:26795] |
| LSM1 | LSM1 homolog, U6 small nuclear RNA associated (S. cerevisiae) [Source:HGNC Symbol;Acc:20472] |
| RBMXL3 | RNA binding motif protein, X-linked-like 3 [Source:HGNC Symbol;Acc:26859] |
| MEX3C | mex-3 homolog C (C. elegans) [Source:HGNC Symbol;Acc:28040] |
| CASKIN2 | CASK interacting protein 2 [Source:HGNC Symbol;Acc:18200] |
| PPFIA3 | protein tyrosine phosphatase, receptor type, f polypeptide (PTPRF), interacting protein (liprin), alpha 3 [Acc:9247] |
| SAMD9L | sterile alpha motif domain containing 9-like [Source:HGNC Symbol;Acc:13409] |
| RBM44 | RNA binding motif protein 44 [Source:HGNC Symbol;Acc:24756] |
| SAMD12 | sterile alpha motif domain containing 12 [Source:HGNC Symbol;Acc:31750] |
| CSTF2T | cleavage stimulation factor, 3' pre-RNA, subunit 2, 64kDa, tau variant [Source:HGNC Symbol;Acc:17086] |
| HNRNPA0 | heterogeneous nuclear ribonucleoprotein A0 [Source:HGNC Symbol;Acc:5030] |
| ZC3H12D | zinc finger CCCH-type containing 12D [Source:HGNC Symbol;Acc:21175] |
| SAMD4B | sterile alpha motif domain containing 4B [Source:HGNC Symbol;Acc:25492] |
| HNRNPCL1 | heterogeneous nuclear ribonucleoprotein C-like 1 [Source:HGNC Symbol;Acc:29295] |
| MKRN3 | makorin ring finger protein 3 [Source:HGNC Symbol;Acc:7114] |
| RBM15B | RNA binding motif protein 15B [Source:HGNC Symbol;Acc:24303] |
| PUF60 | poly-U binding splicing factor 60KDa [Source:HGNC Symbol;Acc:17042] |
| TRNAU1AP | tRNA selenocysteine 1 associated protein 1 [Source:HGNC Symbol;Acc:30813] |
| SFRS2B | splicing factor, arginine/serine-rich 2B [Source:HGNC Symbol;Acc:16988] |
| MEX3D | mex-3 homolog D (C. elegans) [Source:HGNC Symbol;Acc:16734] |
| LSM10 | LSM10, U7 small nuclear RNA associated [Source:HGNC Symbol;Acc:17562] |
| SNRPE | small nuclear ribonucleoprotein polypeptide E [Source:HGNC Symbol;Acc:11161] |
| USH1G | Ushersyndrome 1G (autosomal recessive) [Source:HGNC Symbol;Acc:16356] |
| TDRKH | tudor and KH domain containing [Source:HGNC Symbol;Acc:11713] |
| EPHB3 | EPH receptor 83 [Source:HGNC Symbol;Acc:3394] |
| RBM10 | RNA binding motif protein 10 [Source:HGNC Symbol;Acc:9896] |
| LENG9 | leukocyte receptor cluster (LRC) member 9 [Source:HGNC Symbol;Acc:16306] |
| EWSR1 | Ewing sarcoma breakpoint region 1 [Source:HGNC Symbol;Acc:3508] |
| LSMD1 | LSM domain containing 1 [Source:HGNC Symbol;Acc:28212] |
| | Ephrin type-A receptor 10 Precursor(EC 2.7.10.1) [Source:UniProtKB/Swiss-Prot;Acc:Q5JZY3] |
| DND1 | dead end homolog 1 (zebrafish) [Source:HGNC Symbol;Acc:23799] |
| MEX3B | mex-3 homolog B (C. elegans) [Source:HGNC Symbol;Acc:25297] |
| PCBP3 | poly(rC) binding protein 3 [Source:HGNC Symbol;Acc:8651] |
| THOC4 | THO complex 4 [Source:HGNC Symbol;Acc:19071] |
| RBM12B | RNA binding motif protein 12B [Source:HGNC Symbol;Acc:32310] |
| SNRNP35 | small nuclear ribonucleoprotein 35kDa (U11/U12) [Source:HGNC Symbol;Acc:30852] |
| PABPC1L2B | poly(A) binding protein, cytoplasmic 1-like 2B [Source:HGNC Symbol;Acc:31852] |
| RALYL | RALY RNA binding protein-like [Source:HGNC Symbol;Acc:27036] |
| DDX53 | DEAD (Asp-Glu-Ala-Asp) box polypeptide 53 [Source:HGNC Symbol;Acc:20083] |
| RBM33 | RNA binding motif protein 33 [Source:HGNC Symbol;Acc:27223] |
| RBM43 | RNA binding motif protein 43 [Source:HGNC Symbol;Acc:24790] |
| ANK51B | ankyrin repeat and sterile alpha motif domain containing 18 [Source:HGNC Symbol;Acc:24600] |
| RBM11 | RNA binding motif protein 11 [Source:HGNC Symbol;Acc:9897] |
| ZFP36L1 | zincfinger protein 36, C3H type-like 1 [Source:HGNCSymbol;Acc:1107] |
| YTHDF3 | YTH domain family, member 3 [Source:HGNC Symbol;Acc:26465] |
| RNPC3 | RNA-binding region (RNP1, RRM) containing 3 [Source:HGNC Symbol;Acc:18666] |
| PABPC1L2A | poly(A) binding protein, cytoplasmic 1-like 2A [Source:HGNC Symbol;Acc:27989] |
| ARAP1 | ArfGAP with RhoGAP domain, ankyrin repeat and PH domain 1 [Source:HGNC Symbol;Acc:16925] |
| KIF24 | kinesin family member 24 [Source:HGNC Symbol;Acc:19916] |
| SAMD7 | sterile alpha motif domain containing 7 [Source:HGNC Symbol;Acc:25394] |
| DAZ3 | deleted in azoospermia 3 [Source:HGNC Symbol;Acc:15965] |
| RDM1 | RAD52 motif 1 [Source:HGNC Symbol;Acc:19950] |
| SAMD11 | sterile alpha motif domain containing 11 [Source:HGNC Symbol;Acc:28706] |
| LIN28B | [in-28 homolog B (C. elegans) [Source:HGNC Symbol;Acc:32207] |
| CPSF4L | cleavage and polyadenylation specific factor 4-like [Source:HGNC Symbol;Acc:33632] |
| DAZ1 | deleted in azoospermia 1 [Source:HGNC Symbol;Acc:2682] |
| ZC3H6 | zincfinger CCCH-type containing 6 [Source:HGNC Symbol;Acc:24762] |
| SFRS13A | splicing factor, arginine/serine-rich 13A [Source:HGNC Symbol;Acc:16713] |
| RBM34 | RNA binding motif protein 34 [Source:HGNC Symbol;Acc:28965] |
| RRP7A | ribosomal RNA processing 7 homolog A (S. cerevisiae) [Source:HGNC Symbol;Acc:24286] |
| | Kazrin [Source:UniProtKB/Swiss-Prot;Acc:Q674X7] |
| WDSUB1 | WD repeat, sterile alpha motif and U-box domain containing 1 [Source:HGNC Symbol;Acc:26697] |
| ELAVL3 | ELAV (embryonic lethal, abnormal vision, Drosophila)-like 3 (Hu antigen C) [Source:HGNC Symbol;Acc:3314] |
| EPHB4 | EPH receptor B4 [Source:HGNC Symbol;Acc:3395] |
| C14orf21 | Pumilio domain-containing protein C14orf21 [Source:UniProtKB/Swiss-Prot;Acc:Q86U38] |
| PCBP2 | poly(rC) binding protein 2 [Source:HGNC Symbol;Acc:8648] |
| ZGPAT | zinc finger, CCCH-type with G patch domain [Source:HGNC Symbol;Acc:15948] |
| HNRNPAB | heterogeneous nuclear ribonucleoprotein A/B [Source:HGNCSymbol;Acc:5034] |
| NOL8 | Putative uncharacterized protein ENSP00000371708Putative uncharacterized protein ENSP00000371856 [Source:UniProtKB/TrEMBL;Acc:A6NM12] |
| HELZ | nucleolar protein 8 [Source:HGNC Symbol;Acc:23387] |
| YTHDF2 | helicase with zinc finger [Source:HGNC Symbol;Acc:16878] |
| PAPSS2 | YTH domain family, member 2 [Source:HGNC Symbol;Acc:31675] |
| SFMBT2 | 3'-phosphoadenosine 5'-phosphosulfate synthase 2 [Source:HGNC Symbol;Acc:8604] |
| L3MBTL3 | Scm-iike with fourmbt domains 2 [Source:HGNC Symbol;Acc:20256] |
| SGMS1 | I(3)mbt-like 3 (Drosophila) [Source:HGNC Symbol;Acc:23035] |
| SAMD5 | sphingomyelin synthase 1 [Source:HGNC Symbol;Acc:29799] |
| MEX3A | sterile alpha motif domain containing 5 [Source:HGNC Symbol;Acc:21180] |
| RBM20 | mex-3 homologA (C. elegans) [Source:HGNC Symbol;Acc:33482] |
| DPPA5 | RNA binding motif protein 20 [Source:HGNC Symbol;Acc:27424] |
| | developmental pluripotency associated 5 [Source:HGNC Symbol;Acc:19201] |
| SAMD13 | sterile alpha motif domain containing 13 [Source:HGNC Symbol;Acc:24582] |
| MIR1236 | microRNA 1236 [Source:HGNC Symbol;Acc:33925] |
| | U6 snRNA-associated Sm-like protein LSm2 (snRNP core Sm-like protein Sm-x5)(Small nuclear ribonuclear protein D homolog)(Protein G7b) [Source:UniProtKB/Swiss-Prot;Acc:Q9Y333] |
| PPP1R10 | Serine/threonine-protein phosphatase 1 regulatory subunit 10 (Phosphatase 1 nuclear targeting subunit) (MHC class I region proline-rich protein CAT53)(FB19 protein)(PP1-binding protein of 114,kDa)(p99) |
| | [Source;UniProtKB/Swiss-Prot;Acc:Q96QC0] |
| PRR3 | Proline-rich protein 3 (MHC class I region proline-rich protein CAT56) [Source:UniProtKB/Swiss-Prot; Acc:P79522] |
| PABPN1L | poly(A) binding protein, nuclear 1-like (cytoplasmic) [Source:HGNC Symbol;Acc:37237] |
| SAMD9 | sterile alpha motif domain containing 9 [Source:HGNC Symbol;Acc:1348] |
| RNPS1 | RNA binding protein S1, serine-rich domain [Source:HGNC Symbol;Acc:10080] |
| DAZ2 | deleted in azoospermia 2 [Source:HGNC Symbol;Acc:15964] |
| HNRNPA1P4 | heterogeneous nuclear ribonucleoprotein A1 pseudogene 4 [Source:HGNC Symbol;Acc:32234] |
| MIR1236 | microRNA 1236 [Source:HGNC Symbol;Acc:33925] |
| MIR1236 | microRNA 1236 [Source:HGNC SymbolAcc:33925] |
| | Serine/threonine-protein phosphatase 1 regulatory subunit 10 (Phosphatase 1 nuclear targeting subunit) (MHC class I region proline-rich protein CAT53)(FB19 protein)(PP1-binding protein of 114 kDa)(p99) [Source:UniProtKB/Swiss-Prot;Acc:Q96QC0] |
| PRR3 | proline rich 3 [Source:HGNC Symbol;Acc:21149] |
| ZRSR1 | U2 small nuclear ribonucleoprotein auxillary factor 35 kDa subunit-related protein 1 (U2(RNU2) small nuclear RNA auxiliary factor 1-like 1)(CCCH type zincfinger, RNA-binding motif and serine/arginine rich protein 1) |
| | [Source:UniProtKB/Swiss-Prot;Acc:Q15695] |
| RBM16 | RNA binding motif protein 16 [Source:HGNC Symbol;Acc:20959] |
| | Putative uncharacterized protein ENSP00000395989 [Source:UniProtKB/TrEMBL;Acc:C9JFH9] |
| | Putative uncharacterized protein ENSP00000415385 Fragment [Source:UniProtKB/TrEMBL;Acc:C9JED4] |
| R8MXL1 | RNA binding motif protein, X-linked-like 1 [Source:HGNC Symbol;Acc:25073] |
| CPEB1 | cytoplasmic polyadenylation element binding protein 1 [Source:HGNC Symbol;Acc:21744] |
| | Putative uncharacterized protein ENSP00000381889 [Source:UniProtKB/TrEMBL;Acc:A8MVW9] |
| | Putative uncharacterized protein ENSP00000388079 Fragment [Source:UniProtKB/TrEMBL;Acc:C9JXI7] |
| | Putative uncharacterized protein ENSP00000383298 [Source:UniProtKB/TrEMBL;Acc:C9JCD7] |
| | Putative uncharacterized protein ENSP00000371708Putative uncharacterized protein ENSP00000371856; [Source:UniProtKB/TrEMBL;Acc:A6NM12] |
| | Splicing factor, arginine/serine-rich 13A (FUS-interacting serine-arginine-rich protein 1)(TLS-associated protein with Ser-Arg repeats)(TLS-associated protein with SR repeats)(TASR)(TLS-associated serine-arginine protein) (TLS-associated SR protein)(/.../ SR-repressor protein)(SRrp40)(Splicing factor SRp38) [Source:UniProtKB/ Swiss-Prot;Acc:O75494 |
| | Proline-rich protein 3 (MHC class I region proline-rich-protein CAT56) [Source: Swiss-Prot;Acc:P79522] |
| | Proline-rich protein 3 (MHC class I region proline-rich protein CAT56) [Source: Swiss-Prot;Acc:P79522] |
| RBMY2BP | RNA binding motif protein, Y-linked, family 2, member B pseudogene [Source:HGNC Symbol;Acc:9918] |
| LSM2 | LSM2 homolog, U6 small nuclear RNA associated (S. cerevisiae) [Source:HGNC Symbol;Acc:13940] |
| | Putative uncharacterized protein ENSP00000371708Putative uncharacterized protein ENSP00000371856; [Source:UniProtKB/TrEMBL;Acc:A6NM12] |
| | U6 snRNA-associated Sm-like protein LSm2 (snRNP core Sm-like protein Sm-x5)(Small nuclear ribonuclear protein D homolog)(Protein G7b) [Source:UniProtKB/Swiss-Prot;Acc:Q9Y333] |
| RBMY1J | RNA binding motif protein, Y-linked, family 1, member J [Source:HGNC Symbol;Acc:23917] |
| SNRPEL1 | small nuclear ribonucleoprotein polypeptide E-like 1 [Source:HGNC Symbol;Acc:20733] |
| | Serine/threonine-protein phosphatase 1 regulatory subunit 10 (Phosphatase 1 nuclear targeting subunit) (MHC class I region proline-rich protein CAT53)(FB19 protein)(PP1-binding protein of 114 kDa)(p99) [Source:UniProtKB/Swiss-Prot;Acc:Q96QC0] |
| | Proline-rich protein 3 (MHC class I region proline-rich protein CAT56) [Source:UniProtKB/Swiss-Prot; Acc:P79522] |
| | Proline-rich protein 3 (MHC class I region proline-rich protein CAT56) [Source:UniProtKB/Swiss-Prot; Acc:P79522] |
| MIR1236 | microRNA 1236 [Source:HGNC Symbol;Acc:33925] |
| | Serine/threonine-protein phosphatase 1 regulatory subunit 10 (Phosphatase 1 nuclear targeting subunit) (MHC class I region proline-rich protein CAT53)(FB19 protein)(PP1-bindingprotein of 114 kDa)(p99) [Source:UniProtKB/Swiss-Prot;Acc:Q96QC0] |
| MIR1236 | microRNA 1236 [Source:HGNC Symbol;Acc:33925] |
| | Putative uncharacterized protein ENSP00000403004 [Source:UniProtKB/TrEMBL;Acc:C9JHJ9] |
| | U6 snRNA-associated Sm-like protein LSm2 (snRNP core Sm-like protein Sm-x5)(Small nuclear ribonuclear protein D homolog)(Protein G7b) [Source:UniProtKB/Swiss-Prot;Acc:Q9Y333] |
| PPP1R10 | protein phosphatase 1, regulatory (inhibitor) subunit 10 [Source:HGNC Symbol;Acc:9284] |
| | Putative uncharacterized protein ENSP00000394145 Fragment [Source:UniProtKB/TrEMBL;Acc:C9JK19] |
| MCT51 | malignant T cell amplified sequence 1 [Source:HGNC Symbol;Acc:23357] |
| PABPC4L | poly(A) binding protein, cytoplasmic 4-like [Source:HGNC Symbol;Acc:31955] |
| | Proline-rich protein 3 (MHC class I region proline-rich protein CAT56)[Source:UniProtKB/Swiss-Prot; Acc:P79522] |
| MIR1236 | microRNA 1236 [Source:HGNC Symbol;Acc:33925] |
| RDBP | microRNA 1236 [Source:HGNC Symbol;Acc:33925] |
| | RNA binding motif protein, Y-linked, family 1, member A1 [Source:HGNC Symbol;Acc:9912] |
| RBMY1A1 | Serine/threonine-protein phosphatase 1 regulatory subunit 10 (Phosphatase 1 nuclear targeting subunit) (MHC class I region proline-rich protein CAT53)(FB19 protein)(PP1-binding protein of 114 kDa)(p99) [Source:UniProtKB/Swiss-Prot;AccQ96QC0] |
| | U6 snRNA-associated Sm-like protein LSm2 (snRNP core Sm-like protein Sm-x5)(Small nuclear ribonuclear protein D homolog)(Protein G7b) [Source:UniProtKB/Swiss-Prot;Acc:Q9Y333] |
| | Serine/threonine-protein phosphatase 1 regulatory subunit 10 (Phosphatase 1 nuclear targeting subunit) (MHC class I region proline-rich protein CAT53)(FB19 protein)(PP1-binding protein of 114 kDa)(p99) [Source:UniProtKB/Swiss-Prot;Acc:Q96QC0] |
| MKRNP5 | makorin ring finger protein pseudogene 5 [Source:HGNC Symbol;Acc:7115] |
| RBM14 | RNA binding motif protein 14 [Source:HGNCSymbol;Acc:14219] |
| NSUN6 | NOP2/Sun domain family, member 6 [Source:HGNC Symbol;Acc:23529] |
| UNKL | unkempt homolog (Drosophila)-like [Source:HGNC Symbol;Acc:14184] |
| RBMY1E | RNA binding motif protein, Y-linked, family 1, member E [Source:HGNC Symbol;Acc:23916] |
| HNRNPA1L2 | heterogeneous nuclear ribonucleoprotein A1-like 2 [Source:HGNC Symbol;Acc:27067] |
| RBMY1B | RNA binding motif protein, Y-linked, family 1, member B [Source:HGNC Symbol;Acc:23914] |
| RBMY1D | RNA binding motif protein, Y-linked, family 1, member 0 [Source:HGNCsymbol;Acc:23915] |
| RBM12 | RNA binding motif protein 12 [Source:HGNC Symbol;Acc:9898] |
| DAZ4 | deleted in azoospermia 4 [Source;HGNCSymbol;Acc:15966] |
| DGKQ | diacylglycerol kinase, theta 110kDa [Source:HGNC Symbol;Acc:2856] |
| FAM59A | family with sequence similarity 59, member A [Source:HGNC Symbol;Acc:26136] |
| AC021231.2 | |
| RBM17 | RNA binding motif protein 17 [Source:HGNC Symbol;Acc:16944] |
| AC026410.4 | |
| MCM3AP | minichromosome maintenance complex component 3 associated protein [Source:HGNCSymbol;Acc:6946] |
| PARN | poly(A)-specific ribonuclease (deadenylation nuclease) [Source:HGNC Symbol;Acc:8609] |
| L3MBTL | I(3)mbt-like(Drosophila) [Source:HGNC Symbol;Acc:15905] |
| PHC1B | polyhomeotic homolog 1B (Drosophila) [Source:HGNC Symbol;Acc:34502] |
| FAM59B | family with sequence similarity 59, member B [Source:HGNC Symbol;Acc:27172] |
| TLR2 | toll-like receptor 2 [Source:HGNC Symbol;Acc:11848] |
| PARP10 | Poly (ADP-ribose) polymerase family, member 10 [Source:HGNC Symbol:Acc:25895] |
| MOV10L1 | Mov10I1, Moloney leukemia virus 10-like 1, homolog (mouse) [Source:HGNC Symbol;Acc:7201] |
| CCAR1 | cell division cycle and apoptosis regulator 1 [Source:HGNC Symbol;Acc:24236] |
| LEMD3 | LEM domain containing 3 [Source:HGNC Symbol;Acc:28887] |
| UPF3B | UPF3 regulator of nonsense transcripts homolog B (yeast) [Source:HGNC Symbol;Acc:20439] |
| ZCCHC14 | zincfinger, CCHC domain containing 14 [Source:HGNC Symbol;Acc:24134] |
| RCAN2 | regulator of calcineùrin 2 [Source:H6NC Symbol;Acc:3041] |
| SRRT | serrate RNA effector molecule homolog (Arabidopsis) [Source:HGNC Symbol;Acc:24101] |
| ASZ1 | ankyrin repeat, SAM and basic leucine zipper domain containing 1 [Source:HGNC Symbol;Acc:1350] |
| ZC3H12A | zinc finger CCCH-type containing 12A [Source:HGNC Symbol;Acc:26259] |
| ZC3HAV1L | zinc finger CCCH-type,antiviral 1-like [Source:HGNC Symbol;Acc:22423] |
| PARP14 | poly (ADP-ribose) polymerase family, member 14 [Source:HGNC Symbol;Acc:29232] |
| TMEM63A | transmembrane protein 63A [Source:HGNC Symbol;Acc:29118] |
| STARD13 | StAR-related lipid transfer (START) domain containing 13 [Source:HGNC Symbol;Acc:19164] |
| GTF3A | general transcription factor IIIA [Source:HGNC Symbol;Acc:4662] |
| NUFIP1 | nuclear fragile X mental retardation protein interacting protein 1 [Source:HGNC Symbol;Acc:8057] |
| SRRM2 | serine/arginine repetitive matrix 2 [Source:HGNC Symbol;Acc:16639] |
| APTX | aprataxin [Source:HGNC Symbol;Acc:15984] |
| ZFR | zinc finger RNA binding protein [Source:HGNG Symbol;Acc:17277] |
| UPF1 | UPF1 regulator of nonsense transcripts homolog (yeast) [Source:HGNC Symbol;Acc:9962] |
| KIN | KIN, antigenic determinant of recA protein homolog (mouse) [Source:HGNC Symbol;Acc:6327] |
| ZNF239 | zinc finger protein 239 [Source:HGNC Symbol;Acc:13031] |
| ZNF74 | zincfinger protein 74[Source:HGNC Symbol;Acc:13144] |
| SNRPC | small nuclear ribonucleoprotein polypeptide C [source:HGNC Symbol;Acc:11157] |
| C1orf25 | TRM1-like protein [Source:UniProtKB/Swiss-Prot;Acc:Q7Z2T5] |
| ZFR2 | zinc finger RNA binding protein 2 [Source:HGNC Symbol;Acc:29189] |
| IREB2 | iron-responsive element binding protein 2 [Source:HGNC Symbol;Acc:6115] |
| ACO1 | aconitase 1, soluble [Source:HGNC Symbol;Acc:117] |
| TROVE2 | TROVE domain family, member 2 [Source:HGNC Symbol;Acc:11313] |
| TEP1 | telomerase-associated protein 1 [Source:HGNC Symbol;Acc:11726] |
| GAPDH | glyceraldehyde-3-phosphate dehydrogenase [Source:HGNC Symbol;Acc:4141] |
| ZRANB2 | zinc finger, RAN-binding domain containing 2 [Source:HGNC Symbol;Acc:13058] |
| SLBP | stem-loop binding protein [Source:HGNC Symbol;Acc:10904] |
| GW182 | Trinucleotide repeat-containing gene 6A protein [Source: UniProtK8, Acc: Q8NDV7] |
| TNRC6B | Trinucleotide repeat-containing gene 6B protein [Source: UniProtKB, Acc: Q9UPQ9) |
| TNRC6C | Trinucleotide repeat-containing gene 6C protein [Source: UniProtKB, Acc: Q9HCJ0] |
| TAR8P2 | RISC-loading complex subunit TARBP2 [Source:UniProtKB,Acc:Q15633] |
| STAR | Steroidogenic acute regulatory protein, mitochondrial [Source: UniProtKB, Acc: P49675] |
| AGO1 | Protein AGO1 [Source: UniProtKB, Acc: Q5TA58] |
| TRIM1 | Probable E3 ubiquitin-protein ligase MID2 [Source: UniProtKB, Acc: Q9UJV3] |
| FOX1 | RNA binding protein fox-1 homolog 1 [Source: UniProtKB, Acc: Q9NW81] |
| FOX2 | RNA binding protein fox-1 homolog 2 [Source: UniProtKB, Acc: 043251] |
| FOX3 | Polyprotein [Source: UniProtKB, Acc: Q8QNU0] |
| DGCR8 | Microprocessor complex subunit DGCR8 [Source: UniProtKB, Acc: Q8WYQ5] |
| Dicer1 | Endoribonuclease Dicer, Helicase with RNase motif [Source: UniProtKB, Acc: Q9UPY3] |
| Drosha | Ribonuclease 3 [Source: UniProtKB, Acc: Q9NRR4] |
| DDx6 | Probable ATP-dependent RNA helicase DDX6 [Source: UniProtKB, Acc: P26196] |
| DDx17 | Probable ATP-dependent RNA helicase DDX17 [Source: UniProtKB, Acc: Q92841] |
| MOV10 | Putative helicase MOV-10 [Source: UniProtK8, Acc: Q9HCE1] |
| DDx20 | Probable ATP-dependent RNA helicase DDX20 [Source: UniProtKB, Acc: Q9UHI6] |
| DDx42 | ATP-dependent RNA helicase DDX24 [Source: UniProtKB, Acc: Q9GZR7] |

**Table 2a (preferred miRNAs for practicing the present invention)**

| miRNA designation (e.g. has-let-7a-1), sequence reference (MI...) | | |
|---|---|---|
| hsa-let-7a-1 MI0000060 | hsa-mir-149 MI0000478 | hsa-mir-3149 MI0014176 |
| hsa-let-7a-2 MI0000061 | hsa-mir-150 MI0000479 | hsa-mir-3150a MI0014177 |
| hsa-let-7a-3 M10000062 | hsa-mir-151a MI0000809 | hsa-mir-3150b MI0016426 |
| hsa-let-7b MI0000063 | hsa-mir-151b MI0003772 | hsa-mir-3151 MI0014178 |
| hsa-let-7c MI0000064 | hsa-mir-152 MI0000462 | hsa-mir-3152 MI0014179 |
| hsa-let-7d MI0000065 | hsa-mir-153-1 MI0000463 | hsa-mir-3153 MI0014180 |
| hsa-let-7e MI0000066 | hsa-mir-153-2 MI0000464 | hsa-mir-3154 MI0014182 |
| hsa-let-7f-1 MI0000067 | hsa-mir-1537 MI0007258 | hsa-mir-3155a MI0014183 |
| hsa-let-7f-2 MI0000068 | hsa-mir-1538 MI0007259 | hsa-mir-3155b MI0016839 |
| hsa-let-7g MI0000433 | hsa-mir-1539 MI0007260 | hsa-mir-3156-1 MI0014184 |
| hsa-let-7i MI0000434 | hsa-mir-154 MI0000480 | hsa-mir-3156-2 MI0014230 |
| hsa-mir-100 MI0000102 | hsa-mir-155 MI0000681 | hsa-mir-3156-3 MI0014242 |
| hsa-mir-101-1 MI0000103 | hsa-mir-1587 MI0016905 | hsa-mir-3157 MI0014185 |
| hsa-mir-101-2 MI0000739 | hsa-mir-15a MI0000069 | hsa-mir-3158-1 MI0014186 |
| hsa-mir-103a-1 MI0000109 | hsa-mir-15b MI0000438 | hsa-mir-3158-2 MI0014187 |
| hsa-mir-103a-2 MI0000108 | hsa-mir-16-1 MI0000070 | hsa-mir-3159 MI0014188 |
| hsa-mir-103b-1 MI0007261 | hsa-mir-16-2 MI0000115 | hsa-mir-3160-1 MI0014189 |
| hsa-mir-103b-2 MI0007262 | hsa-mir-17 MI0006071 | hsa-mir-3160-2 MI0014190 |
| hsa-mir-105-1 MI0000111 | hsa-mir-181a-1 MI0000289 | hsa-mir-3161 MI0014191 |
| hsa-mir-105-2 MI0000112 | hsa-mir-181a-2 MI0000269 | hsa-mir-3162 MI0014192 |
| hsa-mir-106a MI0000113 | hsa-mir-181b-1 MI0000270 | hsa-mir-3163 MI0014193 |
| hsa-mir-106b MI0000734 | hsa-mir-181b-2 MI0000683 | hsa-mir-3164 MI0014194 |
| hsa-mir-107 MI0000114 | hsa-mir-181c MI0000271 | hsa-mir-3165 MI0014195 |
| hsa-mir-10a MI0000266 | hsa-mir-181d MI0003139 | hsa-mir-3166 MI0014196 |
| hsa-mir-10b MI0000267 | hsa-mir-182 MI0000272 | hsa-mir-3167 MI0014198 |
| hsa-mir-1-1 MI0000651 | hsa-mir-1825 MI0008193 | hsa-mir-3168 MI0014199 |
| hsa-mir-1178 MI0006271 | hsa-mir-1827 MI0008195 | hsa-mir-3169 MI0014200 |
| hsa-mir-1179 MI0006272 | hsa-mir-183 MI0000273 | hsa-mir-3170 MI0014201 |
| hsa-mir-1180 MI0006273 | hsa-mir-184 MI0000481 | hsa-mir-3171 MI0014202 |
| hsa-mir-1181 MI0006274 | hsa-mir-185 MI0000482 | hsa-mir-3173 MI0014204 |
| hsa-mir-1182 MI0006275 | hsa-mir-186 MI0000483 | hsa-mir-3174 MI0014208 |
| hsa-mir-1183 MI0006276 | hsa-mir-187 MI0000274 | hsa-mir-3175 MI0014209 |
| hsa-mir-1184-1 MI0006277 | hsa-mir-188 MI0000484 | hsa-mir-3176 MI0014210 |
| hsa-mir-1184-2 MI0015971 | hsa-mir-18a MI0000072 | hsa-mir-3177 MI0014211 |
| hsa-mir-1184-3 MI0015972 | hsa-mir-18b MI0001518 | hsa-mir-3178 MI0014212 |
| hsa-mir-1185-1 MI0003844 | hsa-mir-1908 MI0008329 | hsa-mir-3179-1 MI0014213 |
| hsa-mir-1185-2 MI0003821 | hsa-mir-1909 MI0008330 | hsa-mir-3179-2 MI0014216 |
| hsa-mr-1193 MI0014205 | hsa-mir-190a MI0000486 | hsa-mir-3179-3 MI0014221 |
| hsa-mir-1197 MI0006656 | hsa-mir-190b MI0005545 | hsa-mir-3180-1 MI0014214 |
| hsa-mir-1-2 MI0000437 | hsa-mir-191 MI0000465 | hsa-mir-3180-2 MI0014215 |
| hsa-mir-1200 MI0006332 | hsa-mir-1910 MI0008331 | hsa-mir-3180-3 MI0014217 |
| hsa-mir-1202 MI0006334 | hsa-mir-1911 MI0008332 | hsa-mir-3180-4 MI0016408 |
| hsa-mir-1203 MI0006335 | hsa-mir-1912 MI0008333 | hsa-mir-3180-5 MI0016409 |
| hsa-mir-1204 MI0006337 | hsa-mir-1913 MI0008334 | hsa-mir-3181 MI0014223 |
| hsa-mir-1205 MI0006338 | hsa-mir-1914 MI0008335 | hsa-mir-3182 MI0014224 |
| hsa-mir-1206 MI0006339 | hsa-mir-1915 MI0008336 | hsa-mir-3183 MI0014225 |
| hsa-mir-1207 MI0006340 | hsa-mir-192 MI0000234 | hsa-mir-3184 MI0014226 |
| hsa-mir-1208 MI0006341 | hsa-mir-193a MI0000487 | hsa-mir-3185 MI0014227 |
| hsa-mir-122 MI0000442 | hsa-mir-193b MI0003137 | hsa-mir-3186 MI0014229 |
| hsa-mir-1224 MI0003764 | hso-mir-194-1 MI0000488 | hsa-mir-3187 MI0014231 |
| hsa-mir-1225 MI0006311 | hsa-mir-194-2 MI0000732 | hsa-mir-3188 MI0014232 |
| hsa-mir-1226 MI0006313 | hsa-mir-195 MI0000489 | hsa-mir-3189 MI0014233 |
| hsa-mir-1227 MI0006316 | hsa-mir-196a-1 MI0000238 | hsa-mir-3190 MI0014235 |
| hsa-mir-1228 MI0006318 | hsa-mir-196a-2 MI0000279 | hsa-mir-3191 MI0014236 |
| hsa-mir-1229 MI0006319 | hsa-mir-196b MI0001150 | hsa-mir-3192 MI0014237 |
| hsa-mir-1231 MI0006321 | hsa-mir-197 MI0000239 | hsa-mir-3193 MI0014238 |
| hsa-mir-1233-1 MI0006323 | hsa-mir-1972-1 MI0009982 | hsa-mir-3194 MI0014239 |
| hsa-mir-1233-2 MI0015973 | hsa-mir-1972-2 MI0015977 | hsa-mir-3195 MI0014240 |
| hsa-mir-1234 MI0006324 | hsa-mir-1973 MI0009983 | hsa-mir-3196 MI0014241 |
| hsa-mir-1236 MI0006326 | hsa-mir-1976 MI0009986 | hsa-mir-3197 MI0014245 |
| hsa-mir-1237 MI0006327 | hsa-mir-198 MI0000240 | hsa-mir-3198-1 MI0014246 |
| hsa-mir-1238 MI0006328 | hsa-mir-199a-1 MI0000242 | hsa-mir-3198-2 MI0017335 |
| hsa-mir-124-1 MI0000443 | hsa-mir-199a-2 MI0000281 | hsa-mir-3199-1 MI0014247 |
| hsa-mir-124-2 MI0000444 | hsa-mir-199b MI0000282 | hsa-mir-3199-2 MI0014248 |
| hsa-mir-124-3 MI0000445 | hsa-mir-19a MI0000073 | hsa-mir-32 MI0000090 |
| hsa-mir-1243 MI0006373 | hsa-mir-19b-1 MI0000074 | hsa-mir-3200 MI0014249 |
| hsa-mir-1244-1 MI0008379 | hsa-mir-19b-2 MI0000075 | hsa-mir-3201 MI0014250 |
| hsa-mir-1244-2 MI0015974 | hsa-mir-200a MI0000737 | hsa-mir-3202-1 MI0014252 |
| hsa-mir-1244-3 MI0015975 | hsa-mir-200b MI0000342 | hsa-mir-3202-2 MI0014253 |
| hsa-mir-1245a MI0006380 | hsa-mir-200c MI0000650 | hsa-mir-320a MI0000542 |
| hsa-mir-1245b MI0017431 | hsa-mir-202 MI0003130 | hsa-mir-320b-1 MI0003776 |
| hsa-mir-1246 MI0006381 | hsa-mir-203a MI0000283 | hsa-mir-320b-2 MI0003839 |
| hsa-mir-1247 MI0006382 | hsa-mir-203b MI0017343 | hsa-mir-320c-1 MI0003778 |
| hsa-mir-1248 MI0006383 | hsa-mir-204 MI0000284 | hsa-mir-320c-2 MI0008191 |
| hsa-mir-1249 MI0006384 | hsa-mir-205 MI0000285 | hsa-mir-320d-1 MI0008190 |
| hsa-mir-1250 MI0006385 | hsa-mir-2052 MI0010486 | hsa-mir-320d-2 MI0008192 |
| hsa-mir-1251 MI0006386 | hsa-mir-2053 MI0010487 | hsa-mir-320e MI0014234 |
| hsa-mir-1252 MI0006434 | hsa-mir-2054 MI0010488 | hsa-mir-323a MI0000807 |
| hsa-mir-1253 MI0006387 | hsa-mir-206 MI0000490 | hsa-mir-323b MI0014206 |
| hsa-mir-1254-1 MI0006388 | hsa-mir-208a MI0000251 | hsa-mir-324 MI0000813 |
| hsa-mir-1254-2 MI0016747 | hsa-mir-208b MI0005570 | hsa-mir-325 MI0000824 |
| hsa-mir-1255a MI0006389 | hsa-mir-20a MI0000076 | hsa-mir-326 MI0000808 |
| hsa-mir-1255b-1 MI0006435 | hsa-mir-20b MI0001519 | hsa-mir-328 MI0000804 |
| hsa-mir-1255b-2 MI0006436 | hsa-mir-21 MI0000077 | hsa-mir-329-1 MI0001725 |
| hsa-mir-1256 MI0006390 | hsa-mir-210 MI0000286 | hsa-mir-329-2 MI0001726 |
| hsa-mir-1257 MI0006391 | hsa-mir-211 MI0000287 | hsa-mir-330 MI0000803 |
| hsa-mir-1258 MI0006392 | hsa-mir-2110 MI0010629 | hsa-mir-331 MI0000812 |
| hsa-mir-125a MI0000469 | hsa-mir-2113 MI0003939 | hsa-mir-335 MI0000816 |
| hsa-mir-125b-1 MI0000446 | hsa-mir-2114 MI0010633 | hsa-mir-337 MI0000806 |
| hsa-mir-125b-2 MI0000470 | hsa-mir-2115 MI0010634 | hsa-mir-338 MI0000814 |
| hsa-mir-126 MI0000471 | hsa-mir-2116 MI0010635 | hsa-mir-339 MI0000815 |
| hsa-mir-1260a MI0006394 | hsa-mir-2117 MI0010636 | hsa-mir-33a MI0000091 |
| hsa-mir-1260b MI0014197 | hsa-mir-212 MI0000288 | hsa-mir-33b MI0003646 |
| hsa-mir-1261 MI0006396 | hsa-mir-214 MI0000290 | hsa-mir-340 MI0000802 |
| hsa-mir-1262 MI0006397 | hsa-mir-215 MI0000291 | hsa-mir-342 MI0000805 |
| hsa-mir-1263 MI0006398 | hsa-mir-216a MI0000292 | hsa-mir-345 MI0000825 |
| hsa-mir-1264 MI0003758 | hsa-mir-216b MI0005569 | hsa-mir-346 MI0000826 |
| hsa-mir-1265 MI0006401 | hsa-mir-217 MI0000293 | hsa-mir-34a MI0000268 |
| hsa-mir-1266 MI0006403 | hsa-mir-218-1 MI0000294 | hsa-mir-34b MI0000742 |
| hsa-mir-1267 MI0006404 | hsa-mir-218-2 MI0000295 | hsa-mir-34c MI0000743 |
| hsa-mir-1268a MI0006405 | hsa-mir-219-1 MI0000296 | hsa-mir-3529 MI0017351 |
| hsa-mir-1268b MI0016748 | hsa-mir-219-2 MI0000740 | hsa-mir-3591 MI0017383 |
| hsa-mir-1269a MI0006406 | hsa-mir-22 MI0000078 | hsa-mir-3605 MI0015995 |
| hsa-mir-1269b MI0016888 | hsa-mir-221 MI0000298 | hsa-mir-3606 MI0015996 |
| hsa-mir-127 MI0000472 | hsa-mir-222 MI0000299 | hsa-mir-3607 MI0015997 |
| hsa-mir-1270-1 MI0006407 | hsa-mir-223 MI0000300 | hsa-mir-3609 MI0015999 |
| hsa-mir-1270-2 MI0015976 | hsa-mir-224 MI0000301 | hsa-mir-361 MI0000760 |
| hsa-mir-1271 MI0003814 | hsa-mir-2276 MI0011282 | hsa-mir-3610 MI0016000 |
| hsa-mir-1272 MI0006408 | hsa-mir-2277 MI0011284 | hsa-mir-3611 MI0016001 |
| hsa-mir-1273a MI0006409 | hsa-mir-2278 MI0011285 | hsa-mir-3612 MI0016002 |
| hsa-mir-1273c MI0014171 | hsa-mir-2355 MI0015873 | hsa-mir-3613 MI0016003 |
| hsa-mir-1273d MI0014254 | hsa-mir-2392 MI0016870 | hsa-mir-3614 MI0016004 |
| hsa-mir-1273e MI0016059 | hsa-mir-23a MI0000079 | hsa-mir-3615 MI0016005 |
| hsa-mir-1273f MI0018002 | hsa-mir-23b MI0000439 | hsa-mir-3616 MI0016006 |
| hsa-mir-1273g MI0018003 | hsa-mir-23c MI0016010 | hsa-mir-3617 MI0016007 |
| hsa-mir-1275 MI0006415 | hsa-mir-24-1 MI0000080 | hsa-mir-3618 MI0016008 |
| hsa-mir-1276 MI0006416 | hsa-mir-24-2 MI0000081 | hsa-mir-3619 MI0016009 |
| hsa-mir-1277 MI0006419 | hsa-mir-2467 MI0017432 | hsa-mir-362 MI0000762 |
| hsa-mir-1278 MI0006425 | hsa-mir-25 MI0000082 | hsa-mir-3620 MI0016011 |
| hsa-mir-1279 MI0006426 | hsa-mir-2681 MI0012062 | hsa-mir-3621 MI0016012 |
| hsa-mir-128-1 MI0000447 | hsa-mir-2682 MI0012063 | hsa-mir-3622a MI0016013 |
| hsa-mir-1281 MI0006428 | hsa-mir-26a-1 MI0000083 | hsa-mir-3622b MI0016014 |
| hsa-mir-128-2 MI0000727 | hsa-mir-26a-2 MI0000750 | hsa-mir-363 MI0000764 |
| hsa-mir-1282 MI0006429 | hsa-mir-26b MI0000084 | hsa-mir-3646 MI0016046 |
| hsa-mir-1283-1 MI0003832 | hsa-mir-27a MI0000085 | hsa-mir-3648 MI0016048 |
| hsa-mir-1283-2 MI0006430 | hsa-mir-27b MI0000440 | hsa-mir-3649 MI0016049 |
| hsa-mir-1284 MI0006431 | hsa-mir-28 MI0000086 | hsa-mir-3650 MI0016050 |
| hsa-mir-1285-1 MI0006346 | hsa-mir-2861 MI0013006 | hsa-mir-3651 MI0016051 |
| hsa-mir-1285-2 MI0006347 | hsa-mir-2909 MI0013083 | hsa-mir-3652 MI0016052 |
| hsa-mir-1286 MI0006348 | hsa-mir-296 MI0000747 | hsa-mir-3653 MI0016053 |
| hsa-mir-1287 MI0006349 | hsa-mir-2964a MI0017299 | hsa-mir-3654 MI0016054 |
| hsa-mir-1288 MI0006432 | hsa-mir-297 MI0005775 | hsa-mir-3655 MI0016055 |
| hsa-mir-1289-1 MI0006350 | hsa-mir-298 MI0005523 | hsa-mir-3656 MI0016056 |
| hsa-mir-1289-2 MI0006351 | hsa-mir-299 MI0000744 | hsa-mir-3657 MI0016057 |
| hsa-mir-1290 MI0006352 | hsa-mir-29a MI0000087 | hsa-mir-3658 MI0016058 |
| hsa-mir-129-1 MI0000252 | hsa-mir-29b-1 MI0000105 | hsa-mir-3659 MI0016060 |
| hsa-mir-1291 MI0006353 | hsa-mir-29b-2 MI0000107 | hsa-mir-365a MI0000767 |
| hsa-mir-129-2 MI0000473 | hsa-mir-29c MI0000735 | hsa-mir-365b MI0000769 |
| hsa-mir-1292 MI0006433 | hsa-mir-300 MI0005525 | hsa-mir-3660 MI0016061 |
| hsa-mir-1293 MI0006355 | hsa-mir-301a MI0000745 | hsa-mir-3661 MI0016062 |
| hsa-mir-1294 MI0006356 | hsa-mir-301b MI0005568 | hsa-mir-3662 MI0016063 |
| hsa-mir-1295a MI0006357 | hsa-mir-302a MI0000738 | hsa-mir-3663 MI0016064 |
| hsa-mir-1295b MI0019146 | hsa-mir-302b MI0000772 | hsa-mir-3664 MI0016065 |
| hsa-mir-1296 MI0003780 | hsa-mir-302c MI0000773 | hsa-mir-3665 MI0016066 |
| hsa-mir-1297 MI0006358 | hsa-mir-302d MI0000774 | hsa-mir-3666 MI0016067 |
| hsa-mir-1298 MI0003938 | hsa-mir-302e MI0006417 | hsa-mir-3667 MI0016068 |
| hsa-mir-1299 MI0006359 | hsa-mir-302f MI0006418 | hsa-mir-3668 MI0016069 |
| hsa-mir-1301 MI0003815 | hsa-mir-3064 MI0017375 | hsa-mir-3669 MI0016070 |
| hsa-mir-1302-1 MI0006362 | hsa-mir-3065 MI0014228 | hsa-mir-367 MI0000775 |
| hsa-mir-1302-10 MI0015979 | hsa-mir-3074 MI0014181 | hsa-mir-3670-1 MI0016071 |
| hsa-mir-1302-11 MI0015980 | hsa-mir-30a MI0000088 | hsa-mir-3670-2 MI0019112 |
| hsa-mir-1302-2 MI0006363 | hsa-mir-30b MI0000441 | hsa-mir-3671 MI0016072 |
| hsa-mir-1302-3 MI0006364 | hsa-mir-30c-1 MI0000736 | hsa-mir-3672 MI0016073 |
| hsa-mir-1302-4 MI0006365 | hsa-mir-30c-2 MI0000254 | hsa-mir-3673 MI0016074 |
| hsa-mir-1302-5 MI0006366 | hsa-mir-30d MI0000255 | hsa-mir-3674 MI0016075 |
| hsa-mir-1302-6 MI0006367 | hsa-mir-30e MI0000749 | hsa-mir-3675 MI0016076 |
| hsa-mir-1302-7 MI0006368 | hsa-mir-31 MI0000089 | hsa-mir-3676 MI0016077 |
| hsa-mir-1302-8 MI0006369 | hsa-mir-3115 MI0014127 | hsa-mir-3677 MI0016078 |
| hsa-mir-1302-9 MI0015978 | hsa-mir-3116-1 MI0014128 | hsa-mir-3678 MI0016079 |
| hsa-mir-1303 MI0006370 | hsa-mir-3116-2 MI0014129 | hsa-mir-3679 MI0016080 |
| hsa-mir-1304 MI0006371 | hsa-mir-3117 MI0014130 | hsa-mir-3680-1 MI0016081 |
| hsa-mir-1305 MI0006372 | hsa-mir-3118-1 MI0014131 | hsa-mir-3680-2 MI0019113 |
| hsa-mir-1306 MI0006443 | hsa-mir-3118-2 MI0014132 | hsa-mir-3681 MI0016082 |
| hsa-mir-1307 MI0006444 | hsa-mir-3118-3 MI0014133 | hsa-mir-3682 MI0016083 |
| hsa-mir-130a MI0000448 | hsa-mir-3118-4 MI0014207 | hsa-mir-3683 MI0016084 |
| hsa-mir-130b MI0000748 | hsa-mir-3118-5 MI0014243 | hsa-mir-3684 MI0016085 |
| hsa-mir-132 MI0000449 | hsa-mir-3118-6 MI0015981 | hsa-mir-3685 MI0016086 |
| hsa-mir-1321 MI0006652 | hsa-mir-3119-1 MI0014134 | hsa-mir-3686 MI0016087 |
| hsa-mir-1322 MI0006653 | hsa-mir-3119-2 MI0014135 | hsa-mir-3687 MI0016088 |
| hsa-mir-1323 MI0003786 | hsa-mir-3120 MI0014136 | hsa-mir-3688-1 MI0016089 |
| hsa-mir-1324 MI0006657 | hsa-mir-3121 MI0014137 | hsa-mir-3688-2 MI0017447 |
| hsa-mir-133a-1 MI0000450 | hsa-mir-3122 MI0014138 | hsa-mir-3689a MI0016090 |
| hsa-mir-133a-2 MI0000451 | hsa-mir-3123 MI0014139 | hsa-mir-3689b MI0016411 |
| hsa-mir-133b MI0000822 | hsa-mir-3124 MI0014140 | hsa-mir-3689c MI0016832 |
| hsa-mir-134 MI0000474 | hsa-mir-3125 MI0014142 | hsa-mir-3689d-1 MI0016834 |
| hsa-mir-1343 MI0017320 | hsa-mir-3126 MI0014143 | hsa-mir-3689d-2 MI0016835 |
| hsa-mir-135a-1 MI0000452 | hsa-mir-3127 MI0014144 | hsa-mir-3689e MI0016836 |
| hsa-mir-135a-2 MI0000453 | hsa-mir-3128 MI0014145 | hsa-mir-3689f MI0016837 |
| hsa-mir-135b MI0000810 | hsa-mir-3129 MI0014146 | hsa-mir-369 MI0000777 |
| hsa-mir-136 MI0000475 | hsa-mir-3130-1 MI0014147 | hsa-mir-3690-1 MI0016091 |
| hsa-mir-137 MI0000454 | hsa-mir-3130-2 MI0014148 | hsa-mir-3690-2 MI0023561 |
| hsa-mir-138-1 MI0000476 | hsa-mir-3131 MI0014151 | hsa-mir-3691 MI0016092 |
| hsa-mir-138-2 MI0000455 | hsa-mir-3132 MI0014152 | hsa-mir-3692 MI0016093 |
| hsa-mir-139 MI0000261 | hsa-mir-3133 MI0014153 | hsa-mir-370 MI0000778 |
| hsa-mir-140 MI0000456 | hsa-mir-3134 MI0014155 | hsa-mir-3713 MI0016134 |
| hsa-mir-141 MI0000457 | hsa-mir-3135a MI0014156 | hsa-mir-3714 MI0016135 |
| hsa-mir-142 MI0000458 | hsa-mir-3135b MI0016809 | hsa-mir-371a MI0000779 |
| hsa-mir-143 MI0000459 | hsa-mir-3136 MI0014158 | hsa-mir-371b MI0017393 |
| hsa-mir-144 MI0000460 | hsa-mir-3137 MI0014160 | hsa-mir-372 MI0000780 |
| hsa-mir-145 MI0000461 | hsa-mir-3138 MI0014161 | hsa-mir-373 MI0000781 |
| hsa-mir-1468 MI0003782 | hsa-mir-3139 MI0014162 | hsa-mir-374a MI0000782 |
| hsa-mir-1469 MI0007074 | hsa-mir-3140 MI0014163 | hsa-mir-374b MI0005566 |
| hsa-mir-146a MI0000477 | hsa-mir-3141 MI0014165 | hsa-mir-374c MI0016684 |
| hsa-mir-146b MI0003129 | hsa-mir-3142 MI0014166 | hsa-mir-375 MI0000783 |
| hsa-mir-1470 MI0007075 | hsa-mir-3143 MI0014167 | hsa-mir-376a-1 MI0000784 |
| hsa-mir-1471 MI0007076 | hsa-mir-3144 MI0014169 | hsa-mir-376a-2 MI0003529 |
| hsa-mir-147a MI0000262 | hsa-mir-3145 MI0014170 | hsa-mir-376b MI0002466 |
| hsa-mir-147b MI0005544 | hsa-mir-3146 MI0014172 | hsa-mir-376c MI0000776 |
| hsa-mir-148a MI0000253 | hsa-mir-3147 MI0014173 | hsa-mir-377 MI0000785 |
| hsa-mir-148b MI0000811 | hsa-mir-3148 MI0014175 | hsa-mir-378a MI0000786 |

**Table 2b**

| | | |
|---|---|---|
| hsa-mir-4454 MI0016800 | hsa-mir-4721 MI0017356 | hsa-mir-522 MI0003177 |
| hsa-mir-4455 MI0016801 | hsa-mir-4722 MI0017357 | hsa-mir-523 MI0003153 |
| hsa-mir-4456 MI0016802 | hsa-mir-4723 MI0017359 | hsa-mir-524 MI0003160 |
| hsa-mir-4457 MI0016803 | hsa-mir-4724 MI0017361 | hsa-mir-525 MI0003152 |
| hsa-mir-4458 MI0016804 | hsa-mir-4725 MI0017362 | hsa-mir-526a-1 MI0003157 |
| hsa-mir-4459 MI0016805 | hsa-mir-4726 MI0017363 | hsa-mir-526a-2 MI0003168 |
| hsa-mir-4460 MI0016806 | hsa-mir-4727 MI0017364 | hsa-mir-526b MI0003150 |
| hsa-mir-4461 MI0016807 | hsa-mir-4728 MI0017365 | hsa-mir-527 MI0003179 |
| hsa-mir-4462 MI0016810 | hsa-mir-4729 MI0017366 | hsa-mir-532 MI0003205 |
| hsa-mir-4463 MI0016811 | hsa-mir-4730 MI0017367 | hsa-mir-539 MI0003514 |
| hsa-mir-4464 MI0016812 | hsa-mir-4731 MI0017368 | hsa-mir-541 MI0005539 |
| hsa-mir-4465 MI0016816 | hsa-mir-4732 MI0017369 | hsa-mir-542 MI0003686 |
| hsa-mir-4466 MI0016817 | hsa-mir-4733 MI0017370 | hsa-mir-543 MI0005565 |
| hsa-mir-4467 MI0016818 | hsa-mir-4734 MI0017371 | hsa-mir-544a MI0003515 |
| hsa-mir-4468 MI0016819 | hsa-mir-4735 MI0017372 | hsa-mir-544b MI0014159 |
| hsa-mir-4469 MI0016820 | hsa-mir-4736 MI0017373 | hsa-mir-545 MI0003516 |
| hsa-mir-4470 MI0016821 | hsa-mir-4737 MI0017374 | hsa-mir-548a-1 MI0003593 |
| hsa-mir-4471 MI0016822 | hsa-mir-4738 MI0017376 | hsa-mir-548a-2 MI0003598 |
| hsa-mir-4472-1 MI0016823 | hsa-mir-4739 MI0017377 | hsa-mir-548a-3 MI0003612 |
| hsa-mir-4472-2 MI0016824 | hsa-mir-4740 MI0017378 | hsa-mir-548aa-1 MI0016689 |
| hsa-mir-4473 MI0016825 | hsa-mir-4741 MI0017379 | hsa-mir-548aa-2 MI0016690 |
| hsa-mir-4474 MI0016826 | hsa-mir-4742 MI0017380 | hsa-mir-548ab MI0016752 |
| hsa-mir-4475 MI0016827 | hsa-mir-4743 MI0017381 | hsa-mir-548ac MI0016762 |
| hsa-mir-4476 MI0016828 | hsa-mir-4744 M10017382 | hsa-mir-548ad MI0016770 |
| hsa-mir-4477a MI0016829 | hsa-mir-4745 MI0017384 | hsa-mir-548ae-1 MI0016779 |
| hsa-mir-4477b MI0016830 | hsa-mir-4746 MI0017385 | hsa-mir-548ae-2 MI0016780 |
| hsa-mir-4478 MI0016831 | hsa-mir-4747 MI0017386 | hsa-mir-548ag-1 MI0016793 |
| hsa-mir-4479 MI0016838 | hsa-mir-4748 MI0017387 | hsa-mir-548ag-2 MI0016794 |
| hsa-mir-448 MI0001637 | hsa-mir-4749 MI0017388 | hsa-mir-548ah MI0016796 |
| hsa-mir-4480 MI0016841 | hsa-mir-4750 MI0017389 | hsa-mir-548ai MI0016813 |
| hsa-mir-4481 MI0016842 | hsa-mir-4751 MI0017390 | hsa-mir-548aj-1 MI0016814 |
| hsa-mir-4482 MI0016843 | hsa-mir-4752 MI0017391 | hsa-mir-548aj-2 MI0016815 |
| hsa-mir-4483 MI0016844 | hsa-mir-4753 MI0017392 | hsa-mir-548ak MI0016840 |
| hsa-mir-4484 MI0016845 | hsa-mir-4754 MI0017394 | hsa-mir-548al MI0016851 |
| hsa-mir-4485 MI0016846 | hsa-mir-4755 MI0017395 | hsa-mir-548am MI0016904 |
| hsa-mir-4486 MI0016847 | hsa-mir-4756 MI0017397 | hsa-mir-548an MI0016907 |
| hsa-mir-4487 MI0016848 | hsa-mir-4757 MI0017398 | hsa-mir-548ao MI0017871 |
| hsa-mir-4488 MI0016849 | hsa-mir-4758 MI0017399 | hsa-mir-548ap MI0017875 |
| hsa-mir-4489 MI0016850 | hsa-mir-4759 MI0017400 | hsa-mir-548aq MI0019130 |
| hsa-mir-4490 MI0016852 | hsa-mir-4760 MI0017401 | hsa-mir-548ar MI0019131 |
| hsa-mir-4491 MI0016853 | hsa-mir-4761 MI0017402 | hsa-mir-548as MI0018132 |
| hsa-mir-4492 MI0016854 | hsa-mir-4762 MI0017403 | hsa-mir-548at MI0019137 |
| hsa-mir-4493 MI0016855 | hsa-mir-4763 MI0017404 | hsa-mir-548au MI0019145 |
| hsa-mir-4494 MI0016856 | hsa-mir-4764 MI0017405 | hsa-mir-548av MI0019152 |
| hsa-mir-4495 MI0016857 | hsa-mir-4765 MI0017406 | hsa-mir-548aw MI0019283 |
| hsa-mir-4496 MI0016858 | hsa-mir-4766 MI0017407 | hsa-mir-548ax MI0019286 |
| hsa-mir-4497 MI0016859 | hsa-mir-4767 MI0017408 | hsa-mir-548ay MI0022210 |
| hsa-mir-4498 MI0016860 | hsa-mir-4768 MI0017409 | hsa-mir-548az MI0022212 |
| hsa-mir-4499 MI0016862 | hsa-mir-4769 MI0017410 | hsa-mir-548b MI0003596 |
| hsa-mir-449a MI0001648 | hsa-mir-4770 MI0017411 | hsa-mir-548c MI0003630 |
| hsa-mir-449b MI0003673 | hsa-mir-4771-1 MI0017412 | hsa-mir-548d-1 MI0003668 |
| hsa-mir-449c MI0003823 | hsa-mir-4771-2 MI0017413 | hsa-mir-548d-2 MI0003671 |
| hsa-mir-4500 MI0016863 | hsa-mir-4772 MI0017414 | hsa-mir-548e MI0006344 |
| hsa-mir-4501 MI0016864 | hsa-mir-4773-1 MI0017415 | hsa-mir-548f-1 MI0006374 |
| hsa-mir-4502 MI0016865 | hsa-mir-4773-2 MI0017416 | hsa-mir-548f-2 MI0006375 |
| hsa-mir-4503 MI0016866 | hsa-mir-4774 MI0017417 | hsa-mir-548f-3 MI0006376 |
| hsa-mir-4504 MI0016867 | hsa-mir-4775 MI0017418 | hsa-mir-548f-4 MI0006377 |
| hsa-mir-4505 MI0016868 | hsa-mir-4776-1 MI0017419 | hsa-mir-548f-5 MI0006378 |
| hsa-mir-4506 MI0016869 | hsa-mir-4776-2 MI0017420 | hsa-mir-548g MI0006395 |
| hsa-mir-4507 MI0016871 | hsa-mir-4777 MI0017421 | hsa-mir-548h-1 MI0006411 |
| hsa-mir-4508 MI0016872 | hsa-mir-4778 MI0017422 | hsa-mir-548h-2 MI0006412 |
| hsa-mir-4509-1 MI0016873 | hsa-mir-4779 MI0017423 | hsa-mir-548h-3 MI0006413 |
| hsa-mir-4509-2 MI0016874 | hsa-mir-4780 MI0017424 | hsa-mir-548h-4 MI0006414 |
| hsa-mir-4509-3 MI0016875 | hsa-mir-4781 MI0017426 | hsa-mir-548h-5 MI0016751 |
| hsa-mir-450a-1 MI0001652 | hsa-mir-4782 MI0017427 | hsa-mir-548i-1 MI0006421 |
| hsa-mir-450a-2 MI0003187 | hsa-mir-4783 MI0017428 | hsa-mir-548i-2 MI0006422 |
| hsa-mir-450b MI0005531 | hsa-mir-4784 MI0017429 | hsa-mir-548i-3 MI0006423 |
| hsa-mir-4510 MI0016876 | hsa-mir-4785 MI0017430 | hsa-mir-540i-4 MI0006424 |
| hsa-mir-4511 MI0016877 | hsa-mir-4786 MI0017433 | hsa-mir-548j MI0006345 |
| hsa-mir-4512 MI0016878 | hsa-mir-4787 MI0017434 | hsa-mir-548k MI0006354 |
| hsa-mir-4513 MI0016879 | hsa-mir-4788 MI0017435 | hsa-mir-548l MI0006361 |
| hsa-mir-4514 MI0016880 | hsa-mir-4789 MI0017436 | hsa-mir-548m MI0006400 |
| hsa-mir-4515 MI0016881 | hsa-mir-4790 MI0017437 | hsa-mir-548n MI0006399 |
| hsa-mir-4516 MI0016882 | hsa-mir-4791 MI0017438 | hsa-mir-548o MI0006402 |
| hsa-mir-4517 MI0016883 | hsa-mir-4792 MI0017439 | hsa-mir-548o-2 MI0016746 |
| hsa-mir-4518 MI0016884 | hsa-mir-4793 MI0017440 | hsa-mir-548p MI0006420 |
| hsa-mir-4519 MI0016885 | hsa-mir-4794 MI0017441 | hsa-mir-548q MI0010637 |
| hsa-mir-451a MI0001729 | hsa-mir-4795 MI0017442 | hsa-mir-548s MI0014141 |
| hsa-mir-451b MI0017360 | hsa-mir-4796 MI0017443 | hsa-mir-548t MI0014164 |
| hsa-mir-452 MI0001733 | hsa-mir-4797 MI0017444 | hsa-mir-548u MI0014168 |
| hsa-mir-4520a MI0016886 | hsa-mir-4798 MI0017445 | hsa-mir-548v MI0014174 |
| hsa-mir-4520b MI0017358 | hsa-mir-4799 MI0017446 | hsa-mir-548w MI0014222 |
| hsa-mir-4521 MI0016887 | hsa-mir-4800 MI0017448 | hsa-mir-548x MI0014244 |
| hsa-mir-4522 MI0016889 | hsa-mir-4801 MI0017449 | hsa-mir-548x-2 MI0016833 |
| hsa-mir-4523 MI0016890 | hsa-mir-4802 MI0017450 | hsa-mir-548y MI0016595 |
| hsa-mir-4524a MI0016891 | hsa-mir-4803 MI0017451 | hsa-mir-548z MI0016688 |
| hsa-mir-4524b MI0019114 | hsa-mir-4804 MI0017452 | hsa-mir-549a MI0003679 |
| hsa-mir-4525 MI0016892 | hsa-mir-483 MI0002467 | hsa-mir-550a-1 MI0003600 |
| hsa-mir-4526 MI0016893 | hsa-mir-484 MI0002468 | hsa-mir-550a-2 MI0003601 |
| hsa-mir-4527 MI0016894 | hsa-mir-485 MI0002469 | hsa-mir-550a-3 MI0003762 |
| hsa-mir-4528 MI0016895 | hsa-mir-486 MI0002470 | hsa-mir-550b-1 MI0016686 |
| hsa-mir-4529 MI0016896 | hsa-mir-487a MI0002471 | hsa-mir-550b-2 MI0016687 |
| hsa-mir-4530 MI0016897 | hsa-mir-487b MI0003530 | hsa-mir-551a MI0003556 |
| hsa-mir-4531 MI0016898 | hsa-mir-488 MI0003123 | hsa-mir-551b MI0003575 |
| hsa-mir-4532 MI0016899 | hsa-mir-489 MI0003124 | hsa-mir-552 MI0003557 |
| hsa-mir-4533 MI0016900 | hsa-mir-490 MI0003125 | hsa-mir-553 MI0003558 |
| hsa-mir-4534 MI0016901 | hsa-mir-491 MI0003126 | hsa-mir-554 MI0003559 |
| hsa-mir-4535 MI0016903 | hsa-mir-492 MI0003131 | hsa-mir-555 MI0003561 |
| hsa-mir-4536-1 MI0016906 | hsa-mir-493 MI0003132 | hsa-mir-556 MI0003562 |
| hsa-mir-4536-2 MI0019149 | hsa-mir-494 MI0003134 | hsa-mir-557 MI0003563 |
| hsa-mir-4537 MI0016908 | hsa-mir-495 MI0003135 | hsa-mir-5571 MI0019115 |
| hsa-mir-4538 MI0016909 | hsa-mir-496 MI0003136 | hsa-mir-5572 MI0019117 |
| hsa-mir-4539 MI0016910 | hsa-mir-497 MI0003138 | hsa-mir-5579 MI0019133 |
| hsa-mir-454 MI0003820 | hsa-mir-498 MI0003142 | hsa-mir-558 MI0003564 |
| hsa-mir-4540 MI0016911 | hsa-mir-4999 MI0017865 | hsa-mir-5580 MI0019135 |
| hsa-mir-455 MI0003513 | hsa-mir-499a MI0003183 | hsa-mir-5581 MI0019136 |
| hsa-mir-4632 MI0017259 | hsa-mir-499b MI0017396 | hsa-mir-5582 MI0019138 |
| hsa-mir-4633 MI0017260 | hsa-mir-5000 MI0017866 | hsa-mir-5583-1 MI0019139 |
| hsa-mir-4634 MI0017261 | hsa-mir-5001 MI0017867 | hsa-mir-5583-2 MI0019140 |
| hsa-mir-4635 MI0017262 | hsa-mir-5002 MI0017868 | hsa-mir-5584 MI0019141 |
| hsa-mir-4636 MI0017263 | hsa-mir-5003 MI0017869 | hsa-mir-5585 MI0019142 |
| hsa-mir-4637 MI0017264 | hsa-mir-5004 MI0017870 | hsa-mir-5586 MI0019143 |
| hsa-mir-4638 MI0017265 | hsa-mir-5006 MI0017873 | hsa-mir-5587 MI0019144 |
| hsa-mir-4639 MI0017266 | hsa-mir-5007 MI0017874 | hsa-mir-5588 MI0019147 |
| hsa-mir-4640 MI0017267 | hsa-mir-5008 MI0017876 | hsa-mir-5589 MI0019148 |
| hsa-mir-4641 MI0017268 | hsa-mir-5009 MI0017877 | hsa-mir-559 MI0003565 |
| hsa-mir-4642 MI0017269 | hsa-mir-500a MI0003184 | hsa-mir-5590 MI0019150 |
| hsa-mir-4643 MI0017270 | hsa-mir-500b MI0015903 | hsa-mir-5591 MI0019151 |
| hsa-mir-4644 MI0017271 | hsa-mir-501 MI0003185 | hsa-mir-561 MI0003567 |
| hsa-mir-4645 MI0017272 | hsa-mir-5010 MI0017878 | hsa-mir-562 MI0003568 |
| hsa-mir-4646 MI0017273 | hsa-mir-5011 MI0017879 | hsa-mir-563 MI0003569 |
| hsa-mir-4647 MI0017274 | hsa-mir-502 MI0003186 | hsa-mir-564 MI0003570 |
| hsa-mir-4648 MI0017275 | hsa-mir-503 MI0003188 | hsa-mir-566 MI0003572 |
| hsa-mir-4649 MI0017276 | hsa-mir-504 MI0003189 | hsa-mir-567 MI0003573 |
| hsa-mir-4650-1 MI0017277 | hsa-mir-5047 MI0017932 | hsa-mir-568 MI0003574 |
| hsa-mir-4650-2 MI0017278 | hsa-mir-505 MI0003190 | hsa-mir-5680 MI0019280 |
| hsa-mir-4651 MI0017279 | hsa-mir-506 MI0003193 | hsa-mir-5681a MI0019281 |
| hsa-mir-4652 MI0017280 | hsa-mir-507 MI0003194 | hsa-mir-5681b MI0019293 |
| hsa-mir-4653 MI0017281 | hsa-mir-508 MI0003195 | hsa-mir-5682 MI0019282 |
| hsa-mir-4654 MI0017282 | hsa-mir-5087 MI0017976 | hsa-mir-5683 MI0019284 |
| hsa-mir-4655 MI0017283 | hsa-mir-5088 MI0017977 | hsa-mir-5684 MI0019285 |
| hsa-mir-4656 MI0017284 | hsa-mir-5089 MI0017978 | hsa-mir-5685 MI0019287 |
| hsa-mir-4657 MI0017285 | hsa-mir-5090 MI0017979 | hsa-mir-5686 MI0019290 |
| hsa-mir-4658 MI0017286 | hsa-mir-509-1 MI0003196 | hsa-mir-5687 MI0019291 |
| hsa-mir-4659a MI0017287 | hsa-mir-5091 MI0017980 | hsa-mir-5688 MI0019292 |
| hsa-mir-4659b MI0017291 | hsa-mir-509-2 MI0005530 | hsa-mir-5689 MI0019294 |
| hsa-mir-466 MI0014157 | hsa-mir-5092 MI0017981 | hsa-mir-569 MI0003576 |
| hsa-mir-4660 MI0017288 | hsa-mir-509-3 MI0005717 | hsa-mir-5690 MI0019295 |
| hsa-mir-4661 MI0017289 | hsa-mir-5093 MI0017982 | hsa-mir-5691 MI0019296 |
| hsa-mir-4662a MI0017290 | hsa-mir-5094 MI0017983 | hsa-mir-5692a-1 MI0019297 |
| hsa-mir-4662b MI0017293 | hsa-mir-5095 MI0018001 | hsa-mir-5692a-2 MI0019298 |
| hsa-mir-4663 MI0017292 | hsa-mir-5096 MI0018004 | hsa-mir-5692b MI0019311 |
| hsa-mir-4664 MI0017294 | hsa-mir-510 MI0003197 | hsa-mir-5692c-1 MI0019288 |
| hsa-mir-4665 MI0017295 | hsa-mir-5100 MI0019116 | hsa-mir-5692c-2 MI0019289 |
| hsa-mir-4666a MI0017296 | hsa-mir-511-1 MI0003127 | hsa-mir-5693 MI0019300 |
| hsa-mir-4666b MI0019299 | hsa-mir-511-2 MI0003128 | hsa-mir-5694 MI0019301 |
| hsa-mir-4667 MI0017297 | hsa-mir-512-1 MI0003140 | hsa-mir-5695 MI0019302 |
| hsa-mir-4668 MI0017298 | hsa-mir-512-2 MI0003141 | hsa-mir-5696 MI0019303 |
| hsa-mir-4669 MI0017300 | hsa-mir-513a-1 MI0003191 | hsa-mir-5697 MI0019304 |
| hsa-mir-4670 MI0017301 | hsa-mir-513a-2 MI0003192 | hsa-mir-5698 MI0019305 |
| hsa-mir-4671 MI0017302 | hsa-mir-513b MI0006648 | hsa-mir-5699 MI0019306 |
| hsa-mir-4672 MI0017303 | hsa-mir-513c MI0006649 | hsa-mir-570 MI0003577 |
| hsa-mir-4673 MI0017304 | hsa-mir-514a-1 MI0003198 | hsa-mir-5700 MI0019307 |
| hsa-mir-4674 MI0017305 | hsa-mir-514a-2 MI0003199 | hsa-mir-5701-1 MI0019308 |
| hsa-mir-4675 MI0017306 | hsa-mir-514a-3 MI0003200 | hsa-mir-5701-2 MI0019593 |
| hsa-mir-4676 MI0017307 | hsa-mir-514b MI0014251 | hsa-mir-5702 MI0019309 |
| hsa-mir-4677 MI0017308 | hsa-mir-515-1 MI0003144 | hsa-mir-5703 MI0019310 |
| hsa-mir-4678 MI0017309 | hsa-mir-515-2 MI0003147 | hsa-mir-5704 MI0019312 |
| hsa-mir-4679-1 MI0017310 | hsa-mir-516a-1 MI0003180 | hsa-mir-5705 MI0019313 |
| hsa-mir-4679-2 MI0017311 | hsa-mir-516a-2 MI0003181 | hsa-mir=5706 MI0019314 |
| hsa-mir-4680 MI0017312 | hsa-mir-516b-1 MI0003172 | hsa-mir-5707 MI0019315 |
| hsa-mir-4681 MI0017313 | hsa-mir-516b-2 MI0003167 | hsa-mir-5708 MI0019316 |
| hsa-mir-4682 MI0017314 | hsa-mir-517a MI0003161 | hsa-mir-571 MI0003578 |
| hsa-mir-4683 MI0017315 | hsa-mir-517b MI0003165 | hsa-mir-572 MI0003679 |
| hsa-mir-4684 MI0017316 | hsa-mir-517c MI0003174 | hsa-mir-573 MI0003580 |
| hsa-mir-4685 MI0017317 | hsa-mir-5186 MI0018165 | hsa-mir-5739 MI0019412 |
| hsa-mir-4686 MI0017318 | hsa-mir-5187 MI0018166 | hsa-mir-574 MI0003581 |
| hsa-mir-4687 MI0017319 | hsa-mir-5188 MI0018167 | hsa-mir-575 MI0003582 |
| hsa-mir4688 MI0017321 | sa-mir-5189 MI0018168 | hsa-mir-576 MI0003588 |
| hsa-mir-4689 MI0017322 | sa-mir-518a-1 MI0003170 | hsa-mir-577 MI0003584 |
| hsa-mir4690 MI0017323 | sa-mir-518a-2 MI0003173 | hsa-mir-578 MI0003585 |
| hsa-mir-4691 MI0017324 | sa-mir-518b MI0003156 | hsa-mir-5787 MI0019797 |
| hsa-mir-4692 MI0017325 | hsa-mir-518c MI0003159 | hsa-mir-579 MI0003586 |
| hsa-mir-4693 MI0017326 | hsa-mir-518d MI0003171 | hsa-mir-580 MI0003587 |
| hsa-mir-4694 MI0017327 | hsa-mir-518e MI0003169 | hsa-mir-581 MI0003588 |
| hsa-mir-4695 MI0017328 | hsa-mir-518f MI0003154 | hsa-mir-582 MI0003589 |
| hsa-mir-4696 MI0017329 | hsa-mir-5190 MI0018169 | hsa-mir-583 MI0003590 |
| hsa-mir-4697 MI0017330 | hsa-mir-5191 MI0018170 | hsa-mir-584 MI0003591 |
| hsa-mir-4698 MI0017331 | hsa-mir-5192 MI0018171 | hsa-mir-585 MI0003592 |
| hsa-mir-4699 MI0017332 | hsa-mir-5193 MI0018172 | hsa-mir-586 MI0003594 |
| hsa-mir-4700 MI0017333 | hsa-mir-5194 MI0018173 | hsa-mir-587 MI0003595 |
| hsa-mir-4701 MI0017334 | hsa-mir-5195 MI0018174 | hsa-mir-588 MI0003597 |
| hsa-mir-4703 MI0017336 | hsa-mir-5196 MI0018175 | hsa-mir-589 MI0003599 |
| hsa-mir-4704 MI0017337 | hsa-mir-5197 MI0018176 | hsa-mir-590 MI0003602 |
| hsa-mir-4705 MI0017338 | hsa-mir-519a-1 MI0003178 | hsa-mir-591 MI0003603 |
| hsa-mir-4706 MI0017339 | hsa-mir-519a-2 MI0003182 | hsa-mir-592 MI0003604 |
| hsa-mir-4707 MI0017340 | hsa-mir-519b MI0003151 | hsa-mir-593 MI0003605 |
| hsa-mir-4708 MI0017341 | hsa-mir-519c MI0003148 | hsa-mir-595 MI0003607 |
| hsa-mir-4709 MI0017342 | hsa-mir-519d MI0003162 | hsa-mir-596 MI0003608 |
| hsa-mir-4710 MI0017344 | hsa-mir-519e MI0003145 | hsa-mir-597 MI0003609 |
| hsa-mir-4711 MI0017345 | hsa-mir-520a MI0003149 | hsa-mir-598 MI0003610 |
| hsa-mir-4712 MI0017346 | hsa-mir-520b MI003155 | hsa-mir-599 MI0003611 |
| hsa-mir-4713 MI0017347 | hsa-mir-520c MI0003158 | hsa-mir-600 MI0003613 |
| hsa-mir-4714 MI0017348 | hsa-mir-520d MI0003164 | hsa-mir-601 MI0003614 |
| hsa-mir-4715 MI0017349 | hsa-mir-520e MI0003143 | hsa-mir-602 MI0003615 |
| hsa-mir-4716 MI0017350 | hsa-mir-520f MI0003146 | hsa-mir-603 MI0003616 |
| hsa-mir-4717 MI0017352 | hsa-mir-520g MI0003166 | hsa-mir-604 MI0003617 |
| hsa-mir-4718 MI0017353 | hsa-mir-520h MI0003175 | hsa-mir-605 MI0003618 |
| hsa-mir-4719 MI0017354 | hsa-mir-521-1 MI0003176 | hsa-mir-606 MI0003619 |
| hsa-mir-4720 MI0017355 | hsa-mir-521-2 MI0003163 | hsa-mir-6068 MI0020345 |
| hsa-mir-6069 MI0020346 | hsa-mir-641 MI0003656 | hsa-mir-7-3 MI0000265 |
| hsa-mir-607 MI0003620 | hsa-mir-642a MI0003657 | hsa-mir-744 MI0005559 |
| hsa-mir-6070 MI0020347 | hsa-mir-642b MI0016685 | hsa-mir-758 MI0003757 |
| hsa-mir-6071 MI0020348 | hsa-mir-643 MI0003658 | hsa-mir-759 MI0004065 |
| hsa-mir-6072 MI0020349 | hsa-mir-644a MI0003659 | hsa-mir-760 MI0005567 |
| hsa-mir-6073 MI0020350 | hsa-mir-645 MI0003660 | hsa-mir-761 MI0063941 |
| hsa-mir-6074 MI0020351 | hsa-mir-646 MI0003661 | hsa-mir-762 MI0003892 |
| hsa-mir-6075 MI0020352 | hsa-mir-647 MI0003662 | hsa-mir-764 MI0003944 |
| hsa-mir-6076 MI0020353 | hsa-mir-648 MI0003663 | hsa-mir-765 MI0005116 |
| hsa-mir-6077-1 MI0020354 | hsa-mir-649 MI0003664 | hsa-mir-766 MI0003836 |
| hsa-mir-6077-2 MI0023562 | hsa-mir-6499 MI0022209 | hsa-mir-767 MI0003763 |
| hsa-mir-6078 MI0020355 | hsa-mir-650 MI0003665 | hsa-mir-769 MI0003834 |
| hsa-mir-6079 MI0020356 | hsa-mir-6500 MI0022211 | hsa-mir-770 MI0005118 |
| hsa-mir-608 MI0003621 | hsa-mir-6501 MI0022213 | hsa-mir-802 MI0003906 |
| hsa-mir-6080 MI0020357 | hsa-mir-6502 MI0022214 | hsa-mir-873 MI0005564 |
| hsa-mir-6081 MI0020358 | hsa-mir-6503 MI0022215 | hsa-mir-874 MI0005532 |
| hsa-mir-6082 MI0020359 | hsa-mir-6504 MI0022216 | hsa-mir-875 MI0005541 |
| hsa-mir-6083 MI0020360 | hsa-mir-6505 MI0022217 | hsa-mir-876 MI0005542 |
| hsa-mir-6084 MI0020361 | hsa-mir-6506 MI0022218 | hsa-mir-877 MI0005561 |
| hsa-mir-6085 M10020362 | hsa-mir-6507 MI0022219 | hsa-mir-885 MI0005560 |
| hsa-mir-6086 MI0020363 | hsa-mir-6508 MI0022220 | hsa-mir-887 MI0005562 |
| hsa-mir-6087 MI0020364 | hsa-mir-6509 MI0022221 | hsa-mir-888 MI0005537 |
| hsa-mir-6088 MI0020365 | hsa-mir-651 MI0003666 | hsa-mir-889 MI0005540 |
| hsa-mir-6089-1 MI0020366 | hsa-mir-6510 MI0022222 | hsa-mir-890 MI0005533 |
| hsa-mir-6089-2 MI0023563 | hsa-mir-6511a-1 MI0022223 | hsa-mir-891a MI0005524 |
| hsa-mir-609 MI0003622 | hsa-mir-6511a-2 MI0023564 | hsa-mir-891b MI0005534 |
| hsa-mir-6090 MI0020367 | hsa-mir-6511a-3 MI0023565 | hsa-mir-892a MI0005528 |
| hsa-mir-610 MI0003623 | hsa-mir-6511a-4 MI0023566 | hsa-mir-892b MI0005538 |
| hsa-mir-611 MI0003624 | hsa-mir-6511b-1 MI0022552 | hsa-mir-892c MI0022560 |
| hsa-mir-612 MI0003625 | hsa-mir-6512 MI0022224 | hsa-mir-9-1 MI0000466 |
| hsa-mir-6124 MI0021258 | hsa-mir-6513 MI0022225 | hsa-mir-9-2 MI0000467 |
| hsa-mir-6125 MI0021259 | hsa-mir-6514 MI0022226 | hsa-mir-920 MI0005712 |
| hsa-mir-6126 MI0021260 | hsa-mir-6515 MI0022227 | hsa-mir-921 MI0005713 |
| hsa-mir-6127 MI0021271 | hsa-mir-652 MI0003667 | hsa-mir-922 MI0005714 |
| hsa-mir-6128 MI0021272 | hsa-mir-653 MI0003674 | hsa-mir-924 MI0005716 |
| hsa-mir-6129 MI0021274 | hsa-mir-654 MI0003676 | hsa-mir-92a-1 MI0000093 |
| hsa-mir-613 MI0003626 | hsa-mir-655 MI0003677 | hsa-mir-92a-2 MI0000094 |
| hsa-mir-6130 MI0021275 | hsa-mir-656 MI0003678 | hsa-mir-92b MI0003560 |
| hsa-mir-6131 MI0021276 | hsa-mir-657 MI0003681 | hsa-mir-93 MI0000095 |
| hsa-mir-6132 MI0021277 | hsa-mir-658 MI0003682 | hsa-mir-9-3 MI0000468 |
| hsa-mir-6133 MI0021278 | hsa-mir-659 MI0003683 | hsa-mir-933 MI0005755 |
| hsa-mir-6134 MI0021279 | hsa-mir-660 MI0003684 | hsa-mir-934 MI0005756 |
| hsa-mir-614 MI0003627 | hsa-mir-661 MI0003669 | hsa-mir-935 MI0006757 |
| hsa-mir-615 MI0003628 | hsa-mir-662 MI0003670 | hsa-mir-936 MI0005758 |
| hsa-mir-616 MI0003629 | hsa-mir-663a MI0003672 | hsa-mir-937 MI0005759 |
| hsa-mir-6165 MI0021472 | hsa-mir-663b MI0006336 | hsa-mir-938 MI0005760 |
| hsa-mir-617 MI0003631 | hsa-mir-664a MI0006442 | hsa-mir-939 MI0005761 |
| hsa-mir-618 MI0003632 | hsa-mir-664b MI0019134 | hsa-mir-940 MI0005762 |
| hsa-mir-619 MI0003633 | hsa-mir-665 MI0005563 | hsa-mir-941-1 MI0005763 |
| hsa-mir-620 MI0003634 | hsa-mir-668 MI0003761 | hsa-mir-941-2 MI0005764 |
| hsa-mir-621 MI0003635 | hsa-mir-670 MI0003933 | hsa-mir-941-3 MI0005765 |
| hsa-mir-622 MI0003636 | hsa-mir-671 MI0008760 | hsa-mir-941-4.MI0005766 |
| hsa-mir-623 MI0003637 | hsa-mir-6715a MI0022548 | hsa-mir-942 MI0005767 |
| hsa-mir-624 MI0003638 | hsa-mir-6715b MI0022549 | hsa-mir-943 MI0005768 |
| hsa-mir-625 MI0003639 | hsa-mir-6716 MI0022550 | hsa-mir-944 MI0005769 |
| hsa-mir-626 MI0003640 | hsa-mir-6717 MI0022551 | hsa-mir-95 MI0000097 |
| hsa-mir-627 MI0003641 | hsa-mir-6718 MI0022553 | hsa-mir-96 MI0000098 |
| hsa-mir-628 MI6003642 | hsa-mir-6719 MI0022554 | hsa-mir-98 MI0000100 |
| hsa-mir-629 MI0003643 | hsa-mir-6720 MI0022555 | hsa-mir-99a MI0000101 |
| hsa-mir-630 MI0003644 | hsa-mir-6721 MI0022556 | hsa-mir-99b MI0000746 |
| hsa-mir-631 MI0003645 | hsa-mir-6722 MI0022557 | |
| hsa-mir-632 MI0003647 | hsa-mir-6723 MI0022558 | |
| hsa-mir-633 MI0003648 | hsa-mir-6724 MI0022559 | |
| hsa-mir-634 MI0003649 | hsa-mir-675 MI0005416 | |
| hsa-mir-635 MI0003650 | hsa-mir-676 MI0016436 | |
| hsa-mir-636 MI0003651 | hsa-mir-708 MI0005543 | |
| hsa-mir-637 MI0003652 | hsa-mir-7-1 MI0000263 | |
| hsa-mir-638 MI0003653 | hsa-mir-711 MI0012488 | |
| hsa-mir-639 MI0003654 | hsa-mir-718 MI0012489 | |
| hsa-mir-640 MI0003655 | hsa-mir-7-2 MI0000264 | |

## Claims

1. Method for identifying a compound modulating an interaction between two biomo-lecules or two domains of one biomolecule, the first biomolecule or first domain comprising at least one fluorophore donor and the second biomolecule or second domain comprising at least one fluorophore acceptor or a dark quencher, wherein the fluorophore donor and fluorophore acceptor or the fluorophore donor and dark quencher are spectrally paired such that the energy spectrum emitted by the fluorophore donor and the excitation energy spectrum of the fluorophore acceptor or the energy spectrum absorbed by the dark quencher overlap at least partially, comprising the steps of
(i) providing the first and second biomolecules or a biomolecule comprising said first and second domains,
(ii) providing a compound of interest,
(iii) contacting the first and second biomolecules or the biomolecule comprising the first and second domains with the compound of interest under conditions that allow for FRET (Förster Resonance Energy Transfer) between the fluorophore donor and fluorophore acceptor or the fluorophore donor and the dark quencher,
(iv) identifying the compound of interest as a compound modulating, preferably inhibiting or enhancing an interaction between the two biomolecules or the two domains of the biomolecule if the FRET (Förster Resonance Energy Transfer) between the first and second biomolecules or the first and second domains differs in the presence of the compound of interest compared to the FRET between the first and second biomolecules or the first and second domains in the absence of the compound of interest.

2. Method according to claim 1, wherein each of the first and second biomolecules or the biomolecule with the first and second domains is selected from the group consisting of polypeptides, sugars, polynucleotides, polyamines and lipids.

3. The method of claim 2, wherein the first biomolecule is selected from the group consisting of polypeptides interacting with polynucleotides and the second biomolecule is selected from the group consisting of polynucleotides, preferably microRNA(miRNA).

4. The method of claim 3, wherein the polypeptide interacting with polynucleotides, preferably miRNA, is selected from
(i) the group consisting of FOX protein family members (Fox1: NM_001142333.1 → NP_001135805.1; Fox2: N_001031695.2 → NP_001026865.1; Fox3: NM_001082575.2 → NP_001076044.1), DAZ protein family members (BOLL: NM_033030.5 → NP_149019.1; DAZL. NM_001277863.1 → NP_001264792.1; DAZ1: NM_004081.5 → NP_004072.3), DND1 (NM_194249.2 →NP_919225.1), ELAV1 and ELAV-like protein family members (preferably ELAV1: NM_001419.2 → NP_001410.2), GW182 protein family members (GW182:NM_001011116.1 → NP_001011116.1; TNRC6B: NM_001024843.1 → NP_001020014.1; TNRC6C: NM_001142640.1 → NP_001136112.1), hnRNP protein family members, preferably hnRNPA1(NM_002136.2 → NP_002127.1), IGF2BP1 (NM_001160423.1 → NP_001153895.1), PABP protein family members, preferably PABPC1 (NM_002568.3 → NP_002559.2), SFRS1 (NM_001078166.1 → NP_001071634.1), DGCR8 (NM_001190326.1 → NP_001177255.1), Dicer1 (NM_001195573.1→NP_001182502.1), Drosha(NM_001100412.1 → NP_001093882.1), TARBP2 (NM_004178.4 → NP_004169.3), FMR1 (NM_001185075.1 → NP_001172004.1), FXR1 (NM_001013438.2 → NP_001013456.1), FXR2 (NM_004860.3 → NP_004851.2), KHSRP (NM_003685.2 → NP_003676.2), STAR (NM_000349.2 → NP_000340.2), AGO protein family members, preferably AGO1 (NM_312199.2 → NP_036331.1), TRIM proteins with NHL domain, preferably TRIM1 (NM_012216.3 → NP_036348.2), ZFP36(NM_003407.3 → NP_003398.2), PUM1 (NM_001020658.1 → NP_001018494.1), PUM2 (NM_015317.1 → NP_056132.1), DDX5, (NM_004396.3 → NP_004387.1), DDX6 (NM_001257191.1 → NP_001244120.1), DDX17 (NM_001098504.1 → NP_001091974.1), DDX20 (NM_007204.4 → NP_009135.4), DDX42 (NM_007372.2 → NP_031398.2), MOV10 (NM_001130079.1 (mRNA)→ NP_001123551.1), Lin28a (NM_024674.4 → NP_078950.1) and Lin28b (NM_001004317.3 → NP_001004317.1),
(ii) preferably the group consisting of hnRNPA1, KHSRP, Fox1 and Fox2, Lin28a and Lin28b,
(iii) more preferably the group consisting of Lin28b or Lin28a.

5. The method of claim 4, wherein the polypeptide interacting with miRNA is Lin28, preferably selected from the group consisting of mammalian, preferably human, non-human primate, rodent, monkey, mouse, rat, chicken, pig, guinea pig Lin28a and Lin28b, most preferably human Lin28a or Lin28b or a functional fragment or functional derivative thereof.

6. The method of any of claims 2 to 5, wherein the polynucleotide is a pri- or pre-miRNA or mature miRNA, preferably selected from the group consisting of hsa-let-7a-1, hsa-let-7a-2, hsa-let-7a-3 hsa-let-7b, hsa-let-7c, hsa-let-7d, hsa-let-7e, hsa-let-7f-1, hsa-let-7f-2, hsa-let-7g, hsa-let-7i, and functional derivatives or fragments thereof.

7. The method of any of claims 1 to 6, wherein the fluorophore donor is selected from fluorescent proteins and small fluorescent dye molecule,
(i) preferably fluorescent proteins selected from the group consisting of
(i.1) blue fluorescent proteins, preferably selected from the group consisting of EBFP, EBFP2, Azurite and mTagBFP,
(i.2) cyan fluorescent proteins, preferably selected from the group consisting of ECFP, mECFP, Cerulean, mTurquoise, CyPet, AmCyan1, Midori-Ishi Cyan, TagCFP and mTFP1 (Teal),
(i.3) yellow fluorescent proteins, preferably selected from the group consisting of EYFP, Topaz, Venus, mCitrine, YPet, TagYFP, PhiYFP, ZsYellow1 and mBanana,
(i.4) orange fluorescent proteins, preferably selected from the group consisting of Kusabira Orange, Kusabira Orange2, mOrange, mOrange2, dTomato, dTomato-Tandem, TagRFP, TagRFP-T, DsRed, DsRed2, DsRed-Express (T1), DsRed-Monomer and mTangerine,
(i:5) red fluorescent proteins, preferably selected from the group consisting of mRuby, mApple, mStrawberry, AsRed2, mRFP1, JRed, mCherry, HcRed1, m Raspberry, dKeima-Tandem, HcRed-Tandem mPlum and AQ143, and
(i.6) green fluorescent proteins (GFP), preferably selected from the group consisting of EGFP, Emerald, Superfolder GFP, Azami Green, mWasabi, TagGFP, TurboGFP, AcGFP, ZsGreen and T-Sapphire,
more preferably green fluorescent proteins (i.6) and yellow fluorescent proteins (i.3), most preferably EGFP;
(ii) preferably small fluorescent dye molecules selected from the group consisting of
ii.1) acridines, preferably acridine orange or acridine yellow,
(ii.2) cyanines, preferably Cy2, Cy3, Cy3B, Cy3.5, Cy5, Cy5.5, Cy7,
(ii.3) fluorones, preferably Fluorescein, Carboxyfluorescein, Dichlorofluorescein, Eosin, Eosih B, Eosin Y or Erythrosine,
(ii.4) oxazines, preferably Cresyl violet, Nile blue or Nile red,
(ii.5) phenanthridines, preferably Ethidium bromide, Gelred or Propidium iodide, and
(ii.6) rhodamines, preferably Rhodamine, Rhodamine 123, Rhodamine 6G, Rhodamine B, Auramine, Sulforhodamine 101, Sulforhodamine B or Texas. red,
(iii) more preferably cyanine and rhodamine dyes.

8. The method of any of claims 1 to 7, wherein the fluorophore acceptor is selected from the group consisting of
(i) acridines, preferably acridine orange or acridine yellow,
(ii) cyanines, preferably Cy2, Cy3, Cy3B, Cy3.5, Cy5, Cy5.5 or Cy7,
(iii) fluorones, preferably Fluorescein, Carboxyfluorescein, Dichlorofluorescein, Eosin, Eosin B, Eosin Y or Erythrosine,
(iv) oxazines, preferably Cresyl violet, Nile blue or Nile red,
(v) phenanthridines, preferably ethidium bromide, Gelred or propidium iodide, and
(vi) rhodamines, preferably Rhodamine, Rhodamine 123, Rhodamine 6G, Rhodamine B, Auramine, Sulforhodamine 101, Sulforhodamine B or Texas red,
preferably cyanines (ii), more preferably Cy3.

9. The method of any of claims 1 to 8, wherein the dark quencher selected from the group consisting of Dabcyl, Dabsyl, Black Hole Quencher (BHQ™) dyes, preferably BHQ-0, BHQ-1, BHQ-2 or BHQ-3, QXL quenchers, preferably QXL 490, QXL 570, QXL 610, QXL 670, or QXL 680, lowa Black quenchers, preferably lowa black FQ or lowa Black RQ, and IRDyes, preferably IRDye 800, IRDye 800CW, IRDye 800RS, IRDye 680, IRDye 680LT, IRDye 700, or IRDye 700DX, more preferably Black Hole Quencher (BHQ™) dyes, most preferably BHQ-1.

10. The method of any of claims 1 to 9, wherein the spectrally paired fluorophore donor and fluorophore acceptor or the spectrally paired fluorophore donor and dark quencher are selected from the group consisting of
(i) protein-protein pairs, preferably selected from the group consisting of ECFP-Citrine, ECFP-Venus, Cerulean-Citrine, Cerulean-Venus, Cerulean-Ypet, Cerulean-YFP, CyPet-EYFP, CyPet-Venus, CyPet-YPet, CyPet-Citrine, mTurquoise-Venus, mTurquoise-Ypet, mTurquoise-Citrine, ECFP-EYFP, TagGFP-TagRFP, mTFP1-Citrine, Citrine-mKate2, mTurquoise1-SEYFP, mTurquoise2-SEYFP and clover-mRuby2,
(ii) protein-organic dye pairs, preferably selected from the group consisting of EGFP-mCherry, SYFP2-mStrawberry, mTFP1-mOrange, Cloyer-mCherry, GFP-Cy3, YFP-Cy3, ECFP-BHQ-0, EYFP-BHQ-2, EGFP-Cy3 and EGFP-BHQ-1,
(iii) organic dye-organic-dye pairs, preferably selected from the group consisting of mOrange-mCherry, Alexa488-Alexa555, Alexa488-Cy3, Alexa 568-Alexa633, Cy3-Cy5, Alexa 488-Alexa514, Alexa488-Alexa532, Alexa488-546, Alexa488-610, Alexa647-Alexa 680, Alexa647-Alexa680, Alexa647-Aelxa700, Alexa647-Alexa750, BHQ-1-FAM, BHQ-1-TET, BHQ-1-JOE, BHQ-1-HEX, BHQ-1-Oregon green, BHQ-2-TAMRA, BHQ-2-ROX, BHQ-2-Cy3, BHQ-2-Cy3.5, BHQ-2-CAL Red, BHQ-2-Red 640, BHQ-3-Cy5, orBHQ-3-Cy5.5, Dabcyl-Edans and Dabsyl-Edans, fluorescine.

11. The method of any of claims 1 to 10, wherein the first biomolecule is Lin28(a or b) and the second biomolecule is pri- or pre-let-7a-2 or functional derivatives or fragments of these biomolecules.

12. The method of claim 11, wherein the fluorophore donor is at least one green fluorescent protein or yellow fluorescent protein and the fluorophore acceptor is at least one Cy3.

13. The method of claim 11, wherein the fluorophore donor is at least one green fluorescent protein or yellow fluorescent protein and the dark quencher is at least one dark quencher, preferably at least one black hole quencher (BHQ™), more preferably at least one BHQ-1.

14. The method of claim 11, wherein the at least one fluorophore donor is bound to the *N-*and/or C-terminal position of Lin28a or Lin28b and the at least one fluorophore acceptor and/or at least one black quencher (BHQ™) dye is bound to at least one of positions 1 to 67, preferably 5 to 60, more preferably 10 to 57, most preferably one of positions 10, 19, 34 and 57 of pri- or pre-let-7a-2.

15. The method of claim 14, wherein the at least one fluorophore donor, preferably GFP, more preferably EGFP is bound to the *N*- and/or C-terminal position of Lin28a or Lin28b and the at least one fluorophore acceptor, preferably Cy3 and/or at least one black hole quencher (BHQ™), preferably BHQ-1 is bound to at least one, preferably at least two of positions 10, 19, 34 and 57 of pri- or pre-let-7a-2, preferably to positions 10 and 19 or positions 10 and 34.
